# EUROPEAN PATENT SPECIFICATION

(11) **EP 2 971 030 B1**
(45) Date of publication and mention of the grant of the patent: **17.10.2018**
(21) Application number: 14767544.1
(22) Date of filing: 14.03.2014
(51) Int. Cl.: C12P 19/18, C12P 21/00, C12N 1/21

(54) **OLIGOSACCHARIDE COMPOSITIONS, GLYCOPROTEINS AND METHODS TO PRODUCE THE SAME IN PROKARYOTES**
OLIGOSACCHARIDZUSAMMENSETZUNGEN, GLYCOPROTEINE UND VERFAHREN ZUR HERSTELLUNG DAVON IN PROKARYONTEN
COMPOSITIONS D'OLIGOSACCHARIDE, GLYCOPROTÉINES ET PROCÉDÉS POUR PRODUIRE CELLES-CI DANS DES PROCARYOTES

(30) Priority: 14.03.2013 US 201361785586 P
(43) Date of publication of application: 20.01.2016
(73) Proprietor: Glycobia Inc., Ithaca, NY 14853 (US); Fisher, Adam, C., Ithaca, NY 14853 (US); Merritt, Judith H., Ithaca, NY 14853 (US); Hamilton, Brian S., Ithaca, NY 14853 (US); Valderrama-Rinco, Juan D., Ithaca, NY 14853 (US); Delisa, Matthew P., Ithaca, NY 14853 (US)
(72) Inventor: FISHER, Adam, C., Ithaca, NY 14853 (US); MERRITT, Judith, H., Ithaca, NY 14853 (US); HAMILTON, Brian, S., Ithaca, NY 14853 (US); VALDERRAMA-RINCON, Juan, D., Ithaca, NY 14853 (US); DELISA, Matthew, P., Ithaca, NY 14853 (US)
(74) Representative: Finnegan Europe LLP
(86) International application number: PCT/US2014/026990
(87) International publication number: WO 2014/152137

(56) References cited:
- WO-A1-2011/078987
- US-A1- 2002 001 831
- US-A1- 2004 063 911
- US-A1- 2004 181 827
- US-A1- 2007 037 248
- US-A1- 2009 311 744
- US-A1- 2011 039 729
- VALDERRAMA-RINCON JUAN D ET AL: "An engineered eukaryotic protein glycosylation pathway in Escherichia coli", May 2012 (2012-05), NATURE CHEMICAL BIOLOGY, VOL. 8, NR. 5, PAGE(S) 434-436, XP002763489, ISSN: 1552-4450(print) * the whole document * * figure 1 *
- HONGHONG, J ET AL.: 'Synthesis Of GDP-Mannose Using Coupling Fermentation Of Recombinant Escherichia coli.' BIOTECHNOL LETT. vol. 33, no. 6, June 2011, pages 1145 - 1150, XP019903565
- SAHDEV, S ET AL.: 'Production Of Active Eukaryotic Proteins Through Bacterial Expression Systems: A Review Of The Existing Biotechnology Strategies.' MOL CELL BIOCHEM. vol. 307, no. 1-2, January 2008, pages 249 - 264, XP019554846

## Description

This invention was made with government support under grant numbers 1R43GM088905-01, 2R44GM088905-02 and 5R44GM088905-03 by the National Institutes of Health. The government has certain rights in this invention.

### CROSS-REFERENCE TO RELATED APPLICATIONS

This application is related to U.S. Provisional Application No. 61/785,586, filed March 14, 20 13 for all purposes.

### SEQUENCE LISTING

This application contains a Sequence Listing which has been submitted via EFS-Web and is hereby incorporated by reference in its entirety. Said ASCII copy, created on [DATE], is named [.txt] and is [#######] bytes in size.

### FIELD OF INVENTION

The present invention generally relates to the field of glycobiology and protein engineering. More specifically, the embodiments described herein relates to oligosaccharide compositions and therapeutic glycoprotein production in prokaryotes.

### BACKGROUND OF THE INVENTION

### Glycotherapeutics

Protein-based therapeutics currently represent one in every four new drugs approved by the FDA (Walsh, G., "Biopharmaceutical Benchmarks," Nat Biotechnol 18:831-3 (2000); Walsh, G, "Biopharmaceutical Benchmarks," Nat Biotechnol 21:865-70 (2003); and Walsh, G, "Biopharmaceutical Benchmarks," Nat Biotechnol 24:769-76 (2006)).

While several protein therapeutics can be produced using a prokaryotic expression system such as *E. coli* (e.g., insulin), the vast majority of therapeutic proteins require additional post-translational modifications, thought to be absent in prokaryotes, to attain their full biological function. In particular, *N*-linked protein glycosylation is predicted to affect more than half of all eukaryotic protein species (Apweiler et al., "On the Frequency of Protein Glycosylation, as Deduced From Analysis of the SWISS-PROT Database," Biochim Biophys Acta 1473:4-8 (1999)) and is often essential for proper folding, pharmacokinetic stability, tissue targeting and efficacy for a large number of proteins (Helenius et al., "Intracellular Functions of N-linked Glycans," Science 291:2364-9 (2001)). Since most bacteria do not glycosylate their own proteins, expression of most therapeutically relevant glycoproteins, including antibodies, is relegated to mammalian cells. However, mammalian cell culture suffers from a number of drawbacks including: (i) extremely high manufacturing costs and low volumetric productivity of eukaryotic hosts, such as CHO cells, relative to bacteria; (ii) retroviral contamination; (iii) the relatively long time required to generate stable cell lines; (iv) relative inability to rapidly generate stable, "high-producing" eukaryotic cell lines via genetic modification; and (v) high product variability created by glycoform heterogeneity that arises when using host cells, such as CHO, that have endogenous non-human glycosylation pathways (Choi et al., "Use of Combinatorial Genetic Libraries to Humanize N-linked Glycosylation in the Yeast Pichia pastoris," Proc Natl Acad Sci U S A 100:5022-7 (2003)). Expression in *E. coli,* on the other hand, does not suffer from these limitations.

### Expression of therapeutic proteins in E. coli

Many therapeutic recombinant proteins are currently expressed using *E*. *coli* as a host organism. One of the best examples is human insulin, which was first produced in *E. coli* by Eli Lilly in 1982. Since that time, a vast number of human therapeutic proteins have been approved in the U.S. and Europe that rely on *E. coli* expression, including human growth hormone (hGH), granulocyte macrophage colony stimulating factor (GM-CSF), insulin-like growth factor (IGF-1, IGFBP-3), keratinocyte growth factor, interferons (IFN-α, IFN-β1b, IFN-γ1b), interleukins (IL-1, IL-2, IL-11), tissue necrosis factor (TNF-α), and tissue plasminogen activator (tPA). However, almost all glycoproteins are produced in mammalian cells. When a protein that is normally glycosylated is expressed in *E. coli,* the lack of glycosylation in that host can yield proteins with impaired function. For instance, aglycosylated human monoclonal antibodies (mAbs) (e.g., anti-tissue factor IgG1) can be expressed in soluble form and at high levels in *E. coli* (Simmons et al., "Expression of Full-length Immunoglobulins in Escherichia coli: Rapid and Efficient Production of Aglycosylated Antibodies," J Immunol Methods 263:133-47 (2002)). However, while *E.* coli-derived mAbs retained tight binding to their cognate antigen and neonatal receptor and exhibited a circulating half-life comparable to mammalian cell-derived antibodies, they were incapable of binding to C1q and the FcγRI receptor due to the absence of *N*-glycan.

### Eukaryotic and prokaryotic N-linked protein glycosylation

*N*-linked protein glycosylation is an essential and conserved process occurring in the endoplasmic reticulum (ER) of eukaryotic organisms (Burda et al., "The Dolichol Pathway of N-linked Glycosylation," Biochim Biophys Acta 1426:239-57 (1999)). It is important for protein folding, oligomerization, quality control, sorting, and transport of secretory and membrane proteins (Helenius et al., "Intracellular Functions of N-linked Glycans," Science 291:2364-9 (2001)). The eukaryotic *N*-linked protein glycosylation pathway can be divided into two different processes: (i) the assembly of the lipid-linked oligosaccharide at the membrane of the endoplasmic reticulum and (ii) the transfer of the oligosaccharide from the lipid anchor dolichol pyrophosphate to selected asparagine residues of nascent polypeptides. The characteristics of *N*-linked protein glycosylation, namely (i) the use of dolichol pyrophosphate (Dol-PP) as carrier for oligosaccharide assembly, (ii) the transfer of only the completely assembled Glc₃Man₉GlcNAc₂ oligosaccharide, and (iii) the recognition of asparagine residues characterized by the sequence N-X-S/T where N is asparagine, X is any amino acid except proline, and S/T is serine/threonine (Gavel et al., "Sequence Differences Between Glycosylated and Non-glycosylated Asn-X-Thr/Ser Acceptor Sites: Implications for Protein Engineering," Protein Eng 3:433-42 (1990)) are highly conserved in eukaryotes. The oligosaccharyltransferase (OST) catalyzes the transfer of the oligosaccharide from the lipid donor dolichylpyrophosphate to the acceptor protein. In yeast, eight different membrane proteins have been identified that constitute the complex *in vivo* (Kelleher et al., "An Evolving View of the Eukaryotic Oligosaccharyltransferase," Glycobiology 16:47R-62R (2006)). STT3 is thought to represent the catalytic subunit of the OST (Nilsson et al., "Photocross-linking of Nascent Chains to the STT3 Subunit of the Oligosaccharyltransferase Complex," J Cell Biol 161:715-25 (2003) and Yan et al., "Studies on the Function of Oligosaccharyl Transferase Subunits. Stt3p is Directly Involved in the Glycosylation Process," J Biol Chem 277:47692-700 (2002)). It is the most conserved subunit in the OST complex (Burda et al., "The Dolichol Pathway of N-linked Glycosylation," Biochim Biophys Acta 1426:239-57 (1999)).

Conversely, the lack of glycosylation pathways in bacteria has greatly restricted the utility of prokaryotic expression hosts for making therapeutic proteins, especially since by certain estimates "more than half of all proteins in nature will eventually be found to be glycoproteins" (Apweiler et al., "On the Frequency of Protein Glycosylation, as Deduced From Analysis of the SWISS-PROT Database," Biochim Biophys Acta 1473:4-8 (1999)). Recently, however, it was discovered that the genome of a pathogenic bacterium, *C. jejuni,* encodes a pathway for *N*-linked protein glycosylation (Szymanski et al., "Protein Glycosylation in Bacterial Mucosal Pathogens," Nat Rev Microbiol 3:225-37 (2005)). The genes for this pathway, first identified in 1999 by Szymanski and coworkers (Szymanski et al., "Evidence for a System of General Protein Glycosylation in Campylobacter jejuni," Mol Microbiol 32:1022-30 (1999)), comprise a 17-kb locus named *pgl* for protein glycosylation. Following discovery of the *pgl* locus, in 2002 Linton *et al.* identified two *C. jejuni* glycoproteins, PEB3 and CgpA, and showed that *C. jejuni-*derived glycoproteins such as these bind to the *N*-acetyl galactosamine (GalNAc)-specific lectin soybean agglutinin (SBA) (Linton et al., "Identification of N-acetylgalactosamine-containing Glycoproteins PEB3 and CgpA in Campylobacter jejuni," Mol Microbiol 43:497-508 (2002)). Shortly thereafter, Young *et al.* identified more than 30 potential *C. jejuni* glycoproteins, including PEB3 and CgbA, and used mass spectrometry and NMR to reveal that the *N*-linked glycan was a heptasaccharide with the structure GalNAc-α1,4-GaNAc-α1,4-[Glcβ1,3]GalNAc-α1,4-GalNAc-α1,4-GalNAc-α1,3-Bac-β1,*N-*Asn (GalNAc₅GlcBac, where Bac is bacillosamine or 2,4-diacetamido-2,4,6-trideoxyglucose) (Young et al., "Structure of the N-linked Glycan Present on Multiple Glycoproteins in the Gram-negative Bacterium, Campylobacter jejuni," J Biol Chem 277:42530-9 (2002)). The branched heptasaccharide is synthesized by sequential addition of nucleotide-activated sugars on a lipid carrier undecaprenylpyrophosphate (Und-PP) on the cytoplasmic side of the inner membrane (Feldman et al., "Engineering N-linked Protein Glycosylation with Diverse O Antigen Lipopolysaccharide Structures in Escherichia coli," Proc Natl Acad Sci U S A 102:3016-21 (2005)) and, once assembled, is flipped across the membrane by the putative ATP-binding cassette (ABC) transporter WlaB (Alaimo et al., "Two Distinct But Interchangeable Mechanisms for Flipping of Lipid-linked Oligosaccharides," Embo J 25:967-76 (2006) and Kelly et al., "Biosynthesis of the N-linked Glycan in Campylobacter jejuni and Addition Onto Protein Through Block Transfer," J Bacteriol 188:2427-34 (2006)). Next, transfer of the heptasaccharide to substrate proteins in the periplasm is catalyzed by an OST named PglB, a single, integral membrane protein with significant sequence similarity to the catalytic subunit of the eukaryotic OST STT3 (Young et al., "Structure of the N-linked Glycan Present on Multiple Glycoproteins in the Gram-negative Bacterium, Campylobacter jejuni," J Biol Chem 277:42530-9 (2002)). PglB attaches the heptasaccharide to asparagine in the motif D/E-X₁-N-X₂-S/T (where D/E is aspartic acid/glutamic acid, X₁ and X₂ are any amino acids except proline, N is asparagine, and S/T is serine/threonine), a sequon similar to that used in the eukaryotic glycosylation process (N-X-S/T) (Kowarik et al., "Definition of the Bacterial N-glycosylation Site Consensus Sequence," Embo J 25:1957-66 (2006)).

### Glycoengineering of microorganisms

A major problem encountered when expressing therapeutic glycoproteins in mammalian, yeast, or even bacterial host cells is the addition of non-human glycans. For instance, yeast, one of the two most frequently used systems for the production of therapeutic glycoproteins, transfer highly immunogenic mannan-type *N*-glycans (containing up to one hundred mannose residues) to recombinant glycoproteins. Mammalian expression systems can also modify therapeutic proteins with non-human sugar residues, such as the *N*-glycosylneuraminic acid (Neu5Gc) form of sialic acid (produced in CHO cells and in milk) or the terminal α(1,3)-galactose (Gal) (produced in murine cells). Repeated administration of therapeutic proteins carrying non-human sugars can elicit adverse reactions, including an immune response in humans.

As an alternative to using native glycosylation systems for producing therapeutic glycoproteins, the availability of glyco-engineered expression systems could open the door to customizing the glycosylation of a therapeutic protein and could lead to the development of improved therapeutic glycoproteins. Such a system would have the potential to eliminate undesirable glycans and perform human glycosylation to a high degree of homogeneity. The yeast *Pichia pastoris* has been glyco-engineered to provide an expression system with the capacity for glycosylation for specific therapeutic functions (Gerngross, T. U., "Advances in the Production of Human Therapeutic Proteins in Yeasts and Filamentous fungi," Nat Biotechnol 22:1409-14 (2004); Hamilton et al., "Glycosylation Engineering in Yeast: The Advent of Fully Humanized Yeast," Curr Opin Biotechnol 18:387-92 (2007); and Wildt et al., "The Humanization of N-glycosylation Pathways in Yeast," Nat Rev Microbiol 3:119-28 (2005)).

For example, a panel of glyco-engineered *P. pastoris* strains was used to produce various glycoforms of the monoclonal antibody Rituxan (an anti-CD20 IgG1 antibody) (Li et al., "Optimization of Humanized IgGs in Glycoengineered Pichia pastoris," Nat Biotechnol 24:210-5 (2006)). Although these antibodies share identical amino acid sequences to commercial Rituxan, specific glycoforms displayed ∼100-fold higher binding affinity to relevant FcyRIII receptors and exhibited improved *in vitro* human B-cell depletion (Li et al., "Optimization of Humanized IgGs in Glycoengineered Pichia pastoris," Nat Biotechnol 24:210-5 (2006)). The tremendous success and potential of glyco-engineered *P. pastoris* is not without some drawbacks. For instance, in yeast and all other eukaryotes *N*-linked glycosylation is essential for viability (Herscovics et al., "Glycoprotein Biosynthesis in Yeast," FASEB J 7:540-50 (1993) and Zufferey et al., "STT3, a Highly Conserved Protein Required for Yeast Oligosaccharyl Transferase Activity In vivo," EMBO J 14:4949-60 (1995)). Gerngross and coworkers systematically eliminated and re-engineered many of the unwanted yeast *N*-glycosylation reactions (Choi et al., "Use of Combinatorial Genetic Libraries to Humanize N-linked Glycosylation in the Yeast Pichia pastoris," Proc Natl Acad Sci USA 100:5022-7 (2003)). However, elimination of the mannan-type *N*-glycans is only half of the glycosylation story in yeast. This is because yeast also perform *O*-linked glycosylation whereby O-glycans are linked to Ser or Thr residues in glycoproteins (Gentzsch et al., "The PMT Gene Family: Protein O-glycosylation in Saccharomyces cerevisiae is Vital," EMBO J 15:5752-9 (1996)). As with *N*-linked glycosylation, O-glycosylation is essential for viability (Gentzsch et al., "The PMT Gene Family: Protein O-glycosylation in Saccharomyces cerevisiae is Vital," EMBO J 15:5752-9 (1996)) and thus cannot be genetically deleted from glyco-engineered yeast. Since there are differences between the O-glycosylation machinery of yeast and humans, the possible addition of O-glycans by glyco-engineered yeast strains has the potential to provoke adverse reactions including an immune response.

Aebi and his coworkers transferred the *C*. *jejuni* glycosylation locus into *E. coli* and conferred upon these cells the extraordinary ability to post-translationally modify proteins with *N*-glycans (Wacker et al., "N-linked Glycosylation in Campylobacter jejuni and its Functional Transfer into E. coli," Science 298:1790-3 (2002)). However, despite the functional similarity shared by the prokaryotic and eukaryotic glycosylation mechanisms, the oligosaccharide chain attached by the prokaryotic glycosylation machinery (GalNAc₅GlcBac) is structurally distinct from that attached by eukaryotic glycosylation pathways (Szymanski et al., "Protein Glycosylation in Bacterial Mucosal Pathogens," Nat Rev Microbiol 3:225-37 (2005); Young et al., "Structure of the N-linked Glycan Present on Multiple Glycoproteins in the Gram-negative Bacterium, Campylobacter jejuni," J Biol Chem 277:42530-9 (2002); and Weerapana et al., "Asparagine-linked Protein Glycosylation: From Eukaryotic to Prokaryotic Systems," Glycobiology 16:91R-101R (2006)). Numerous attempts (without success) have been made to reprogram *E. coli* with a eukaryotic *N-*glycosylation pathway to express *N*-linked glycoproteins with structurally homogeneous human-like glycans.

More recently, Vaderrama-Rincon et al. "An engineered eukaryotic protein glycosylation pathway in E. coli," Nat Chem Bio 8, 434-436 (2012) showed that prokaryotic host cells can be glycoengineered with eukaryotic glycosyltransferases. Specifically, expression of UDP-GlcNAc transferases and GDP-mannose transferases in a prokaryotic host cell demonstrated the production of the trimannosyl core structure, which is the basis of nearly all eukaryotic *N*-linked oligosaccharide structures. Fully elaborated human-like glycans, however, still require additional glycoengineering.

The present invention, therefore, is directed to producing human-like glycans such as high-mannose, hybrid and complex types.

### SUMMARY OF THE INVENTION

The invention provides methods and materials for the production of oligosaccharide compositions and for the production of recombinant glycoproteins in prokaryotic host cells as described in the claims. Various glycoprotein compositions comprising specific N-glycans are produced using the methods of the disclosure. In certain embodiments, desired glycoforms are produced as the predominant species.

The disclosure also provides methods and materials for the production of vaccines antigens comprising specific oligosaccharide compositions, for example, to induce immunity or immunological tolerance (e.g., anergy) within a subject. Various aspects of the present disclosure are directed to antigen-carbohydrate conjugates able to bind lectins expressable on the surfaces of dendritic cell and/or other antigen-presenting cell.

A first aspect of the invention relates to a method of producing an oligosaccharide composition, said method comprising: culturing a recombinant prokaryotic host cell that produces an oligosaccharide composition having a terminal mannose residue to express one or more *N*-acetylglucosaminyl transferase enzyme activity (EC 2.4.1.101; EC 2.4.1.143; EC 2.4.1.145; EC 2.4.1.155; EC 2.4.1.201) that catalyzes the transfer of a UDP-GlcNAc residue onto said terminal mannose residue, said culturing step carried out under conditions effective to produce an oligosaccharide composition having a terminal GlcNAc residue.

A second aspect of the invention relates to a method of producing an oligosaccharide composition, said method comprising: culturing a host cell to express one or more galactosyltransferase enzyme activity (EC 2.4.1.38) that catalyzes the transfer of a UDP-Galactose residue onto said terminal GlcNAc residue, said culturing step carried out under conditions effective to produce an oligosaccharide composition having a terminal galactose residue.

A third aspect of the invention relates to a method of producing an oligosaccharide composition, said method comprising: culturing the host cell to express one or more sialyltransferase enzyme activity (EC 2.4.99.4 and EC 2.4.99.1) that catalyzes the transfer of a CMP-NANA residue onto said terminal galactose residue, said culturing step carried out under conditions effective to produce an oligosaccharide composition having a terminal sialic acid residue.

Other aspects of the disclosure relates to expression of one or more of the enzymes as solubility enhanced fusion proteins. Further aspects of the invention include transfer of the glycans onto a gene encoding a protein of interest, whereby the host cell produces a glycosylated protein.

Additional aspects include culturing conditions and overexpression of additional enzymes for the production of predominant glycoforms as described in the claims. Featured aspects of the invention provide prokaryotic host cells to express various glycosyltransferase activities to produce high-mannose, hybrid and/or complex oligosaccharide compositions as well as high-mannose, hybrid and/or complex glycosylated as described in the claims.

In preferred aspects, the present disclosure commercializes technologies for the design, discovery, and development of glycoprotein therapeutics and diagnostics. Specifically, the present disclosure provides for the development of an efficient, low-cost strategy for efficient production of authentic human glycoproteins in microbial cells. In various aspects, the glyco-engineered bacteria of the invention are capable of stereospecific production of *N*-linked glycoproteins. In one embodiment, bacteria are transformed with genes encoding a novel glycosylation pathway that is capable of efficiently glycosylating target proteins at specific asparagine acceptor sites (e.g., *N-*linked glycosylation). Using these specially engineered cell lines, various recombinant protein-of-interest can be expressed and glycosylated.

Further, the disclosure provides methods for engineering permutations of oligosaccharide structures in prokaryotes, which is expected to alter e.g., pharmacokinetic properties of proteins and elucidate the role of glycosylation in biological phenomena. In various aspects, the disclosure provides biotechnological synthesis of therapeutic proteins, novel glycoconjugates, immunostimulating agents (e.g., vaccines) for research, industrial, and therapeutic applications.

### BRIEF DESCRIPTION OF THE DRAWINGS

**FIG. 1****. Production of a high-mannose type Man₅GlcNAc₂ glycoform.** MALDI-TOF mass spectra of lipid-released glycans (A) extracted from GLY02 consistent with the expected Man₅GlcNAc₂ (m/z 1257.6) glycoform and (B) further treated with an α1,2-mannosidase consistent with the expected Man₃GlcNAc₂ glycoform (m/z 933.4).
**FIG. 2****. Production of a hybrid GlcNAcMan₃GlcNAc₂ glycoform.** MALDI-TOF mass spectra of lipid-released glycans (A) extracted from GLY03 consistent with the expected GlcNAcMan₃GlcNAc₂ glycoform (m/z 1136.5) and (B) further treated with a β-N-acetylglucosaminidase consistent with the expected Man₃GlcNAc₂ glycoform (m/z 933.5).
**FIG. 3****. Production of a complex GlcNAc₂Man₃GlcNAc₂ glycoform.** MALDI-TOF mass spectrum of lipid-released glycans extracted from GLY06.1 consistent with the expected GlcNAc₂Man₃GlcNAc₂ glycform (m/z 1339.8).
**FIG. 4****. Production of a hybrid, branched glycoform.** MALDI-TOF mass spectra of lipid-released glycans (A) extracted from GLY05 consistent with the expected GlcNAc₂Man₃GlcNAc₂ glycoform (m/z 1339.7) and (B) further treated with a β-N-acetylglucosaminidase consistent with the expected Man₃GlcNAc₂ glycoform (m/z 933.5).
**FIG. 5****. Production of a multiple-antennary glyoform.** MALDI-TOF mass spectrum of (A) glycans synthesized *ex vivo* and (B) lipid-released glycans extracted from GLY06.1 consistent with the expected GlcNAc₃Man₃GlcNAc₂ (m/z 1543.1).
**FIG. 6****. Production of a GalGlcNAcMan₃GlcNAc₂ glycoform.** MALDI-TOF mass spectrum of lipid-released glycans extracted from GLY04.1 consistent with the expected GalGlcNAcMan₃GlcNAc₂ glycoform (m/z 1298.7).
**FIG. 7****. Production of a Gal₂GlcNAc₂Man₃GlcNAc₂ glycoform.** MALDI-TOF mass spectrum of (A) glycans synthesized *ex vivo* and (B) lipid-released glycans extracted from GLY04.2 consistent with expected Gal₂GlcNAc₂Man₃GlcNAc₂ (m/z 1662.2).
**FIG. 8****. Production of a NANAGalGlcNAcMan₃GlcNAc₂ glycoform.** MALDI-TOF mass spectrum in positive ion mode of glycans synthesized *ex vivo* consistent with the expected NANAGalGlcNAcMan₃GlcNAc₂ (m/z 1565.7).

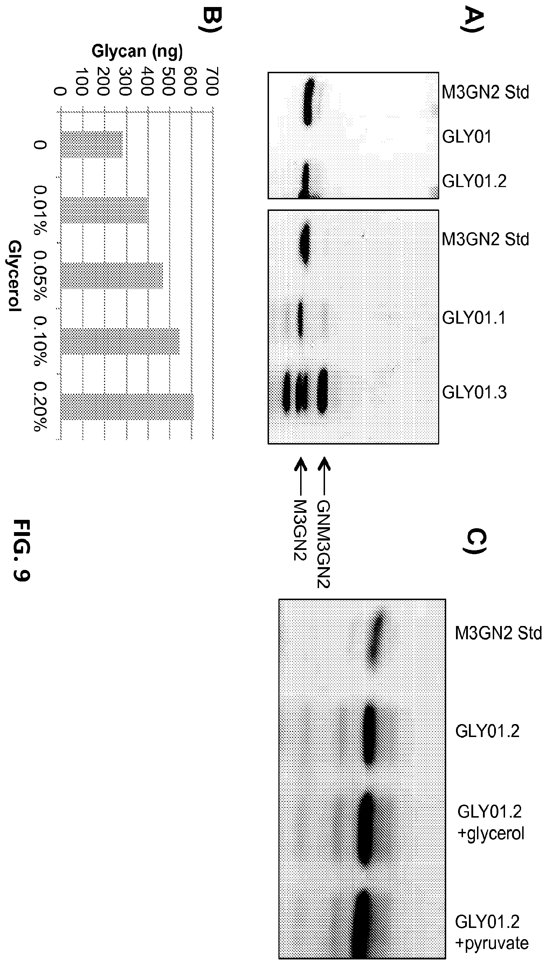

**FIG. 9****. Increased glycan yield.** (A) Fluorophore-assisted carbohydrate electrophoresis (FACE) of lipid-released glycan extracted from *E. coli* ran with a Man₃GlcNAc₂ glycan standard (M3GN2 Std): with (GLY01.2) or without (GLY01) overexpression of ManC/B (left) consistent with the Man₃GlcNAc₂ glycoform and with (GLY01.1) or without (GLY01.3) the overexpression of GlmS (right) consistent with the GlcNAcMan₃GlcNAc₂ glycoform (GNM3GN2). (B) Quantity of lipid-released glycan extracted from GLY01.2 with overexpression of ManC/B and glycerol supplementation, as indicated. (C) FACE of lipid-released glycan extracted from GLY01.2 with either 0.2% glycerol or pyruvate supplementation.
**FIG. 10****. Increased product formation.** MADLI-TOF mass spectra of lipid-released glycans extracted from strain (A) GLY01, (B) GLY02, and (C) GLY01.1 without overexpression of ManC/B and (D) GLY01.4, (E) GLY02.1, and (F) GLY01.5 with overexpression of ManC/B. The loss of peaks corresponding to intermediate glycoforms was observed with the addition of ManC/B.
**FIG. 11****. Glycosylated glucagon production.** MALDI-TOF MS of partially purified glucagon appended with a C-terminal glycosylation site from various glycoengineered strains, which produce M3, M5, GlcNAcMan₃GlcNAc₂, and GalGlcNAcMan₃GlcNAc₂ glycopeptides.
**FIG. 12****. Glycosylated antigens.** Western blot of partially purified (A) 3473 and (B) 1275 proteins originally from extraintestinal pathogenic *E. coli* (ExPEC) appended with four consecutive C-terminal glycosylation sites and expressed in GLY01 detected with anti-hexahistidine antibody (left) and the Concanavalin A lectin specific for terminal alpha-mannose (right).

### DETAILED DESCRIPTION OF THE INVENTION

### Definitions

The following definitions of terms and methods are provided to better describe the present disclosure and to guide those of ordinary skill in the art in the practice of the present disclosure.

EC numbers are established by the Nomenclature Committee of the International Union of Biochemistry and Molecular Biology (NC-IUBMB) (available at http://www.chem.qmul.ac.uk/iubmb/enzyme/). The EC numbers referenced herein are derived from the KEGG Ligand database, maintained by the Kyoto Encyclopedia of Genes and Genomics, sponsored in part by the University of Tokyo. Unless otherwise indicated, the EC numbers are as provided in the database as of March 2013.

The accession numbers referenced herein are derived from the NCBI database (National Center for Biotechnology Information) maintained by the National Institute of Health, U.S.A. Unless otherwise indicated, the accession numbers are as provided in the database as of March 2013.

The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989); Ausubel et al., Current Protocols in Molecular Biology, Greene Publishing Associates (1992, and Supplements to 2002); Harlow and Lane, Antibodies: A Laboratory Manual, Cold Spring Harbor Laboratory Press, Cold Spring Harbor, N.Y. (1990); Taylor and Drickamer, Introduction to Glycobiology, Oxford Univ. Press (2003); Worthington Enzyme Manual, Worthington Biochemical Corp., Freehold, N.J.; Handbook of Biochemistry: Section A Proteins, Vol I, CRC Press (1976); Handbook of Biochemistry: Section A Proteins, Vol II, CRC Press (1976); Essentials of Glycobiology, Cold Spring Harbor Laboratory Press (1999).

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting. Other features of the disclosure are apparent from the following detailed description and the claims.

The term "claim" in the provisional application is synonymous with embodiments or preferred embodiments.

As used herein, "comprising" means "including" and the singular forms "a" or "an" or "the" include plural references unless the context clearly dictates otherwise. For example, reference to "comprising a cell" includes one or a plurality of such cells. The term "or" refers to a single element of stated alternative elements or a combination of two or more elements, unless the context clearly indicates otherwise.

The term "human-like" with respect to a glycoproteins refers to proteins having attached *N*-acetylglucosamine (GlcNAc) residue linked to the amide nitrogen of an asparagine residue (*N*-linked) in the protein, that is similar or even identical to those produced in humans.

"*N*-glycans" or "*N*-linked glycans" refer to *N*-linked oligosaccharide structures. The *N*-glycans can be attached to proteins or synthetic glycoprotein intermediates, which can be manipulated further *in vitro* or *in vivo.* The predominant sugars found on glycoproteins are glucose (Glu), galactose (Gal), mannose (Man), fucose (Fuc), *N*-acetylgalactosamine (GalNAc), *N*-acetylglucosamine (GlcNAc), and sialic acid (e.g., *N*-acetyl-neuraminic acid (NeuAc or NANA). Hexose (Hex) may also be found. *N*-glycans differ with respect to the number of branches ("antennae" or "arms") comprising peripheral sugars (e.g., GlcNAc, galactose, fucose and sialic acid) that are added to the "triamannosyl core". The term "triamannosyl core", also referred to as "M3", "M3GN2", the "triamannose core", the "pentasaccharide core" or the "paucimannose core" reflects Man₃GlcNAc₂ oligosaccharide structure where Manα1,3 arm and the Manα1,6 arm extends from the di-GlcNAc structure (GlcNAc₂): β1,4GlcNAc-β1,4GlcNAc. *N*-glycans are classified according to their branched constituents (e.g., high-mannose, complex or hybrid).

A "high-mannose" type *N*-glycan comprises four or more mannose residues on the di-GlcNAc oligosaccharide structure. "M4" reflects Man₄GlcNAc₂. "M5" reflects Man₅GlcNAc₂

A "hybrid" type *N*-glycan has at least one GlcNAc residue on the terminal end of the α1,3 mannose (Man α1,3) arm of the trimannose core and zero or more mannoses on the α1,6 mannose (Man α1,3) arm of the trimannose core. The various *N*-glycans are also referred to as "glycoforms". An example of a hybrid glycan is "GNM3GN2", which is GlcNAcMan₃GlcNAc₂.

A "complex" type *N*-glycan typically has at least one GlcNAc residue attached to the Manα1,3 arm and at least one GlcNAc attached to the Manα1,6 arm of the trimannose core. Complex *N*-glycans may also have galactose or *N-*acetylgalactosamine residues that are optionally modified with sialic acid or derivatives (e.g., "Neu" refers to neuraminic acid and "Ac" refers to acetyl). Complex *N*-glycans may also have intrachain substitutions comprising "bisecting" GlcNAc and core fucose. Complex *N*-glycans may also have multiple antennae on the trimannose core, often referred to as "multiple antennary glycans" or also termed "multi-branched glycans," which can be tri-antennary tetra-antennary or penta-antennary glycans.

The term "G0" refers to GlcNAc₂Man₃GlcNAc₂. The term "G0(1)" refers to GlcNAc₃Man₃GlcNAc₂, the term "G0(2)" refers to GlcNAc₄Man₃GlcNAc₂ and the term "G0(3)" refers to GlcNAc₅Man₃GlcNAC₂. The terms "G1" refers to GalGlcNAc₂Man₃GlcNAc₂, "G2" refers to Gal₂GlcNAc₂Man₃GlcNAc₂, "G3" refers to Gal₃GlcNAC₃₋₅Man₃GlcNAc₂, "G4" refers to Gal₄GlcNAC₄₋₅Man₃GlcNAC₂, "G5" refers to Gal₅GlcNAC₅Man₃GlcNAC₂. The terms "S1" refers to NANAGal₁₋₅GlcNAc₁₋₅Man₃GlcNAc₂, "S2" refers to NANA₂Gal₂₋₅GlcNAc₂₋₅Man₃GlcNAc₂. "S3" refers to NANA₃Gal₃₋₅GlcNAc₃₋₅Man₃GlcNAc₂, "S4" refers to NANA₄Gal₄₋₅GlcNAC₄₋₅Man₃GlcNAc₂, "S5" refers to NANA₅Gal₅GlcNAc₅Man₃GlcNAc₂.

As used herein, the term "predominantly" or variations such as "the predominant" or "which is predominant" will be understood to mean the glycan species as measured that has the highest mole percent (%) of total *N*-glycans after the glycoprotein has been removed (e.g., treated with PNGase and the glycans released) and are analyzed by mass spectroscopy, for example, MALDI-TOF MS. In other words, the phrase "predominantly" is defined as an individual entity, such as a specific glycoform, present in greater mole percent than any other individual entity. For example, if a composition consists of species A in 40 mole percent, species B in 35 mole percent and species C in 25 mole percent, the composition comprises predominantly species A. The term "enriched", "uniform", "homogenous" and "consisting essentially of' are also synonymous with predominant in reference to the glycans.

The mole % of *N*-glycans as measured by MALDI-TOF-MS in positive mode refers to mole % saccharide transfer with respect to mole % total N-glycans. Certain cation adducts such as K+ and Na+ are normally associated with the peaks eluted increasing the mass of the *N*-glycans by the molecular mass of the respective adducts.

Unless otherwise indicated, and as an example for all sequences described herein under the general format "SEQ ID NO:", "nucleic acid comprising SEQ ID NO:1" refers to a nucleic acid, at least a portion of which has either (i) the sequence of SEQ ID NO:1, or (ii) a sequence complementary to SEQ ID NO:1. The choice between the two is dictated by the context. For instance, if the nucleic acid is used as a probe, the choice between the two is dictated by the requirement that the probe be complementary to the desired target.

An "isolated" or "substantially pure" nucleic acid or polynucleotide (e.g., RNA, DNA, or a mixed polymer) or glycoprotein is one which is substantially separated from other cellular components that naturally accompany the native polynucleotide in its natural host cell, e.g., ribosomes, polymerases and genomic sequences with which it is naturally associated. The term embraces a nucleic acid, polynucleotide that (1) has been removed from its naturally occurring environment, (2) is not associated with all or a portion of a polynucleotide in which the "isolated polynucleotide" is found in nature, (3) is operatively linked to a polynucleotide which it is not linked to in nature, or (4) does not occur in nature. The term "isolated" or "substantially pure" also can be used in reference to recombinant or cloned DNA isolates, chemically synthesized polynucleotide analogs, or polynucleotide analogs that are biologically synthesized by heterologous systems.

However, "isolated" does not necessarily require that the nucleic acid, polynucleotide or glycoprotein so described has itself been physically removed from its native environment. For instance, an endogenous nucleic acid sequence in the genome of an organism is deemed "isolated" if a heterologous sequence is placed adjacent to the endogenous nucleic acid sequence, such that the expression of this endogenous nucleic acid sequence is altered. In this context, a heterologous sequence is a sequence that is not naturally adjacent to the endogenous nucleic acid sequence, whether or not the heterologous sequence is itself endogenous (originating from the same host cell or progeny thereof) or exogenous (originating from a different host cell or progeny thereof). By way of example, a promoter sequence can be substituted (e.g., by homologous recombination) for the native promoter of a gene in the genome of a host cell, such that this gene has an altered expression pattern. This gene would now become "isolated" because it is separated from at least some of the sequences that naturally flank it.

A nucleic acid is also considered "isolated" if it contains any modifications that do not naturally occur to the corresponding nucleic acid in a genome. For instance, an endogenous coding sequence is considered "isolated" if it contains an insertion, deletion, or a point mutation introduced artificially, e.g., by human intervention. An "isolated nucleic acid" also includes a nucleic acid integrated into a host cell chromosome at a heterologous site and a nucleic acid construct present as an episome. Moreover, an "isolated nucleic acid" can be substantially free of other cellular material or substantially free of culture medium when produced by recombinant techniques or substantially free of chemical precursors or other chemicals when chemically synthesized.

As used herein, the term "therapeutically effective amount" of a therapeutic protein refers to an amount sufficient to cure, alleviate or partially arrest the clinical manifestations of a given disease and/or its complications. An amount adequate to accomplish this is defined as a "therapeutically effective amount". Effective amounts for each purpose will depend on the severity of the disease or injury, as well as on the weight and general state of the subject. It will be understood that determination of an appropriate dosage may be achieved using routine experimentation, by constructing a matrix of values and testing different points in the matrix, all of which is within the level of ordinary skill of a trained physician or veterinarian.

The terms "treatment", "treating" and other variants thereof as used herein refer to the management and care of a patient or subject for the purpose of combating a condition, such as a disease or a disorder. The terms are intended to include the full spectrum of treatments for a given condition from which the patient is suffering, such as administration of the active compound(s) in question to alleviate symptoms or complications thereof, to delay the progression of the disease, disorder or condition, to cure or eliminate the disease, disorder or condition, and/or to prevent the condition, in that prevention is to be understood as the management and care of a patient for the purpose of combating the disease, condition, or disorder, and includes the administration of the active compound(s) in question to prevent the onset of symptoms or complications. The patient to be treated is preferably a mammal, in particular a human being, but treatment of other animals, such as dogs, cats, cows, horses, sheep, goats or pigs, is within the scope of the disclosure.

For example, a therapeutically effective amount of glucagon peptide of the present invention for a patient suffering from insulin coma or insulin reaction resulting from severe hypoglycemia (low blood sugar) is 1mg (1unit) for an adult. For children weighing less than 44lb (20 kg), it is 0.5mg. Glucagon is given if (1) the patient is unconscious, (2) the patient is unable to eat sugar or a sugar-sweetened product, (3) the patient is having a seizure, or (4) repeated administration of sugar or a sugar-sweetened product such as a regular soft drink or fruit juice does not improve the patient's condition. In other instances, the dose can be in the range of 0.25 units to 2 units, which can be administered intramuscular or intravenously. A milligram of pure glucagon is approximately equivalent to 1 unit. A dosing schedule can vary but can be from about once a day to as needed per event. The actual schedule will depend on a number of factors including the type of glucagon administered to a patient (glucagon or glycosylated-glucagon) and the response of the individual patient. The higher dose ranges are not typically used in hypoglycemia applications but may be useful on other therapeutic applications. The means of achieving and establishing an appropriate dose for a patient is well known and commonly practiced in the art.

As used herein, the term "pharmaceutically acceptable" is given its ordinary meaning. Pharmaceutically acceptable compositions are generally compatible with other materials of the formulation and are not generally deleterious to the subject.

Any of the compositions of the present invention may be administered to the subject in a therapeutically effective dose. For vaccines, a "therapeutically effective" or an "effective" amount or dose, as used herein means that amount necessary to induce immunity or tolerance within the subject, and/or to enable the subject to more effectively resist a disease (e.g., against foreign pathogens, cancer, an autoimmune disease, etc.). When administered to a subject, effective amounts will depend on the particular condition being treated and the desired outcome. A therapeutically effective dose may be determined by those of ordinary skill in the art, for instance, employing factors such as those further described below and using no more than routine experimentation.

In some embodiments, a therapeutically effective amount can be initially determined from cell culture assays. For instance the effective amount of a composition of the invention useful for inducing dendritic cell response can be assessed using the *in vitro* assays with respect to a stimulation index. The stimulation index can be used to determine an effective amount of a particular composition of the invention for a particular subject, and the dosage can be adjusted upwards or downwards to achieve desired levels in the subject. Therapeutically effective amounts can also be determined from animal models. The applied dose can be adjusted based on the relative bioavailability and potency of the administered composition. Adjusting the dose to achieve maximal efficacy based on the methods described above and other methods are within the capabilities of those of ordinary skill in the art. These doses can be adjusted using no more than routine experimentation.

In administering the compositions of the invention to a subject, dosing amounts, dosing schedules, routes of administration, and the like may be selected so as to affect known activities of these compositions. Dosages may be estimated based on the results of experimental models, optionally in combination with the results of assays of compositions of the present invention. Dosage may be adjusted appropriately to achieve desired compositional levels, local or systemic, depending upon the mode of administration. The doses may be given in one or several administrations per day. In the event that the response of a particular subject is insufficient at such doses, even higher doses (or effectively higher doses by a different, more localized delivery route) may be employed to the extent that subject tolerance permits. Multiple doses per day are also contemplated in some cases to achieve appropriate systemic levels of the composition within the subject or within the active site of the subject.

The dose of the composition to the subject may be such that a therapeutically effective amount of the composition reaches the active site of the composition within the subject, i.e., dendritic cells and/or other antigen-presenting cells within the body. The dosage may be given in some cases at the maximum amount while avoiding or minimizing any potentially detrimental side effects within the subject. The dosage of the composition that is actually administered is dependent upon factors such as the final concentration desired at the active site, the method of administration to the subject, the efficacy of the composition, the longevity of the composition within the subject, the timing of administration, the effect of concurrent treatments (e.g., as in a cocktail), etc. The dose delivered may also depend on conditions associated with the subject, and can vary from subject to subject in some cases. For example, the age, sex, weight, size, environment, physical conditions, or current state of health of the subject may also influence the dose required and/or the concentration of the composition at the active site. Variations in dosing may occur between different individuals or even within the same individual on different days. It may be preferred that a maximum dose be used, that is, the highest safe dose according to sound medical judgment. Preferably, the dosage form is such that it does not substantially deleteriously affect the subject. In certain embodiments, the composition may be administered to a subject as a preventive measure. In some embodiments, the inventive composition may be administered to a subject based on demographics or epidemiological studies, or to a subject in a particular field or career.

Administration of a composition of the invention may be accomplished by any medically acceptable method, which allows the composition to reach its target, i.e., dendritic cells and/or other antigen-presenting cells within the body. The particular mode selected will depend of course, upon factors such as those previously described, for example, the particular composition, the severity of the state of the subject being treated, the dosage required for therapeutic efficacy, etc. As used herein, a "medically acceptable" mode of treatment is a mode able to produce effective levels of the composition within the subject without causing clinically unacceptable adverse effects.

Any medically acceptable method may be used to administer the composition to the subject. The administration may be localized (i.e., to a particular region, physiological system, tissue, organ, or cell type) or systemic, depending on the condition to be treated. For example, the composition may be administered pulmonary, nasally, transdermally, through parenteral injection or implantation, via surgical administration, or any other method of administration where access to the target by the composition of the invention is achieved. Examples of parenteral modalities that can be used with the invention include intravenous, intradermal, subcutaneous, intracavity, intramuscular, intraperitoneal, epidural, or intrathecal. Examples of implantation modalities include any implantable or injectable drug delivery system.

In certain embodiments of the disclosure, the administration of the composition of the invention may be designed so as to result in sequential exposures to the composition over a certain time period, for example, hours, days, weeks, months or years. This may be accomplished, for example, by repeated administrations of a composition of the invention by one of the methods described above, or by a sustained or controlled release delivery system in which the composition is delivered over a prolonged period without repeated administrations. Administration of the composition using such a delivery system may be, for example, by oral dosage forms, bolus injections, transdermal patches or subcutaneous implants. Maintaining a substantially constant concentration of the composition may be preferred in some cases.

The composition may also be administered on a routine schedule, but alternatively, may be administered as symptoms arise. A "routine schedule" as used herein, refers to a predetermined designated period of time. The routine schedule may encompass periods of time which are identical or which differ in length, as long as the schedule is predetermined. For instance, the routine schedule may involve administration of the composition on a daily basis, every two days, every three days, every four days, every five days, every six days, a weekly basis, a bi-weekly basis, a monthly basis, a bimonthly basis or any set number of days or weeks there-between, every two months, three months, four months, five months, six months, seven months, eight months, nine months, ten months, eleven months, twelve months, etc. Alternatively, the predetermined routine schedule may involve administration of the composition on a daily basis for the first week, followed by a monthly basis for several months, and then every three months after that. Any particular combination would be covered by the routine schedule as long as it is determined ahead of time that the appropriate schedule involves administration on a certain day.

In some cases, the composition is administered to the subject in anticipation of an allergic event in order to prevent an allergic event. The allergic event may be, but need not be limited to, an asthma attack, seasonal allergic rhinitis (e.g., hay-fever, pollen, ragweed hypersensitivity) or perennial allergic rhinitis (e.g., hypersensitivity to allergens such as those described herein). In some instances, the composition is administered substantially prior to an allergic event. As used herein, "substantially prior" means at least six months, at least five months, at least four months, at least three months, at least two months, at least one month, at least three weeks, at least two weeks, at least one week, at least 5 days, or at least 2 days prior to the allergic event.

Similarly, the composition may be administered immediately prior to an allergic event (e.g., within 48 hours, within 24 hours, within 12 hours, within 6 hours, within 4 hours, within 3 hours, within 2 hours, within 1 hour, within 30 minutes or within 10 minutes of an allergic event), substantially simultaneously with the allergic event (e.g., during the time the subject is in contact with the allergen or is experiencing the allergy symptoms) or following the allergic event. In order to desensitize a subject to a particular allergen, the conjugate containing that antigen or allergen may be administered in very small doses over a period of time, consistent with traditional desensitization therapy.

Other delivery systems suitable for use with the present invention include time-release, delayed release, sustained release, or controlled release delivery systems. Such systems may avoid repeated administrations of the composition in many cases, increasing convenience to the subject. Many types of release delivery systems are available and known to those of ordinary skill in the art. They include, for example, polymer-based systems such as polylactic and/or polyglycolic acids, polyanhydrides, polycaprolactones and/or combinations of these; nonpolymer systems that are lipid-based including sterols such as cholesterol, cholesterol esters, and fatty acids or neutral fats such as mono-, di- and triglycerides; hydrogel release systems; liposome-based systems; phospholipid based-systems; silastic systems; peptide based systems; wax coatings; compressed tablets using conventional binders and excipients; or partially fused implants. The formulation may be as, for example, microspheres, hydrogels, polymeric reservoirs, cholesterol matrices, or polymeric systems. In some embodiments, the system may allow sustained or controlled release of the composition to occur, for example, through control of the diffusion or erosion/degradation rate of the formulation containing the composition. In addition, a pump-based hardware delivery system may be used to deliver one or more embodiments of the invention.
Use of a long-term release device may be particularly suitable in some embodiments of the invention. "Long-term release," as used herein, means that a device containing the composition is constructed and arranged to deliver therapeutically effective levels of the composition for at least 30 or 45 days, and preferably at least 60 or 90 days, or even longer in some cases. Long-term release implants are well known to those of ordinary skill in the art, and include some of the release systems described above.

### Glycosylation Engineering

Using the novel expression system and methods as provided herein, various aspects of the invention are provided for the production of high-mannose, hybrid and complex glycans through glycoengineering of prokaryotic host cells as described in the claims. One aspect of the present invention relates to a recombinant prokaryotic host comprising a biosynthetic pathway to express *N*-linked glycoproteins with structurally homogeneous human-like glycans as described in the claims. Applications of the present disclosure include improved biochemical and pharmacokinetic stability for therapeutic proteins. Additional embodiments provide methods and compositions for producing carbohydrate-conjugated vaccines capable of eliciting protective immunity in subjects. A rapid, microbial-based manufacturing process to produce safe and more effective glycoproteins and vaccines is an object of the present disclosure.

### High-Mannose Type Glycan Production in Prokaryotes

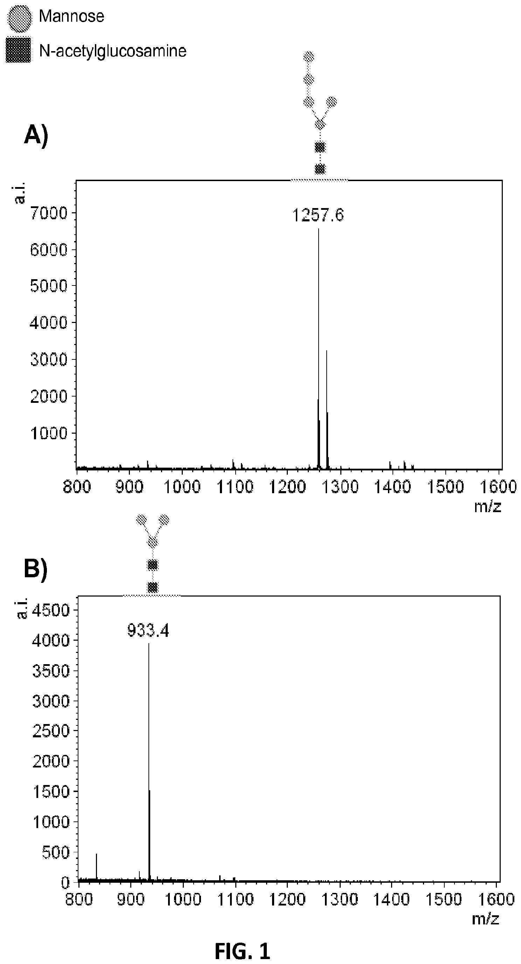

Building from the trimannosyl core, the present disclosure provides methods for the recombinant expression of a mannosyltransferase enzyme to produce a high-mannose type glycan as shown in **FIG. 1****.** In one embodiment, the method provides culturing a recombinant prokaryotic host cell to express one or more alpha-1,2-mannosyltransferase enzyme activities (EC 2.4.1.131) that catalyzes the transfer of two GDP-Mannose residues onto a trimannose oligosaccharide composition in a prokaryotic host cell. **Example 3** describes expression of a α-1,2-mannosyltransferase enzyme activity (EC 2.4.1.131). Preferred α-1,2-mannosyltransferase enzyme activity is encoded by a *S*. *cerevisiae alg11* fused to GST, a solubility enhancer. **Table I** lists a variety of solubility enhancers.

Accordingly, the disclosure provides a method of producing a high-mannose type oligosaccharide composition, said method comprising: culturing a recombinant prokaryotic host cell that produces an oligosaccharide composition having a terminal mannose residue to express one or more alpha-1,2-mannosyltransferase enzyme activity (EC 2.4.1.131) that catalyzes the transfer of a GDP-Mannose residue onto the terminal mannose residue, said culturing step carried out under conditions effective to produce an oligosaccharide composition having at least 4 terminal mannose residues. In certain embodiments, the oligosaccharide composition comprises at least 2 additional mannose residues on the trimannose core. In preferred embodiments, vaccine candidates are recombinantly expressed in the prokaryotic host cell where they are *N*-linked to the M5 glycoform. The expected structure of the major glycoform shown in **FIG. 1** is Manα1-2 Manα1-2Manα1-3(Manα1-6)-Manβ1-4-GlcNAcβ1-4-GlcNAc.

In the prokaryotic host cell of the disclosure, the glycosylation enzymes act on lipid-linked glycans prior to the glycosylation of the glycoprotein. In eukaryotes, the alpha-1,2-mannosyltransferase acts on the trimannose core glycan linked to dolichol pyrophosphate on the cytosolic side of the endoplasmic reticulum membrane. The Man₅GlcNAc₂-dolichol pyrophosphate is then flipped into the endoplasmic reticulum by an endogenous flippase enzyme that is highly specific for Man₅GlcNAc₂-dolichol pyrophosphate to ensure the complete assembly of the oligosaccharide prior to flipping (Sanyal & Menon, PNAS 2009). In prokaryotes, it has been shown that the Man₃GlcNAc₂ lipid can be flipped (Valderrama-Rincon, et. al. "An engineered eukaryotic protein glycosylation pathway in Escherichia coli," Nat. Chem. Biol. AOP (2012)) and there is no known specificity for flipping, jeopardizing assembly of the oligosaccharide beyond the trimannose core. Therefore, it is an object of the invention to produce a high-mannose type oligosaccharide composition including Man₇₋₉GlcNAc₂, Man₆GlcNAc, Man₅GlcNAc₂ and Man₄GlcNAc₂ in a prokaryotic system that transfers mannose residues onto the M3 oligosaccharide substrates and, furthermore, catalyzes the flipping activity of the oligosaccharides into the periplasm. In preferred embodiments, the host cell produces 50 mole % or more of the high-mannose type glycans.

### GnT Expression in Prokaryotes

In certain aspects, a method is provided for producing an oligosaccharide composition, said method comprising: culturing a recombinant prokaryotic host cell that produces an oligosaccharide composition having a terminal mannose residue to express one or more *N*-acetylglucosaminyl transferase enzyme activity (EC 2.4.1.101; EC 2.4.1.143; EC 2.4.1.145) that catalyzes the transfer of a UDP-GlcNAc residue onto said terminal mannose residue, said culturing step carried out under conditions effective to produce an oligosaccharide composition having a terminal GlcNAc residue. In eukaryotes, *N*-acetylglucosaminyl transerases act on oligosaccharides that are covalently linked to asparagine residues of glycosylated proteins. In prokaryotes, oligosaccharides are produced independently of the protein glycosylation process jeopardizing the production of hybrid and complex oligosaccharides.

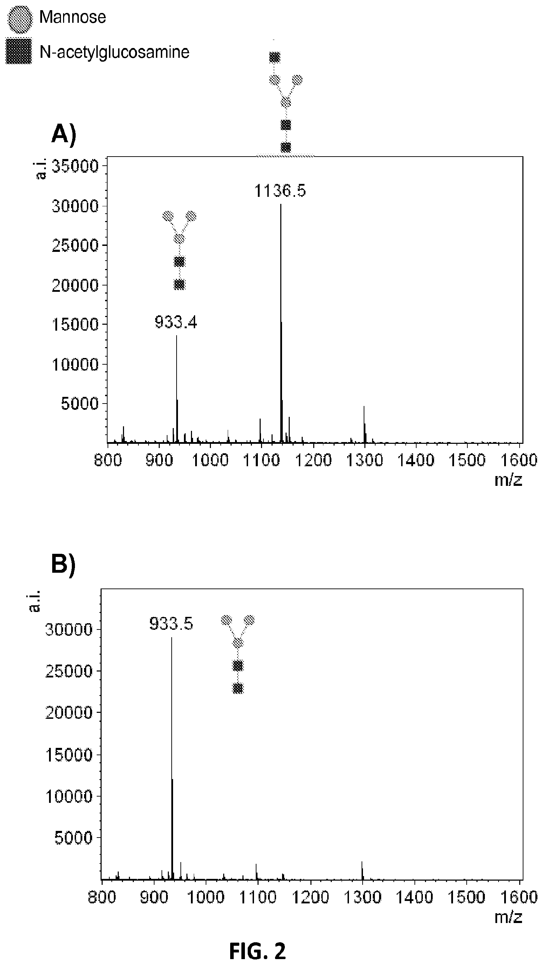

To produce a hybrid glycoform, UDP-GlcNAc residue is transferred onto the Manα1,3 arm of the trimannosyl core oliogosaccharide structure, the acceptor substrate. In an exemplary embodiment, the disclosure provides a prokaryotic host cell transformed with a gene encoding *N. tabacum* GnTI fused to MBP a solubility enhancer in a host cell expressing Alg13, Alg14, Alg1 and Alg2. A hybrid glycoform GlcNAcMan₃GlcNAc₂ is produced as shown in **FIG. 2A****.** The expected structure of the glycoform shown is β1-2-GlcNAcManα1-3(Manα1-6)-Manβ1-4-GlcNAcβ1-4-GlcNAc.

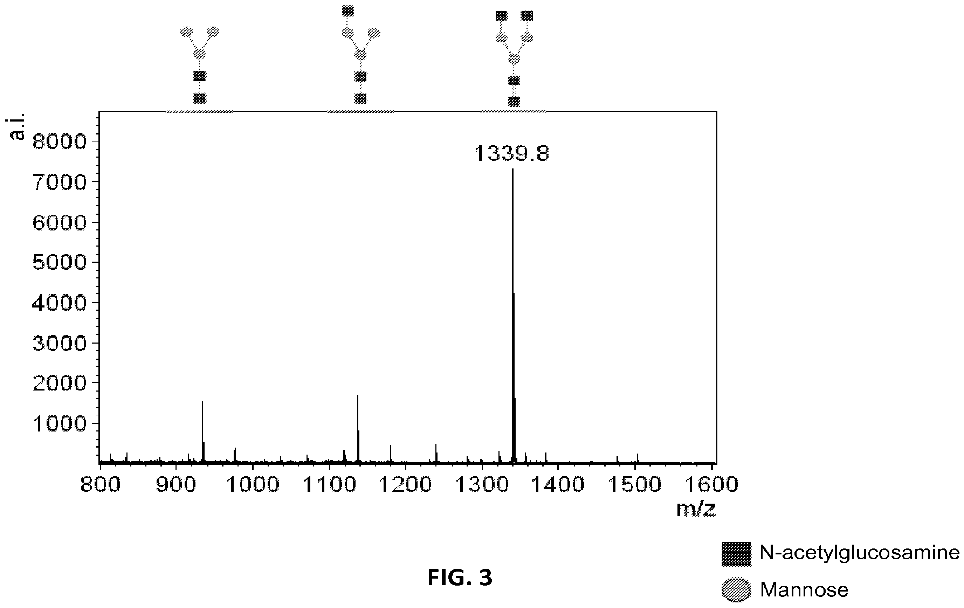

To produce a complex glycoform, UDP-GlcNAc residue is transferred onto both the Manα1,3 and Manα1,6 arm of the trimannosyl core oliogosaccharide structure, the acceptor substrate. In this embodiment, a prokaryotic host cell is transformed with a gene encoding human GnTII fused to MBP in a host cell expressing Alg13, Alg14, Alg1, Alg2 and GnTI. A complex GlcNAc₂Man₃GlcNAc₂ (G0) glycoform is produced as shown in **FIG. 3** and the expected structure is β1-2-GlcNAcManα1-3(β1-2-GlcNAc Manα1-6)-Manβ1-4-GlcNAcβ1-4-GlcNAc.

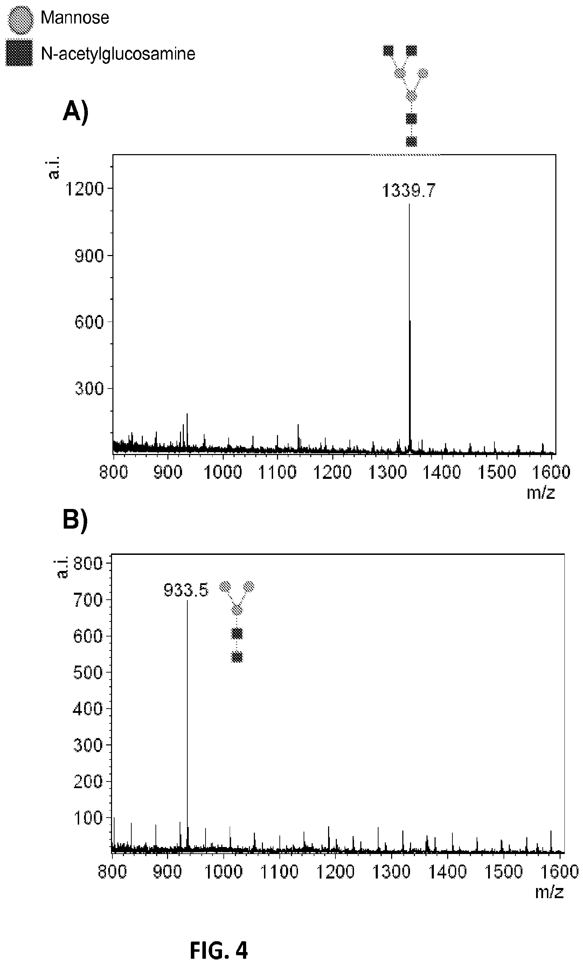

In further aspects of the disclosure, multiple-antennary glycans are produced. For instance, a prokaryotic host cell is transformed with a gene encoding bovine GnTIV fused to MBP in a host cell expressing Alg13, Alg14, Alg1, Alg2 and GnTI. **FIG. 4A** demonstrates GlcNAc₂Man₃GlcNAc₂ hybrid glycoform produced using the methods of the invention wherein two UDP-GlcNAc residues are transferred onto the Manα1,3 arm of the trimannosyl core. The expected structure of the glycoform shown is β1-2-GlcNAc(β1-2-GlcNAc) Manα1-3(Manα1-6)-Manβ1-4-GlcNAcβ1-4-GlcNAc.

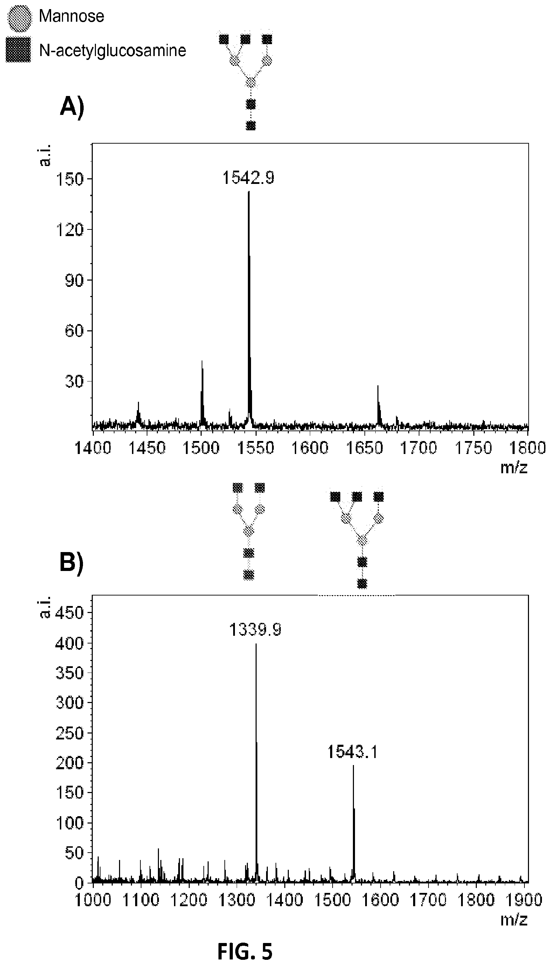

In alternative embodiments, glycans can also be formed *ex vivo,* e.g., through enzymatic synthesis of oligosacchardies as described in **Example 7.** For instance **FIG. 5** depicts a MS of complex, multiple-antennary glycans comprising GlcNAc₃Man₃GlcNAc₂ glycoform, which is produced by expressing GnTI, GnTII, GnTIV (*ex vivo*), Alg13, Alg14, Alg1 and Alg2 resulting in the transfer of two UDP-GlcNAc residues onto the Manα1,3 arm and one UDP-GlcNAc residue onto the Manα1,6 arm of the trimannosyl core oliogosaccharide structure. The expected structure of the glycoform shown is (β1-2-GlcNAcManα1-3)β1-2-GlcNAc(β1-2-GlcNAc Manα1-6)-Manβ1-4-GlcNAcβ1-4-GlcNAc.

Additional GnT activites such as GnTV (EC 2.4.1.155) and GnTVI (2.4.1.201) can be expressed in the prokaroytoic system. As a result, multiple antennary glycans of up to 5 branches on the trimannose core are possible using the methods of the invention. Mulitple branched glycans enable, for example, enhanced sialylation on erythropoietin, increasing serum half-life and potentcy (Elliot, Nature Biotech 2003; Misaizu, Blood 1995).

### Glycosyltransferase Solubility Enhancers

While various GnTs can be expressed in a host cell, in preferred embodiments, GnTs are fused to, for example, MBP and expressed as a fusion protein to transfer a terminal UDP-GlcNAc residue onto the trimannosyl core, in effect, enhancing solubility of the glycosyltransferase. **Table 1** provides a list provides a class of membrane targeting domains and solubility enhancers.

**Table 1. Solubility Enhancers**

| | **Glycan Synthesis** | |
|---|---|---|
| **FUSION PARTNER** | **Alg11** | **GnTI** |
| None | - | - |
| DsbA | - | + |
| GlpF | +/- | + |
| GST | + | + |
| MBP (EC# P0AEX9) | +/- | + |
| MstX | + | + |
| NusA | - | N/A |
| TrxA | - | N/A |

Using a library of fusions, glycans such as GlcNAc₍₁₋₅₎Man₃GlcNAc₂ are produced in the prokaryotic system of the present invention. In certain aspects of the invention, MBP-fused glycosyltransferases are expressed in a prokaryotic host. Other membrane targeting domains and solubility enhancers, such as MstX can also be expressed. Such *N*-acetylglucosaminyl transferase-MBP or *N*-acetylglucosaminyl transferase-MstX fusions are screened for the addition of UDP-GlcNAc residue onto the acceptor oligosaccharide substrate. In preferred embodiments, the following fusions: *N. tabacum* GnTI-MBP, *H. Sapiens* GnTII-MBP, *B. taurus* GnT IV-MBP confer UDP-GlcNAc transfer onto the trimannosyl core. Accordingly, a library of GnT fusions can be made to produce hybrid, complex and multi-antennary glycans in prokaryotic host cells. Various GnT fusion constructs can be made using the methods of the present invention. Such fusion constructs are within the scope of disclosure and can be screened for better activity or enhanced solubility.

### Galactosyltransferase Expression in Prokaryotes

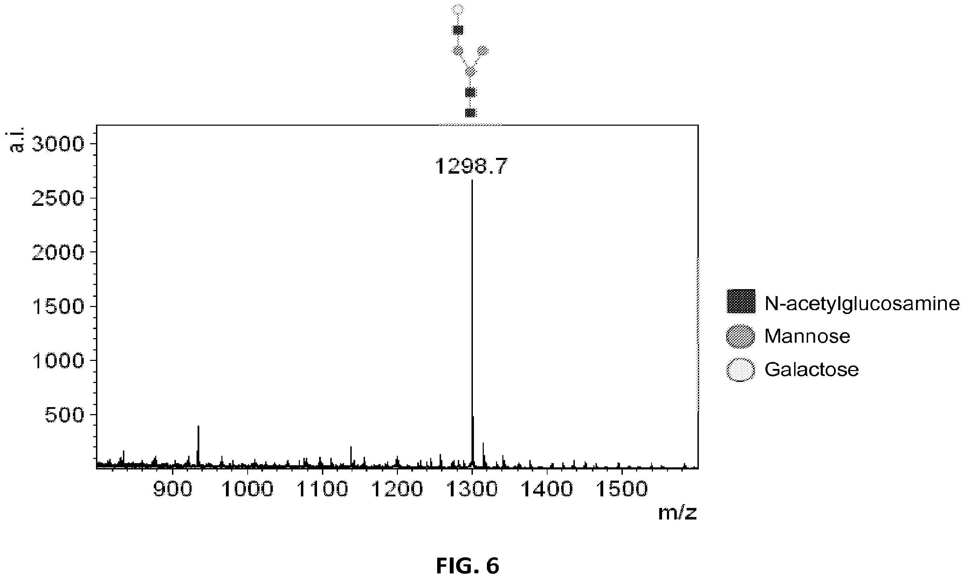

In further aspects of the disclosure, a method is provided for producing an oligosaccharide composition, said method comprising: culturing the host cell to express one or more galactosyltransferase enzyme activity (EC 2.4.1.38, EC 2.7.8.18) that catalyzes the transfer of a UDP-Galactose residue onto said terminal GlcNAc residue, said culturing step carried out under conditions effective to produce an oligosaccharide composition having a terminal galactose residue. **FIG. 6** depicts a MS of the hybrid glycoform GalGlcNAcMan₃GlcNAc₂ produced in *E. coli.* **Example 5** describes expression of *Helicobacter pylori* β-1,4GalT in *E. coli,* which transfers a UDP-galactose residue onto the GlcNAcMan₃GlcNAc₂ acceptor oligosaccharide.

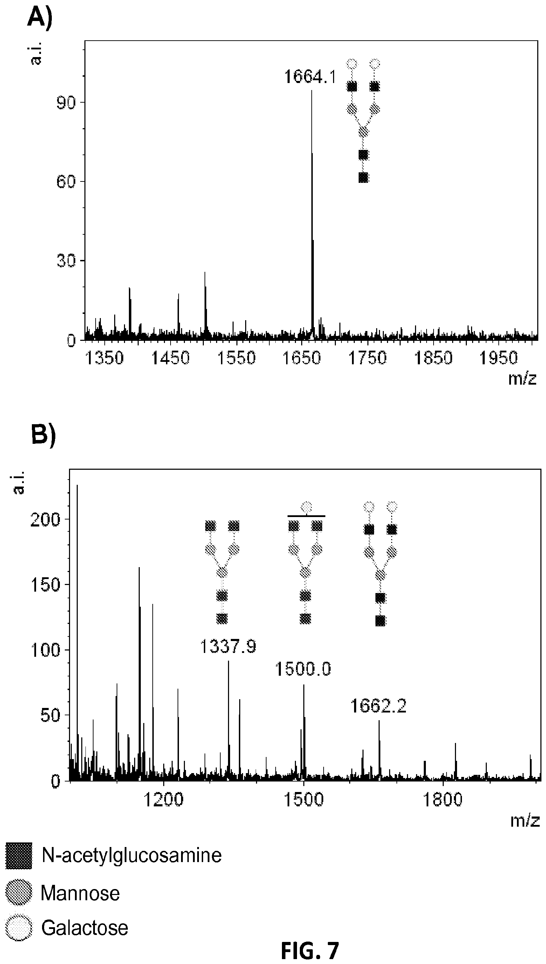

To produce a hybrid galactosylated glycoform in a prokaryote, UDP-galactose residue is transferred onto the β-1,2GlcNAcManα1,3 of the trimannosyl core and both β-1,2GlcNAcManα1,3 and β-1,2GlcNAcManα1,6 arms of the trimannosyl core for the complex glycoform. In such embodiments, a prokaryotic host cell is transformed with a gene encoding *H. pylori* GalT in a host cell expressing the Alg13, Alg14, Alg1, Alg2, GnTI and GnTII. **Example 8** provides methods for producing a complex Gal₂GlcNAc₂Man₃GlcNAc₂ glycoform. **FIG. 7** shows a peak at m/z 1662.2, which correlates with the mass of the complex galactosylated glycan Gal₂GlcNAc₂Man₃GlcNAc₂. Additional galactosylated glycoforms can be produced including: Gal₍₁₋₄₎GlcNAc₂Man₃GlcNAc₂. The expected structure of the hybrid terminal galactose glycan is β1-4Galβ1-2-GlcNAcManα1-3(Manα1-6)-Manβ1-4-GlcNAcβ1-4-GlcNAc and the complex terminal galactose glycan is β1-4Galβ1-2-GlcNAcManα1-3(β1-4Galβ1-2-GlcNAc Manα1-6)-Manβ1-4-GlcNAc(β1-4-GlcNAc.

Galactosyltransferases from various other organisms can be expressed, which include but are not limited to *Helicobacter pylori, Neisseria meningitides, Neisseria gonorrhoeae, Leishmania donovani, Homo sapiens* (GALT), *Bos Taurus, Drosophia, melanogaster, Rattus norvegicus* (GalT I), *Mus musculus, Cricetulus griseus, Equus caballus, Macropus eugenii* (4β-GalT), *Danio rerio* (GalT I) and *Sus scrofa, Ovis aries.*

In some embodiments, various galactosyltransferase enzyme activities are fused to solubility enhancers such as MBP or mstX and screened for addition of UDP-Galactose onto the acceptor oligosaccharide substrate. Unlike the GnTs, the human and bovine GaIT-mstX fusions did not appear to transfer UDP-Galactose onto the terminal GlcNAc oligosaccharide substrate.

In more preferred embodiments, oxidative bacterial strains are used for the expression of *H. pylori* β-1,4-GalT.

In an exemplary embodiment, the following enzymes are expressed in a prokaryotic host: Alg13, Alg14, Alg1, Alg2, *Nicotiana tabaccum* GnTI, human GnTII, bovine GnTIV, *Helicobacter pylori* β-1,4GalT. The GnTs and the GalT are expressed in an oxidative bacterial host.

### Sialyltransferase Expression in Prokaryotes

Full complex oligosaccharide structures end in a terminal sialic acid, e.g., NANA residues. Expression of sialyltransferases in prokaryotes has been a considerable interest. While several groups have undertaken the task of sialic acid transfer for glycoprotein production for many years, to date, no reports exist for production of sialic acid transfer to produce a human-like glycan in prokaryotes.

Accordingly, the present disclosure provides methods to produce oligosaccharide compositions by culturing a recombinant prokaryotic host to express one or more sialyltransferase enzyme activity (EC 2.4.99.4 and EC 2.4.99.1) that catalyzes the transfer of a CMP-NANA residue onto said terminal galactose residue, said culturing step carried out under conditions effective to produce an oligosaccharide composition having a terminal sialic acid residue. Various sialyltransferases are expressed using the methods of the disclosure, either *in vivo* or *ex vivo.* In one embodiment, an α-2,3 sialyltransferase (EC 2.4.99.4) is expressed in a host cell or in the culture medium. In futher embodiments, an α-2,6 sialyltransferase (EC 2.4.99.1) is expressed in a host cell or in the culture medium.

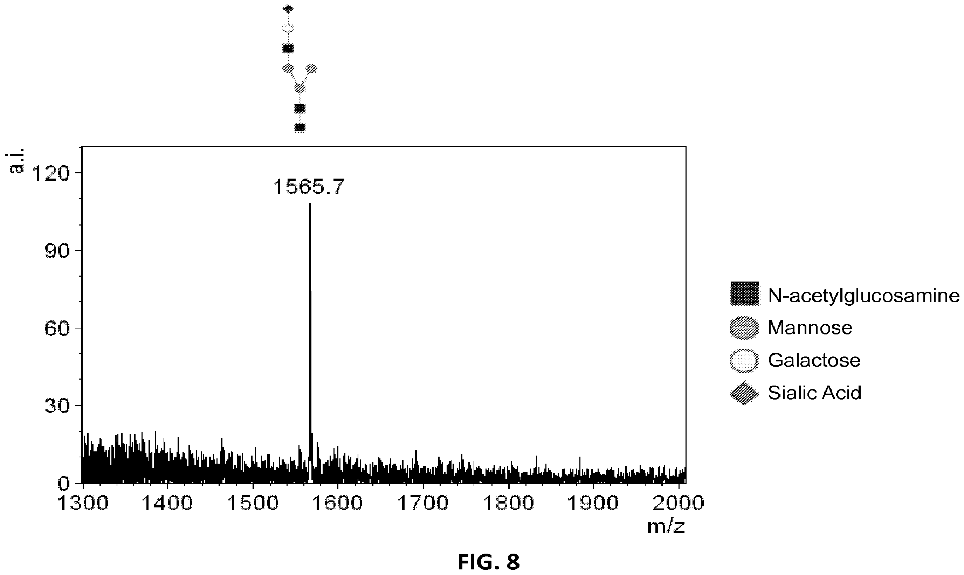

In preferred embodiments, the following enzymes are expressed in a prokaryotic host: Alg13, Alg14, Alg1, Alg2, *Nicotiana tabaccum* GnTI, bovine GnTIV, *Helicobacter pylori* β-1,4-GalT and *P*. *damselae* ST6. The method allows for a combination of *in vivo* and *ex vivo* reactions that demonstrate the proper transfer of CMP-NANA onto the correct oligosaccharide substrates. As shown in **Fig. 8****,** the hybrid sialylated glycoform is produced where the expected structure of the glycoform shown is 2,6NANAβ1-4Gal β1-2-GlcNAcManα1-3(Manα1-6)-Manβ1-4-GlcNAcβ1-4-GlcNAc.

### Sugar Nucleotide Precursors

In yet other embodiments, the method provides for culturing the host cell to increase sugar nucleotide precursors. For instance, enzymes that catalyze GDP-Mannose synthesis are expressed in the system. Phosphomannomutase enzyme activity (ManB) (EC 5.4.2.8) and mannose-1-phosphate guanylyltransferase enzyme activity (ManC) (EC 2.7.7.13) are introduced in the host cell of the invention. **FIG. 9A** (left) shows increased production of the trimannosyl core when ManC/B is overexpressed.

In additional embodiments, a sufficient pool of glycosyl donors in the cytoplasm is generated. UDP-GlcNAc, the substrate for GnTI and GnTII, is naturally present in the *E. coli* cytoplasm but the host cell can be engineered for increased UDP-GlcNAc synthesis. In such embodiments, the method provides for culturing the host cell to increase UDP-GlcNAc by expressing glutamine-fructose-6-phosphate transaminase enzyme activity GlmS (EC 2.6.1.16), GlmU (EC 2.7.7.23 & EC 2.3.1.157), GlmM (EC 5.4.2.10), which catalyze UDP-GlcNAc synthesis. **FIG. 9A** (right) shows an increase in GlcNAcMan₃GlcNAc₂ when GlmS was overexpressed. Addition of glycerol with ManC/B results in increased glycan yield as shown in **FIG. 9B****.** Pyruvate also appears to increase glycan yield as shown in **FIG. 9C****.**

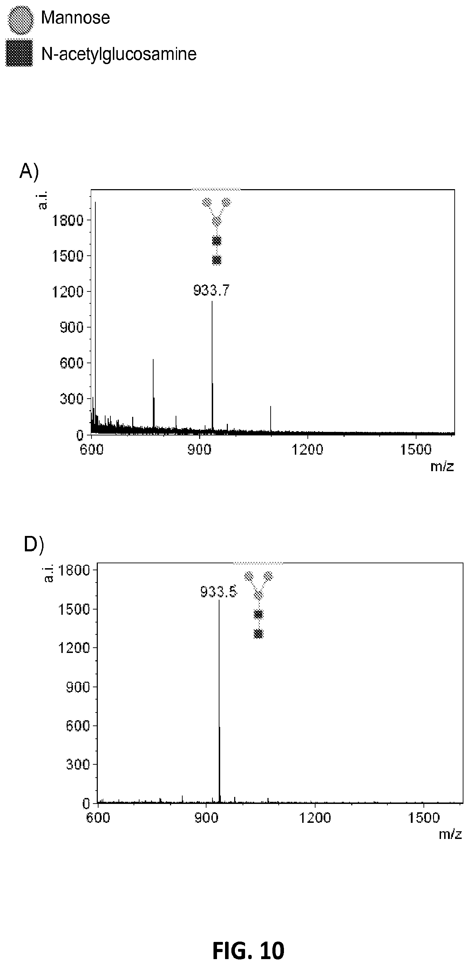

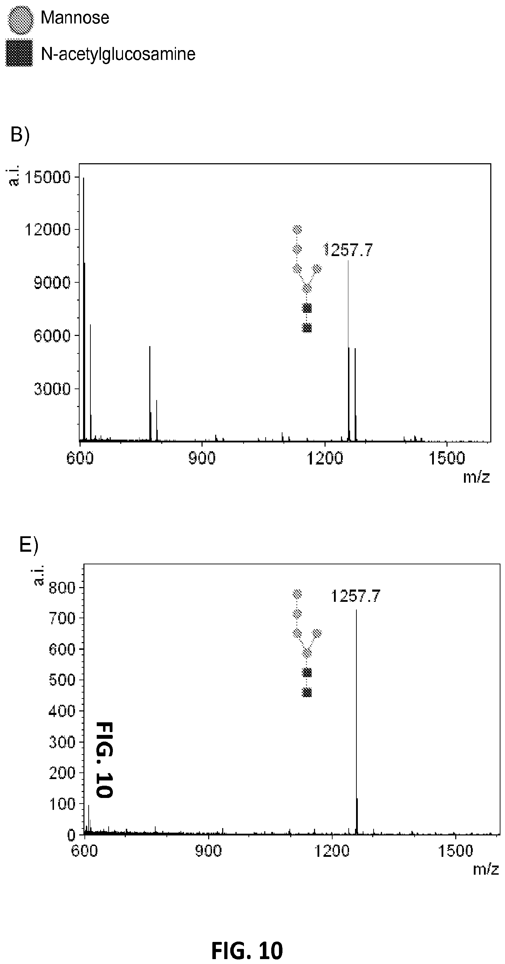

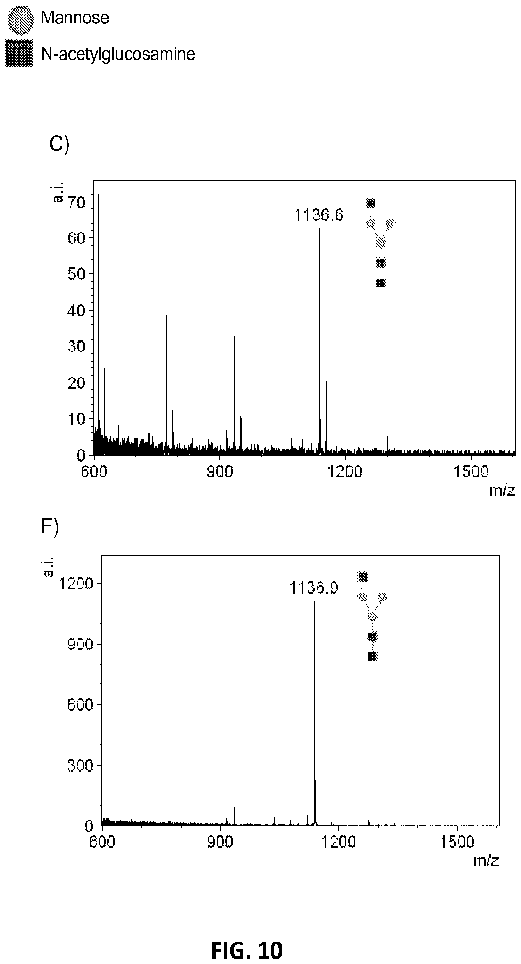

Overexpression of ManC/B had a dramatic effect on the homogeneity of the glycans produced as evidenced in **FIG. 10****.** The M3 glycoform (**D**), the M5 glycoform (**E**) and the GNM3GN2 (**F**) resulted in glycans that are predominant and appears to have removed the peaks that may be due to the incomplete nucleotide sugar transfer of the reaction. Accordingly, the host cell is capable of producing and controlling the precise glycoform produced.

In yeast, Bobrowicz et al., showed increased production of terminally galactosylated glycans *Pichia* thorugh expression of UDP-galactose transporter, UDP-galactose 4-epimerase and β1,4GalT in *P. pastoris.* (Bobrowicz et al., Engineering of an artificial glycosylation pathway blocked in core oligosaccharide assembly in the yeast Pichia pastoris: production of complex humanized glycoproteins with terminal galactose. Glycobiology 2004 Sep;14(9):757-66.). UDP-Galactose is also naturally present in the cytoplasm of *E. coli,* however studies have shown that the availability of UDP-Galactose can be increased by overexpression of UDP-Gal synthesis genes including uridylate kinase (*pyrH*), Glc-1-P uridyltransferase (*galU*), Gal-1-P uridyltransferase (*galT*), galactokinase (*galK*), and UDP-galactose epimerase (*galE*) (Chung, S., et al., Galactosylation and sialylation of terminal glycan residues of human immunoglobulin G using bacterial glycosyltransferases with in situ regeneration of sugar-nucleotides. Enzyme and Microbial Technology, 2005. 39(1): p. 60-66.). Thus, in preferred embodiments one or more genes selected from *galETK, galU,* and *pyrH* from *E. coli* K12 is cloned using yeast-based recombination and subsequently expressed in the host strain to ensure a sufficient UDP-Gal pool of glycosyl donor substrates for transfer of galactose onto the acceptor oligosaccharide composition.

The modulation of CMP-NANA levels has been shown in both yeast and insect cells. Hamilton et al. showed increased cellular CMP-NANA pool for successful sialic acid transfer in *P. pastoris* using CMP-sialic acid transporter, UDP-GlcNAc 2-epimerase/N-acetylmannosamine kinase, CMP-sialic acid synthase, N-acetylneuraminate-9-phosphate synthase, and sialyltransferase (Hamilton, S.R., et al.,. Production of complex human glycoproteins in yeast.Science, 301, 1244 (2003)). Lawrence et al., showed coexpression of cytidine monophosphate sialic acid synthase (CMP-SA) and sialic acid phosphate synthase (SAS) gene with *N*-acetylmannosamine feeding for increased CMP-SA substrate production insect cells (Lawrence et al., Cloning and expression of human sialic acid pathway genes to generate CMP-sialic acids in insect cells. Glycoconj J. 2001 Mar;18(3):205-13). Only a select few host cells such as *E. coli* K1 has endogenous CMP-NANA mechanism, however, many prokaryotes lack the machinery to produce CMP-NANA and it is at least expected that increased CMP-NANA levels is required for proper sialylation in prokaryotes.

The successful expression of eukaryotic proteins, especially membrane proteins, in *E. coli* and other bacteria is a nontrivial task (Baneyx et al., "Recombinant Protein Folding and Misfolding in Escherichia coli," Nat Biotechnol 22:1399-1408 ((2004)). Thus, consideration has to be given to numerous issues in order to achieve high expression yields of correctly folded and correctly localized proteins (e.g., insertion into the inner membrane). All of these factors collectively dictate whether the eukaryotic proteins will be functional when expressed inside *E. coli* cells.

### Additional Glycoengineering

Host cells that lack certain enzyme activities are preferred, such host cells that do not express or are attenuated in certain enzymes that compete with sugar biosynthesis (e.g., mannosyltransferases). In a preferred embodiment, the method provides for culturing the host cell that is attenuated in GDP-D-mannose dehydratase enzyme activity (EC 4.2.1.47) as shown in Valderrama-Rincon et al. An *E*. *coli* strain that lack the *gmd* gene encoding GDP-mannose dehydratase (GMD) is constructed that would in effect increase the availability of the substrate for Alg1 and Alg2, GDP-mannose, which is converted to GDP-4-keto-6-deoxymannose by GMD as the first step in the synthesis of GDP-L-fucose (Ruffing, A. & Chen, R.R. Metabolic engineering of microbes for oligosaccharide and polysaccharide synthesis. Microb Cell Fact 5, 25 (2006). Additional engineering of the host cell may be required to knock-out certain competing pathways.

### Codon Optimization

In additional embodiments of the present disclosure, eukaryotic glycosyltransferases are codon optimized to overcome limitations associated with the codon usage bias between *E. coli* (and other bacteria) and higher organisms, such as yeast and mammalian cells. Codon usage bias refers to differences among organisms in the frequency of occurrence of codons in protein-coding DNA sequences (genes). A codon is a series of three nucleotides (triplets) that encodes a specific amino acid residue in a polypeptide chain. Codon optimization can be achieved by making specific transversion nucleotide changes, i.e. a purine to pyrimidine or pyrimidine to purine nucleotide change, or transition nucleotide change, i.e. a purine to purine or pyrimidine to pyrimidine nucleotide change. In some instances, the codon optimized polypeptide variants retain the same biological function as the uncodon optimized polypeptides. For expression in *E. coli,* one or more codons can be optimized as described in, e.g., Grosjean et al., Gene 18:199-209 (1982). As used herein, "*" indicate stop codons.

The nucleic acid molecules, polypeptide molecules and homologs, variants and derivatives of the *alg, N*-acetylglucosaminyl transferase, galactosytransferase, sialyltransferase, ManB/C, *glmS,* oligosaccharyl transferaes described herein also comprise polynucleotide and polypeptide variants, which can be naturally occurring or created *in vitro* including chemical synthesis using known genetic engineering techniques. In some embodiments, the polynucleotide sequences have at least 75%, 77%, 80%, 85%, 90%, or 95% identity to SEQ ID NO:1, 3, 5, 7, 9, 11, 13, 15, 17, 19, 21, 23, 25, 27, or 29. In other embodiments, polypeptide variants have at least about 50% ,55%, 60%, 65%, 70%, 75%, 77%, 80%, 85%, 90%, or 95% homology to SEQ ID NO:2, 4, 6, 8,10, 12, 14, 16, 18, 20, 22, 24, 26, 28, 30, 31, 32, or 33.

The present disclosure also encompasses nucleic acid molecules that hybridize under stringent conditions to the above-described nucleic acid molecules. As defined above, and as is well known in the art, stringent hybridizations are performed at about 25°C below the thermal melting point (Tₘ) for the specific DNA hybrid under a particular set of conditions, where the Tₘ is the temperature at which 50% of the target sequence hybridizes to a perfectly matched probe. Stringent washing can be performed at temperatures about 5°C lower than the Tₘ for the specific DNA hybrid under a particular set of conditions.

The polynucleotides or nucleic acid molecules of the present disclosure refer to the polymeric form of nucleotides of at least 10 bases in length. These include DNA molecules (e.g., linear, circular, cDNA, chromosomal, genomic, or synthetic, double stranded, single stranded, triple-stranded, quadruplexed, partially double-stranded, branched, hair-pinned, circular, or in a padlocked conformation) and RNA molecules (e.g., tRNA, rRNA, mRNA, genomic, or synthetic) and analogs of the DNA or RNA molecules of the described as well as analogs of DNA or RNA containing non-natural nucleotide analogs, non-native inter-nucleoside bonds, or both. The isolated nucleic acid molecule of the disclosure includes a nucleic acid molecule free of naturally flanking sequences (i.e., sequences located at the 5' and 3' ends of the nucleic acid molecule) in the chromosomal DNA of the organism from which the nucleic acid is derived. In various embodiments, an isolated nucleic acid molecule can contain less than about 10 kb, 5 kb, 4 kb, 3 kb, 2 kb, 1 kb, 0.5 kb, 0.1 kb, 50 bp, 25 bp or 10 bp of naturally flanking nucleotide chromosomal DNA sequences of the microorganism from which the nucleic acid molecule is derived.

The heterologous nucleic acid molecule is inserted into the expression system or vector in proper sense (5' → 3') orientation relative to the promoter and any other 5' regulatory molecules, and correct reading frame. The preparation of the nucleic acid constructs can be carried out using standard cloning methods well known in the art, as described by Sambrook et al., Molecular Cloning: A Laboratory Manual, Cold Springs Laboratory Press, Cold Springs Harbor, New York (1989). U.S. Patent No. 4,237,224 to Cohen and Boyer, also describes the production of expression systems in the form of recombinant plasmids using restriction enzyme cleavage and ligation with DNA ligase.

Suitable expression vectors include those which contain replicon and control sequences that are derived from species compatible with the host cell. For example, if *E. coli* is used as a host cell, plasmids such as pUC19, pUC18, or pBR322 may be used. Other suitable expression vectors are described in Molecular Cloning: a Laboratory Manual: 3rd edition, Sambrook and Russell, 2001, Cold Spring Harbor Laboratory Press. Many known techniques and protocols for manipulation of nucleic acids, for example in preparation of nucleic acid constructs, mutagenesis, sequencing, introduction of DNA into cells and gene expression, and analysis of proteins, are described in detail in Current Protocols in Molecular Biology, Ausubel et al. eds., (1992).

Different genetic signals and processing events control many levels of gene expression (e.g., DNA transcription and messenger RNA ("mRNA") translation) and subsequently the amount of fusion protein that is displayed on the ribosome surface. Transcription of DNA is dependent upon the presence of a promoter, which is a DNA sequence that directs the binding of RNA polymerase, and thereby promotes mRNA synthesis. Promoters vary in their "strength" (i.e., their ability to promote transcription). For the purposes of expressing a cloned gene, it is desirable to use strong promoters to obtain a high level of transcription and, hence, expression and surface display. Therefore, depending upon the host system utilized, any one of a number of suitable promoters may also be incorporated into the expression vector carrying the deoxyribonucleic acid molecule encoding the protein of interest coupled to a stall sequence. For instance, when using *E. coli,* its bacteriophages, or plasmids, promoters such as the T7 phage promoter, *lac* promoter, *trp* promoter, recA promoter, ribosomal RNA promoter, the P_{R} and P_{L} promoters of coliphage lambda and others, including but not limited, to *lac*UV5, *omp*F, *bla, lpp,* and the like, may be used to direct high levels of transcription of adjacent DNA segments. Additionally, a hybrid *trp-lac*UV5 (*tac*) promoter or other *E. coli* promoters produced by recombinant DNA or other synthetic DNA techniques may be used to provide for transcription of the inserted gene.

Translation of mRNA in prokaryotes depends upon the presence of the proper prokaryotic signals, which differ from those of eukaryotes. Efficient translation of mRNA in prokaryotes requires a ribosome binding site called the Shine-Dalgarno ("SD") sequence on the mRNA. This sequence is a short nucleotide sequence of mRNA that is located before the start codon, usually AUG, which encodes the amino-terminal methionine of the protein. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, Methods in Enzymology, 68:473 (1979).

### Host Cells

In accordance with the present invention, the host cell is a prokaryote. Such cells serve as a host for expression of recombinant proteins for production of recombinant therapeutic proteins of interest. Exemplary host cells include *E. coli* and other *Enterobacteriaceae, Escherichia sp., Campylobacter sp., Wolinella sp., Desulfovibrio sp. Vibrio sp., Pseudomonas sp. Bacillus sp., Listeria sp., Staphylococcus sp., Streptococcus sp., Peptostreptococcus sp., Megasphaera sp., Pectinatus sp., Selenomonas sp., Zymophilus sp., Actinomyces sp., Arthrobacter sp., Frankia sp., Micromonospora sp., Nocardia sp., Propionibacterium sp., Streptomyces sp., Lactobacillus sp., Lactococcus sp., Leuconostoc sp., Pediococcus sp., Acetobacterium sp., Eubacterium sp., Heliobacterium sp., Heliospirillum sp., Sporomusa sp., Spiroplasma sp., Ureaplasma sp., Erysipelothrix, sp., Corynebacterium sp. Enterococcus sp., Clostridium sp., Mycoplasma sp., Mycobacterium sp., Actinobacteria sp., Salmonella sp., Shigella sp., Moraxella sp., Helicobacter sp, Stenotrophomonas sp., Micrococcus sp., Neisseria sp., Bdellovibrio sp., Hemophilus sp., Klebsiella sp., Proteus mirabilis, Enterobacter cloacae, Serratia sp., Citrobacter sp., Proteus sp., Serratia sp., Yersinia sp., Acinetobacter sp., Actinobacillus sp. Bordetella sp., Brucella sp., Capnocytophaga sp., Cardiobacterium sp., Eikenella sp., Francisella sp., Haemophilus sp., Kingella sp., Pasteurella sp., Flavobacterium sp. Xanthomonas sp., Burkholderia sp., Aeromonas sp., Plesiomonas sp., Legionella sp.* and alpha-proteobacteria such as *Wolbachia sp.,* cyanobacteria, spirochaetes, green sulfur and green non-sulfur bacteria, Gram-negative cocci, Gram negative bacilli which are fastidious, Enterobacteriaceae -glucose-fermenting gram-negative bacilli, Gram negative bacilli - non-glucose fermenters, Gram negative bacilli - glucose fermenting, oxidase positive.

In one embodiment of the present disclosure, the *E. coli* host strain C41(DE3) is used, because this strain has been previously optimized for general membrane protein overexpression (Miroux et al., "Over-production of Proteins in Escherichia coli: Mutant Hosts That Allow Synthesis of Some Membrane Proteins and Globular Proteins at High Levels," J Mol Biol 260:289-298 (1996). Further optimization of the host strain includes deletion of the gene encoding the DnaJ protein (e.g., Δ*dnaJ* cells). The reason for this deletion is that inactivation of *dnaJ* is known to increase the accumulation of overexpressed membrane proteins and to suppress the severe cytotoxicity commonly associated with membrane protein overexpression (Skretas et al., "Genetic Analysis of G Protein-coupled Receptor Expression in Escherichia coli: Inhibitory Role of DnaJ on the Membrane Integration of the Human Central Cannabinoid Receptor," Biotechnol Bioeng (2008)). Applicants have observed this following expression of Alg1 and Alg2. Furthermore, deletion of competing sugar biosynthesis reactions is required to ensure optimal levels of *N-*glycan biosynthesis. For instance, the deletion of genes in the *E. coli* 016 antigen biosynthesis pathway (Feldman et al., "The Activity of a Putative Polyisoprenol-linked Sugar Translocase (Wzx) Involved in Escherichia coli O Antigen Assembly is Independent of the Chemical Structure of the O Repeat," J Biol Chem 274:35129-35138 (1999)) will ensure that the bactoprenol-GlcNAc-PP substrate is available for desired mammalian *N*-glycan reactions. To eliminate unwanted side reactions, the following are representative genes that are deleted from the *E. coli* host strain: *wbbL, glcT, glf, gafT, wzx, wzy, waaL.* Yet other strains include MC4100, BL21, ORIGAMI™, Shuffle®.

Methods for transforming/transfecting host cells with expression vectors are well-known in the art and depend on the host system selected, as described in Sambrook et al., Molecular Cloning: A Laboratory Manual, Cold Springs Laboratory Press, Cold Springs Harbor, New York (1989). For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation, and transfection using bacteriophage.

A key advantage of the prokaryotic host cell of disclosure includes: (i) the massive volume of data surrounding the genetic manipulation of bacteria; (ii) the established track record of using bacteria for protein production ∼30% of protein therapeutics approved by the FDA since 2003 are produced in *E. coli* bacteria; and (iii) the existing infrastructure within numerous companies for bacterial production of protein drugs.

In comparison to various eukaryotic protein expression systems, the process employed using the methods and composition of the invention provides a scalable, cost-effective, optimal recombinant glycoprotein expression, free of human pathogens, free of immunogenic N- and *O*-linked glycosylation reactions, capable of rapid cloning and fast growth rate, fast doubling time (∼20 minutes), high growth (high OD), high titer and protein yields (in the range of 50% of the total soluble protein (TSP)), ease of product purification from the periplasm or supernatant, genetically tractable, thoroughly studied, compatible with the extensive collection of expression optimization methods (e.g., promoter engineering, mRNA stabilization methods, chaperone co-expression, protease depletion, etc.).

Another major advantage of prokaryotes, e.g., *E. coli* as a host for glycoprotein expression is that, unlike yeast and all other eukaryotes, there are no native glycosylation systems. Thus, the addition (or subsequent removal) of glycosylation-related genes is expected to have little to no bearing on the viability of glycoengineered *E. coli* cells. Furthermore, the potential for non-human glycan attachment to target proteins by endogenous glycosylation reactions is essentially eliminated in these cells.

Accordingly, in various embodiments, an alternative for glycoprotein expression and production of various oligosaccharide compositions (e.g., high-mannose, hybrid, complex) is disclosed where a prokaryotic host cell is used to produce the same and produce *N*-linked glycoproteins, which provide an attractive solution for circumventing the significant hurdles associated with eukaryotic cell culture. The use of bacteria as a production vehicle that yields structurally homogeneous human-like *N*-glycans while at the same time dramatically lowering the cost and time associated with protein drug development and manufacturing is an object of the disclosure.

### Site-Specific Transfer of Oligosaccharide onto Target Proteins in Prokaryotes

As described in Valderrama-Rincon et al., to begin "humanizing" the bacterial glycosylation machinery, the Man₃GlcNAc₂ oligosaccharide structure is generated via a recombinant pathway comprising lipid-linked biosynthesis in *E. coli.* Specifically, one of several eukaryotic glycosyltransferases is functionally expressed in *E. coli* and the resulting lipid-linked oligosaccharides are transferred onto a protein via an oligosaccharyl transferase.

Glycan assembly in the prokaryotic host cells is lipid-linked on undecaprenyl phosphate (Und-P) unlike eukaryotes where they are assembled on dolichol phosphate (Dol-P). In *C*. *jejuni, N*-linked glycosylation proceeds through the sequential addition of nucleotide-activated sugars onto a lipid carrier, resulting in the formation of a branched heptasaccharide. This glycan is then flipped across the inner membrane by PglK (formerly WlaB) and the OTase PglB then catalyzes the transfer of the glycan to an asparagine side chain. Bac is 2,4-diacetamido-2,4,6-trideoxyglucose; GalNAc is *N*-acetylgalactosamine; HexNAc is *N*-acetylhexosamine; Glc is glucose. See Szymanski et al., "Protein Glycosylation in Bacterial Mucosal Pathogens," Nat Rev Microbiol 3:225-37 (2005). The PglK flippase is responsible for translocating the lipid-linked *C. jejuni* heptasaccharide across the inner membrane. Fortuitously, PglK exhibits relaxed specificity towards the glycan structure of the lipid-linked oligosaccharide intermediate (Alaimo et al., "Two Distinct But Interchangeable Mechanisms for Flipping of Lipid-linked Oligosaccharides," Embo J 25:967-76 (2006) and Wacker et al., "Substrate Specificity of Bacterial Oligosaccharyltransferase Suggests a Common Transfer Mechanism for the Bacterial and Eukaryotic Systems," Proc Natl Acad Sci USA 103:7088-93 (2006).

In preferred embodiments, the host cell of the disclosure expresses a flippase enzyme activity (Genbank AN AP009048.1), which translocates the undecaprenol-linked oligosaccharide across the inner membrane. Such enzyme activity may be endogenous or heterologous or engineered to be modified in expression. In additional embodiments, the prokaryotic host cell comprises a flippase activity including *pglK* and *rft1.*

Production of a human-like oligosaccharide structure in prokaryotes entails the transfer of various oligosaccharides to N-X-S/T sites on polypeptide chains. This requires functional expression of an integral membrane protein or protein complex known as an oligosaccharyltransferase (OST) that is responsible for the transfer of oligosaccharides to the target protein. Various prokaryotic and eukaryotic OSTs have the ability to transfer the lipid-linked oligosaccharide onto the target protein. The present invention discloses a prokaryotic system that demonstrates the transfer of high-mannose, hybrid and complex glycans onto a protein. Accordingly, the prokaryotic protein expression system comprises at least one OST activity to produce a glycosylated target protein. In such embodiments, the host cell expresses an oligosaccharyl transferase enzyme activity (EC 2.4.1.119) in addition to the glycosyltransferase enzymes. Various OSTs (**Table 2**) can be expressed and may be endogenous or heterologous or engineered to be modified in expression. In further embodiments, the prokaryotic host cell comprises at least one oligosaccharyl transferase activity, such as PglB from *C. jejuni* (Aebi et al.) or *C*. *lari* (Valderrama-Rincon et al.). The oligosaccharide transferred onto the protein is *N*-linked to the protein.

**Table 2. List of Oligosaccharyltransferases.**

| **Protein EC #** | ***Organism*** | **Gen Bank** |
|---|---|---|
| CCC13826_0460 | *Campylobacter concisus 13826* | EAT99324.2 |
| CFF8240_1383 | *Campylobacter fetus subsp. fetus 82-40* | ABK82109.1 |
| CHAB381_0954 | *Campylobacter hominis ATCC BAA-381* | ABS52339.1 |
| OrfA (fragment) | *Campylobacter jejuni NCTC 11351* | AAD09300.1 |
| WlaF | *Campylobacter jejuni 81116* | CAA72355.1 ABV52665.1 |
| WlaF | *Campylobacter jejuni D450* | AAK97437.1 |
| CJE1268 | *Campylobacter jejuni RM1221* | AAW35590.1 |
| JJD26997_0595 | *Campylobacter jejuni subsp. doylei 269.97* | ABS43894.1 |
| OST (PglB;WlaF) EC 2.4.1.119 | *Campylobacter jejuni subsp. jejuni 81-176* | AAK97438.1 AAD51383.1 |
| OST (PglB;WlaF;Cj1126c) EC 2.4.1.119 | *Campylobacter jejuni subsp. jejuni NCTC 11168* | CAB73381.1 NP_282274.1 CAL35243.1 AAD09293.1 |
| Cla_1253 (PglB) | *Campylobacter lari RM2100 RM2100; ATCCBAA-1060D* | ACM64573.1 |
| Ddes_0746 | *Desulfovibrio desulfuricans subsp. desulfuricans str. ATCC 27774* | ACL48654.1 |
| Dde_3699 | *Desulfovibrio desulfuricans subsp. desulfuricans str. G20* | ABB40492.1 |
| DvMF_0846 | *Desulfovibrio vulgaris str. 'Miyazaki F'* | ACL07802.1 |
| Dvul_1810 | *Desulfovibrio vulgaris DP4* | ABM28827.1 |
| DVU1252 | *Desulfovibrio vulgaris str. Hildenborough* | AAS95730.1 |
| Geob_1424 Geob_2990 | *Geobacter sp. FRC-32* | ACM19784.1 |
| NAMH_1652 | *Nautilia profundicola AmH* | ACM92784.1 |
| NIS_1250 | *Nitratiruptor sp. SB155-2* | BAF70358.1 |
| Tmden_1474 | *Sulfurimonas denitrificans DSM 1251* | ABB44751.1 |
| SUN_0103 | *Sulfurovum sp. NBC37-1* | BAF71063.1 |
| WS0043 (WlaF) | *Wolinella succinogenes DSM 1740* | CAE09214.1 NP_906314.1 |
| OST, STT3 subunit | *Campylobacterales bacterium GD 1* | EDZ62411.1 |
| BACPLE_02950 | *Bacteroides plebeius DSM 17135* | EDY94544.1 |
| BACPLE_02943 | *Bacteroides plebeius DSM 17135* | EDY94539.1 |
| RHECIAT_CH0002772 | *Rhizobium etli CIAT 652* | ACE91723.1 |
| BACINT_01142 | *Bacteroides intestinalis DSM 17393* | EDV06057.1 |
| IMP (possible OST) | *Hydrogenivirga sp. 128-5-R1-1* | EDP74595.1 |
| OST (PglB) | *Campylobacter coli RM2228* | EAL57053.1 |
| OST (PglB) | *Campylobacter upsaliensis RM3195* | EAL53100.1 |

### Oligosaccharide Compositions

Recently, several eukaryotic expression hosts have been introduced as alternatives to mammalian cell culture for making *N*-glycoproteins. These include the genetically engineered yeast *Pichia pastoris* (Hamilton, S.R., et al., Humanization of yeast to produce complex terminally sialylated glycoproteins. Science, 2006. 313(5792): p. 1441-3), cultured insect cells as hosts for recombinant baculovirus (Aumiller, J.J., J.R. Hollister, and D.L. Jarvis, A transgenic insect cell line engineered to produce CMP-sialic acid and sialylated glycoproteins. Glycobiology, 2003. 13(6): p. 497-507), and plant cells (Aviezer, D., et al., A plant-derived recombinant human glucocerebrosidase enzyme--a preclinical and phase I investigation. PLoS One, 2009. 4(3): p. e4792). Unfortunately, nonhuman glycoforms arise from native glycosylation pathways when using *any* eukaryotic host cell including mammalian, plant, insect, and yeast cells. Mammalian host cells have been shown to add uncontrollable levels of mannose-6-phosphate and fucose to glycans and often lack terminal sialic acid (Van Patten, S.M., et al., Effect of mannose chain length on targeting of glucocerebrosidase for enzyme replacement therapy of Gaucher disease. Glycobiology, 2007. 17(5): p. 467-78.). Plant cells add immunogenic beta-1,2 xylose and core alpha-1,3 fucose (Bardor, M., et al., Immunoreactivity in mammals of two typical plant glyco-epitopes, core alpha(1,3)-fucose and core xylose. Glycobiology, 2003. 13(6): p. 427-34), the latter is also found in insect cells (Bencurova, M., et al., Specificity of IgG and IgE antibodies against plant and insect glycoprotein glycans determined with artificial glycoforms of human transferrin. Glycobiology, 2004. 14(5): p. 457-66). *O*-linked glycosylation is also an essential process in yeast (Gentzsch, M. and W. Tanner, The PMT gene family: protein O-glycosylation in Saccharomyces cerevisiae is vital. Embo J, 1996. 15(21): p. 5752-9) and undesired *O-*glycans can be covalently attached to target glycoproteins.

The oligosaccharide chain attached by the prokaryotic glycosylation machinery is structurally distinct from that attached by higher eukaryotic and human glycosylation pathways (Weerapana et al., "Asparagine-linked Protein Glycosylation: From Eukaryotic to Prokaryotic Systems," Glycobiology 16:91R-101R (2006). The oligosaccharide compositions produced in the prokaryotes and from the methods of the present invention are also distinguishable from eukaryotic systems such as yeast, insect, mammalian and even human cells.

Several features distinguish oligosaccharide compositions produced by the methods of the invention in comparison to eukaryotic host cell expression systems, e.g., CHO, NS0, lemna, Sf9. For instance, the oligosaccharide compositions of the present invention lack fucose. The absence of fucose in antibodies has been associated w increased ADCC and CDC activities (Shinkawa T et al., The absence of fucose but not the presence of galactose or bisecting N-acetylglucosamine of human IgG1 complex-type oligosaccharides shows the critical role of enhancing antibody-dependent cellular cytotoxicity. J Bio Chem, 278, 3466-73, 2003). Furthermore, prokaryotes inherently lack *O*-linked glycans, which is associated with immunogenicity. The oligosaccharide compositions of the present invention do not express abhorrent glycans that are present in many eukaryotic expression systems such as high-mannose or mannose phosphates. In addition, glycoengineered *E. coli* provides (i) control of the specific site and stoichiometry of glycosylation including at the N- or C-terminus, (ii) selection of the glycoform (iii) ability to engineer novel glycoforms because glycosylation is not an essential process in *E. coli,* and (iv) lack of competing glycosylation pathways including O-glycosylation and mannose 6-phosphate which improves product uniformity and may help avoid mislocalization to other receptors within the human host such as the mannose 6-phosphate receptor (Hayette, M.P. et al. Presence of human antibodies reacting with Candida albicans O-linked oligomannosides revealed by using an enzyme-linked immunosorbent assay and neoglycolipids. J Clin Microbiol 30, 411-417 (1992). Podzorski, R.P., Gray, G.R. & Nelson, R.D. Different effects of native Candida albicans mannan and mannan-derived oligosaccharides on antigen-stimulated lymphoproliferation in vitro. J Immunol 144, 707-716 (1990).).

The oligosaccharide compositions of the present invention can be uniform and also be enriched so as to boost anti-inflammatory properties, e.g., enriching for α2,6 sialic acid on Fc of intravenous Ig (IVIG) (Anthony et al., Identification of a receptor required for the anti-inflammatory activity of IVIG. Natl Acad Sci USA 2008 Dec 16;105(50):19571-8). Additional studies have indicated the presence of Neu5Gc-specific antibodies in all humans, sometimes at high levels (Ghaderi et al., Implications of the presence of N-glycolylneuraminic acid in recombinant therapeutic glycoproteins. Nat Biotechnol, 2010 Aug;28(8): 863-7). Thus, enriching for therapeutic proteins, e.g., antibodies with specific sialic acid residues (e.g., NeuNAc as opposed to Neu5Ac, Neu5Gc) may reduce adverse reaction such as immunogenicity or inefficacy of protein therapeutics.

As reflected herein, the prokaryotic system can yield homogenous glycans at a relatively high yield. In preferred embodiments, the oligosaccharide composition consists essentially of a single glycoform in at least 50, 60, 70, 80, 90, 95, 99 mole %. In further embodiments, the oligosaccharide composition consists essentially of two desired glycoforms of at least 50, 60, 70, 80, 90, 95, 99 mole %. In yet further embodiments, the oligosaccharide composition consists essentially of three desired glycoforms of at least 50, 60, 70, 80, 90, 95, 99 mole %.

In certain embodiments, the oligosaccharide compositions produced are GlcNAc₁₋₅Man₃GlcNAc₂ and Man₃GlcNAc₂. Certain glycol-engineered host cells produce oligosaccharide composition that is predominantly GlcNAcMan₃GlcNAc₂ or GlcNAc₂Man₃GlcNAc₂.

In other embodiments, the oligosaccharide compositions produced are Gal₁₋₅GlcNAc₁₋₅Man₃GlcNAc₂ and Man₃GlcNAc₂. Certain glycol-engineered host cells produce oligosaccharide composition that is predominantly GalGlcNAcMan₃GlcNAc₂, GalGlcNAc₂Man₃GlcNAc₂ or Gal₂GlcNAc₂Man₃GlcNAc₂.

In yet other embodiments, the oligosaccharide compositions produced are NANA₁₋₅Gal₁₋₅GlcNAc₁₋₅Man₃GlcNAc₂. Certain glycol-engineered host cells produce oligosaccharide composition that is predominantly NANAGalGlcNAcMan₃GlcNAc₂ or NANA₂Gal₂GlcNAc₂Man₃GlcNAc₂.

In still other embodiments, the oligosaccharide compositions produced are Man₂GlcNAc₂, Man₄GlcNAc, Man₃GlcNAc₂, HexMan₃GlcNAc₂, HexMan₅GlcNAc Man₆GlcNAc and Man₅GlcNAc₂. Certain glycol-engineered host cells produce oligosaccharide composition that is predominantly Man₅GlcNAc₂.

The present invention, therefore, provides stereospecific biosynthesis of a vast array of novel oligosaccharide compositions and *N*-linked glycoproteins. In certain embodiments, reconstitution of a eukaryotic *N*-glycosylation pathway in *E. coli* using metabolic pathway and protein engineering techniques results in *N-*glycoproteins with structurally homogeneous human-like glycans. This ensures that each glycoengineered cell line corresponds to a unique carbohydrate signature.

The glycans can be analyzed by metabolic labeling of cells with ³H-GlcNAc and ³H-mannose or with fluorescent lectins (e.g., AlexaFluor-ConA). Glycans can also be released with PNGase and detected under MALDI/TOF-MS.

Quantification of the glycans can be estimated with the MS or more exactly done through HPLC. NMR can determine the glycosidic linkages of the glycan structures.

### Target Glycoproteins

To produce various glycoproteins of interest, a gene encoding a target protein is introduced into the host cell.

"Target proteins", "proteins of interest", or "therapeutic proteins" include without limitation cytokines such as interferons, G-CSF, coagulation factors such as factor VIII, factor IX, and human protein C, soluble IgE receptor α-chain, IgG, IgG fragments, IgM, interleukins, urokinase, chymase, and urea trypsin inhibitor, IGF-binding protein, epidermal growth factor, growth hormone-releasing factor, annexin V fusion protein, angiostatin, vascular endothelial growth factor-2, myeloid progenitor inhibitory factor-1, osteoprotegerin, α-1 antitrypsin, DNase II, α-feto proteins, AAT, rhTBP-1 (aka TNF binding protein 1), TACI-Ig (transmembrane activator and calcium modulator and cyclophilin ligand interactor), FSH (follicle stimulating hormone), GM-CSF, glucagon, glucagon peptides, GLP-1 w/ and w/o FC (glucagon like protein 1) IL-I receptor agonist, sTNFr (aka soluble TNF receptor Fc fusion), CTLA4-Ig (Cytotoxic T Lymphocyte associated Antigen 4-Ig), receptors, hormones such as human growth hormone, erythropoietin, peptides, stapled peptides, human vaccines, animal vaccines, serum albumin and enzymes such as ATIII, rhThrombin, glucocerebrosidase and asparaginase.

Already approved therapeutics from *E. coli* are also target proteins. They include hormones (human insulin and insulin analogues, calcitonin, parathyroid hormone, human growth hormone, glucagons, somatropin and insulin growth factor 1), interferons (α1, α2a, α2b and γ1b), interleukins 2 and 11, light and heavy chains raised against vascular endothelial growth factor-α, tumor necrosis factor α, cholera B subunit protein, B-type natriuretic peptide, granulocyte colony stimulating factor and tissue plasminogen activator.

Target proteins also include a glycoprotein conjugate comprising a protein and at least one peptide comprising a D-X₁-N-X₂-T motif fused to the protein, wherein D is aspartic acid, X₁ and X₂ are any amino acid other than proline, N is asparagine, and T is threonine.

In preferred embodiments, at least 30, 50, 70, 90, 95 and preferably 100 mol% of glycans are transferred onto a target protein by an OST.

### Culture Conditions

In other embodiments, the methods provide culturing the host cells under oxidative conditions. Preferably, an oxidative bacterial strain is used. Culture conditions may result in increased yield and titre of glycoproteins and glycans. Such process conditions and parameters include regulating pH, temperature, osmolality, culture duration, media, nutrients, concentration of dissolved oxygen, nitrogen, level or availability of nucleotide sugars and even carbon source, e.g., glycerol (**FIG. 9B**) can influence the production system. Culture conditions may vary depending on the product and the specific host cell utilized. Productivity of the system is also likely to be affected by the culture conditions. Additional metabolic engineering may be required for maximum productivity and to limit growth-inhibiting metabolites.

### Enzymatic Synthesis of Oligosaccharides

In alternative aspects of the disclosure, glycans are synthesized in a cell-free extract using an acceptor glycan, purified enzyme/lysate and adding nucleotide sugars as described in **Example 7.**

In certain embodiments, the present disclosure provides a cell culture comprising a recombinant prokaryote, UDP-GlcNAc and a GnT (EC 2.4.1.101; EC 2.4.1.143; EC 2.4.1.145) wherein said GnT catalyzes the transfer of a UDP-GlcNAc residue onto said terminal mannose residue, cultured under conditions effective to produce an oligosaccharide composition having a terminal GlcNAc residue.

In further embodiments, the present disclosure provides a cell culture comprising a recombinant prokaryote, UDP-Galactose and a GalT (EC 2.4.1.38) wherein said GalT catalyzes the transfer of a UDP-Galactose residue onto said terminal GlcNAc residue, cultured under conditions effective to produce an oligosaccharide composition having a terminal galactose residue.

In preferred embodiments, the present disclosure provides a cell culture comprising a recombinant prokaryote, CMP-NANA and a sialyltransferase (EC 2.4.99.4 and EC 2.4.99.1) wherein said sialyltransferase catalyzes the transfer of a CMP-NANA residue onto said terminal galactose residue, cultured under conditions effective to produce an oligosaccharide composition having a terminal sialic acid residue.

### Aglycosylated vs. Glycosylated IgGs

Another aspect of the present disclosure relates to a glycosylated antibody comprising an Fv portion which recognizes and binds to a native antigen and an Fc portion which is glycosylated at a conserved asparagine residue. Alternative embodiments include diabody, scFv, scFv-Fc, scFv-CH, Fab and scFab.

The glycosylated antibody of the present disclosure can be in the form of a monoclonal or polyclonal antibody.

A single immunoglobulin molecule is comprised of two identical light (L) chains and two identical heavy (H) chains. Light chains are composed of one constant domain (C_{L}) and one variable domain (V_{L}) while heavy chains are consist of three constant domains (C_{H}1, C_{H}2 and C_{H}3) and one variable domain (V_{H}). Together, the V_{H} and V_{L} domains compose the antigen-binding portion of the molecule known as the Fv. The Fc portion is glycosylated at a conserved Asn297 residue. Attachment of *N*-glycan at this position results in an "open" conformation that is essential for effector interaction.

Monoclonal antibodies can be made using recombinant DNA methods, as described in U.S. Patent No. 4,816,567 to Cabilly et al. and Anderson et al., "Production Technologies for Monoclonal Antibodies and their Fragments," Curr Opin Biotechnol. 15:456-62 (2004). The polynucleotides encoding a monoclonal antibody are isolated, such as from mature B-cells or hybridoma cell, such as by RT-PCR using oligonucleotide primers that specifically amplify the genes encoding the heavy and light chains of the antibody, and their sequence is determined using conventional procedures. The isolated polynucleotides encoding the heavy and light chains are then cloned into suitable expression vectors, which are then transfected into the host cells of the present invention, and monoclonal antibodies are generated. In one embodiment, recombinant DNA techniques are used to modify the heavy and light chains with N-terminal export signal peptides (e.g., PelB signal peptide) to direct the heavy and light chain polypeptides to the bacterial periplasm. Also, the heavy and light chains can be expressed from either a bicistronic construct (e.g., a single mRNA that is translated to yield the two polypeptides) or, alternatively, from a two cistron system (e.g., two separate mRNAs are produced for each of the heavy and light chains). To achieve high-level expression and efficient assembly of full-length IgGs in the bacterial periplasm, both the bicistronic and two cistron constructs can be manipulated to achieve a favorable expression ratio. For example, translation levels can be raised or lowered using a series of translation initiation regions (TIRs) inserted just upstream of the bicistronic and two cistron constructs in the expression vector (Simmons et al., "Translational Level is a Critical Factor for the Secretion of Heterologous Proteins in Escherichia coli," Nat Biotechnol 14:629-34 (1996)). When this antibody producing plasmid is introduced into a bacterial host that also harbors plasmid- or genome-encoded genes for expressing glycosylation enzymes, the resulting antibodies are glycosylated in the periplasm. Recombinant monoclonal antibodies or fragments thereof of the desired species can also be isolated from phage display libraries as described (McCafferty et al., "Phage Antibodies: Filamentous Phage Displaying Antibody Variable Domains," Nature 348:552-554 (1990); Clackson et al., "Making Antibody Fragments using Phage Display Libraries," Nature 352:624-628 (1991); and Marks et al., "By-Passing Immunization. Human Antibodies from V-Gene Libraries Displayed on Phage," J. Mol. Biol. 222:581-597 (1991)).

The polynucleotide(s) encoding a monoclonal antibody can further be modified in a number of different ways using recombinant DNA technology to generate alternative antibodies. In one embodiment, the constant domains of the light and heavy chains of, for example, a mouse monoclonal antibody can be substituted for those regions of a human antibody to generate a chimeric antibody. Alternatively, the constant domains of the light and heavy chains of a mouse monoclonal antibody can be substituted for a non-immunoglobulin polypeptide to generate a fusion antibody. In other embodiments, the constant regions are truncated or removed to generate the desired antibody fragment of a monoclonal antibody. Furthermore, site-directed or high-density mutagenesis of the variable region can be used to optimize specificity and affinity of a monoclonal antibody.

In some embodiments, the antibody of the present disclosure is a humanized antibody. Humanized antibodies are antibodies that contain minimal sequences from non-human (e.g. murine) antibodies within the variable regions. Such antibodies are used therapeutically to reduce antigenicity and human anti-mouse antibody responses when administered to a human subject. In practice, humanized antibodies are typically human antibodies with minimal to no non-human sequences. A human antibody is an antibody produced by a human or an antibody having an amino acid sequence corresponding to an antibody produced by a human.

Humanized antibodies can be produced using various techniques known in the art. An antibody can be humanized by substituting the complementarity determining region (CDR) of a human antibody with that of a non-human antibody (e.g. mouse, rat, rabbit, hamster, etc.) having the desired specificity, affinity, and capability (Jones et al., "Replacing the Complementarity-Determining Regions in a Human Antibody With Those From a Mouse," Nature 321:522-525 (1986); Riechmann et al., "Reshaping Human Antibodies for Therapy," Nature 332:323-327 (1988); Verhoeyen et al., "Reshaping Human Antibodies: Grafting an Antilysozyme Activity," Science 239:1534-1536 (1988)). The humanized antibody can be further modified by the substitution of additional residues either in the Fv framework region and/or within the replaced non-human residues to refine and optimize antibody specificity, affinity, and/or capability.

Bispecific antibodies are also suitable for use in the methods of the present disclosure. Bispecific antibodies are antibodies that are capable of specifically recognizing and binding at least two different epitopes. Bispecific antibodies can be intact antibodies or antibody fragments. Techniques for making bispecific antibodies are common in the art (Traunecker et al., "Bispecific Single Chain Molecules (Janusins) Target Cytotoxic Lymphocytes on HIV Infected Cells," EMBO J. 10:3655-3659 (1991) and Gruber et al., "Efficient Tumor Cell Lysis Mediated by a Bispecific Single Chain Antibody Expressed in Escherichia coli," J. Immunol. 152:5368-74 (1994)).

### Glycosylated Glucagon Peptide Production in Prokaryotes

Simple *in vitro* glycoconjugation techniques have been demonstrated to improve glucagon peptides, however drawbacks of therapeutic such peptides still exist as they are small and generally monomeric, have short half-lives of generally less than a few hours and PEGylation very rarely works well with small peptides. Current approaches still suffer from activity that is significantly inhibited.

The present disclosure relates to novel glycosylated peptides with desired glycans. Advantages of glycosylated glucagon peptide include improved solubility, improved physical stability toward gel and fibril formation, with increased half-life and improved activity and pharmacokinetic properties. Other advantages include the capability of a single or simultaneous *in vivo* process to produce both protein and glycans thereby avoiding multiple steps. In some embodiments, the novel glycosylated glucagon peptides have prolonged exposure *in vivo* due to prolonged plasma elimination half-life and a prolonged absorption phase and improved aqueous solubility at neutral pH or slightly basic pH. In other embodiments, the present disclosure has improved stability towards formation of gels and fibrils in aqueous solutions. In preferred embodiments, the predominant *N*-glycan is one that does not illicit immunogenicity to mammals.
*N*-glycosylation site occupancy can vary in eukaryotic systems, e.g., CHO and yeast for any particular glycoproteins produced. Growth conditions can be made to control occupancy at sites.

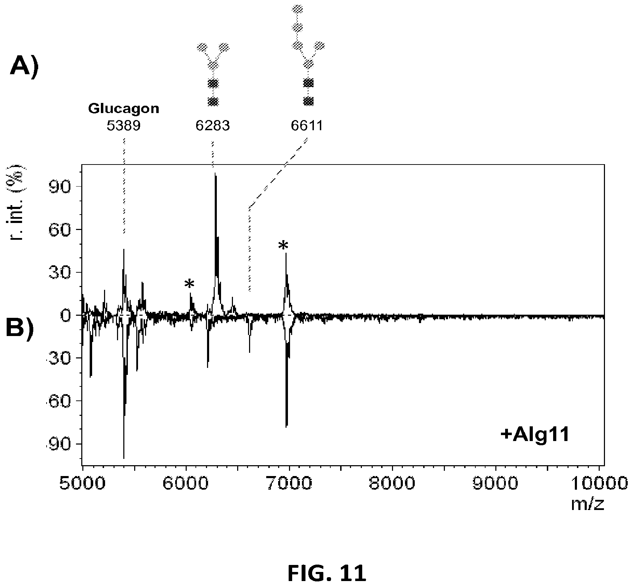

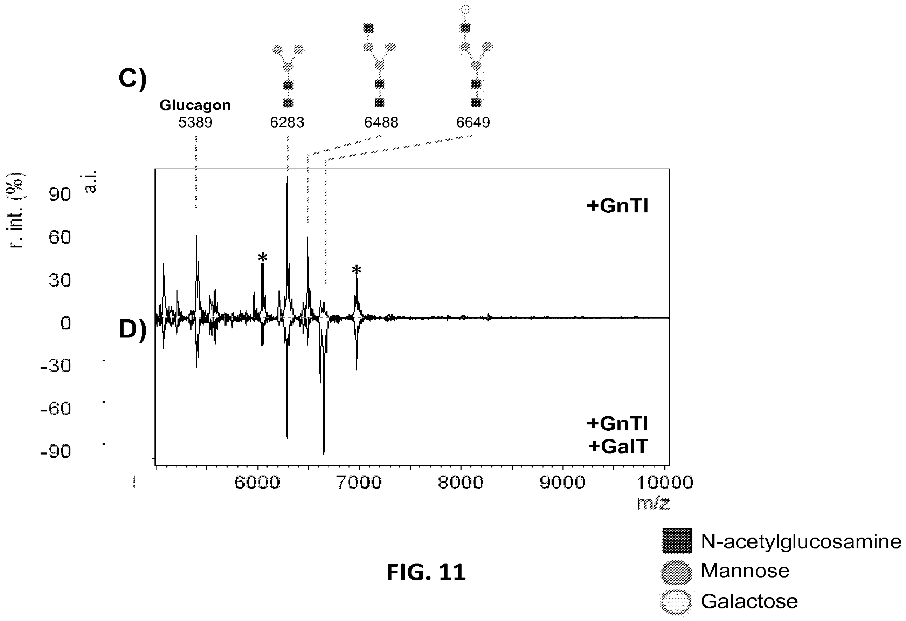

Typically, glucagon peptide has no glycosylation. In certain embodiments, glycosylation sites are engineered onto the peptide. In an exemplary embodiment, the glucagon peptide of the present invention has one glycosylation site. In certain embodiments, the method provides adding multiple glycans per peptide to confer better activity. In further embodiments, the host cells are engineered to produce glucagon peptides, with specific *N*-glycan as the predominant species. Exemplary glycosylation patterns are shown in **FIG. 11****.**

Accordingly, the methods of the present disclosure provide glycoproteins and glycopeptides comprising one or more glycoforms. Preferably, the glycoforms include M4, M5, G0, G0(1), G0(2), G0(3), G1, G2, G3, G4, G5, S1, S2, S3, S4, S5 which confer improved solubility or stability properties as well as increased receptor binding activity. In comparison to aglycosylated peptides, such as glucagon, the present invention is expected to increase half-life for the peptide. Additional peptides have been produced by the methods of the prevention invention such as hGH, ASNase, and IL1-Ra. Production of other peptides are within the scope of the invention. In preferred embodiments, at least 50 mol% of glucagon peptide is glycosylated.

### Vaccine Preparation

A generalized method to enhance immunogenicity of candidate antigens would reduce the time and costs invested in the early stages of vaccine development and could be applied to any disease of interest. One documented strategy to enhance immunogenicity is mannosylation, the conjugation of mannose-terminal glycans to proteins. Mannose targets antigens to specific receptors including CD206 and CD209 on antigen presenting cells (APC) for internalization by receptor-mediated endocytosis resulting in up to a 200-fold increase in antigen presentation compared to antigens taken up via pinocytosis (Engering, A., et al., The mannose receptor functions as a high capacity and broad specificity antigen receptor in human dendritic cells. Eur J Immunol, 1997. 27(9): p. 2417-25. Lam, J.S., et al., A Model Vaccine Exploiting Fungal Mannosylation to Increase Antigen Immunogenicity. The Journal of Immunology, 2005. 175(11): p. 7496-7503.). Mannosylation of antigens confers several advantages including: (i) increased antigen uptake by APC, (ii) enhanced MHC class II-mediated antigen presentation by up to 10,000-fold, (iii) promotion of T cell proliferation and maturation, and (iv) improved humoral immune response including bactericidal activity of serum (Arigita, C., et al., Liposomal Meningococcal B Vaccination: Role of Dendritic Cell Targeting in the Development of a Protective Immune Response. Infection and Immunity, 2003. 71(9): p. 5210-5218.). *E. coli* has not been used as a platform for vaccine production primarily because it does not naturally encode a pathway for *N*-glycosylation and has therefore been unsuitable for the manufacture of glycoproteins.

In certain embodiments, the present disclosure provides methods and compositions for mannosylated vaccine antigens through glycoengineered strains of *E.* coli. The effect of mannosylation on immunogenicity is assessed in a mouse model. The ability to produce vaccine candidates in bacteria provides multiple advantages. *E. coli* is an excellent platform for expression of ExPEC (extraintestinal pathogenic) and other bacterial proteins, offers facile recombinant DNA manipulation, can be used to generate large combinatorial libraries, allows for rapid and low cost strain development and quick ramp-up to production, and eliminates the risk for viral contamination encountered with eukaryotic expression systems (Aguilar-Yanez, J., et al., An influenza A/H1N1l2009 hemagglutinin vaccine produced in Escherichia coli. PLoS One, 2010. 5(7): p. e11694. Choi, B.-K., et al., Use of combinatorial genetic libraries to humanize N-linked glycosylation in the yeast Pichia pastoris. Proceedings of the National Academy of Sciences, 2003. 100(9): p. 5022-5027.). Production of mannosylated candidate antigens in *E. coli* would allow for synthesis of the desired glycoprotein *in vivo* without the need for further chemical or enzymatic modification. Accordingly, a new paradigm for vaccine development is provided by a method to augment the efficacy of *E*. *coli*-produced vaccine candidates.

Glycoengineered *E. coli* of the present disclosure is contemplated to produce mannosylated proteins with enhanced immunogenicity. Synthesis of mannosylated antigens in *E. coli* represents a significant advance in vaccine development allowing for inexpensive, rapid production of candidate proteins with enhanced immunogenic properties. In the past, several strategies have been employed for mannosylating antigens including *in vitro* chemical conjugation of mannan or mannose-terminal glycans, *in vivo* expression of proteins in *Pichia pastoris* for glycosylation with yeast high mannose oligosaccharides, or *in vitro* encapsulation of antigen in a mannosylated liposome (Lam, J.S., et al., Arigita, C., et al., Apostolopoulos, V., et al., Oxidative/reductive conjugation of mannan to antigen selects for T1 or T2 immune responses. Proceedings of the National Academy of Sciences, 1995. 92(22): p. 10128-10132. Sheng, K., et al., Delivery of antigen using a novel mannosylated dendrimer potentiates immunogenicity in vitro and in vivo. Eur J Immunol, 2008. 38(2): p. 424-36.). However, to date, the direct *in vivo* conjugation of mannose-terminal glycans to proteins in bacteria for vaccine development has never been achieved. An *E. coli* expression platform would provide multiple advantages over existing technologies both in terms of general protein production and an ectopic host for expression of glycans.

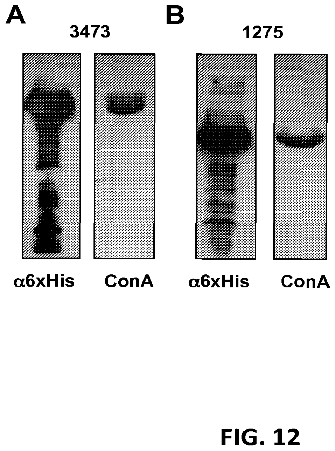

A uropathogenic *E. coli* (UPEC) antigen, c1275, was selected for preliminary expression and glycosylation. The c1275 protein was targeted to the periplasm of the glycoengineered *E. coli,* modified with a GlycTag (Fig 3a), and co-expressed with the OST PglB and the glycosyltransferases necessary to assemble the *C. jejuni* heptasaccharide glycan. Upon purification, the glycosylated c1275 was evident based on the appearance of slower-migrating bands on a Western blot and reactivity of these products with the GalNAc specific lectin soybean agglutinin (SBA), which is known to recognize this oligosaccharide (**FIG. 12**) (Young, N.M., et al., Structure of the N-linked glycan present on multiple glycoproteins in the Gram-negative bacterium, Campylobacter jejuni. J Biol Chem, 2002. 277(45): p. 42530-9.). Interestingly, glycosylation of c1275 is not necessarily dependent on the presence of the GlycTag. Sequence analysis reveals the presence of a native DSNAT motif in this protein that satisfies the published acceptor site requirements for PglB. Successful glycosylation of c1275 supports the hypothesis that candidate vaccine antigens can be successfully expressed and glycosylated in the *E. coli* periplasm.

The conventional vaccines induce merely a humoral immune response. DNA vaccines hold great promise since they evoke both humoral and cell-mediated immunity, without the same dangers associated with live virus vaccines. In contrast to live attenuated virus vaccines DNA vaccines may be delivered to same or different tissue or cells than the live virus that has to bind to specific receptors. The production of antigens in their native forms improves the presentation of the antigens to the host immune system. Unlike live attenuated vaccines, DNA vaccines are not infectious and can not revert to virulence.

Candidate antigens are modified with the various oligosaccharides such as Man₃GlcNAc₂. This can result in generation of antigens modified with a eukaryotic mannose-terminal glycan for use in vaccine formulations. Numerous target antigens are selected from a published assessment of ExPEC vaccine candidates that are known to confer protection in a mouse model. It should be pointed out, however, that the invention is highly modular and thus could be widely applied to enhance vaccine development for a variety of protein and peptide candidates.

### Pharmaceutical Formulations

Therapeutic formulations of the glycoprotein can be prepared by mixing the glycoprotein having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICST™ or polyethylene glycol (PEG).

A glycan is a convenient anchor for a PEG polymer because certain sugars, such as mannose or galactose, can easily be converted to reactive aldehydes in the presence of a mild oxidizer such as sodium periodate (Soares, A.L., et al., Effects of polyethylene glycol attachment on physicochemical and biological stability of E. coli L-asparaginase. Int J Pharm, 2002. 237(1-2): p. 163-70). A PEG polymer functionalized with a hydrazine group can then be used to create a glycoPEGylated bioconjugate. This allows the synthesis of site-specific, highly controlled, homogeneous, and active protein conjugates. PEGylation often results in problems of heterogeneity and activity loss as a result of the often non-specific process. Site-specific PEGylation methods involve either: (i) mutating lysines to allow PEG targeting to a specific lysine (Narimatsu, S., et al., Lysine-deficient lymphotoxin-alpha mutant for site-specific PEGylation. Cytokine, 2011. 56(2): p. 489-93. Youn, Y.S. and K.C. Lee, Site-specific PEGylation for high-yield preparation of Lys(21)-amine PEGylated growth hormone-releasing factor (GRF) (1-29) using a GRF(1-29) derivative FMOC-protected at Tyr(1) and Lys(12). Bioconjug Chem, 2007. 18(2): p. 500-6) or to the amine group of the N-terminus (Lee, H., et al., N-terminal site-specific mono-PEGylation of epidermal growth factor. Pharm Res, 2003. 20(5): p. 818-25. Yamamoto, Y., et al., Site-specific PEGylation of a lysine-deficient TNF-alpha with full bioactivity. Nat Biotechnol, 2003. 21(5): p. 546-52) or (ii) adding unpaired cysteine residues to allow targeting of free thiol groups (Shaunak, S., et al., Site-specific PEGylation of native disulfide bonds in therapeutic proteins. Nat Chem Biol, 2006. 2(6): p. 312-3. Doherty, D.H., et al., Site-specific PEGylation of engineered cysteine analogues of recombinant human granulocyte-macrophage colony-stimulating factor. Bioconjug Chem, 2005. 16(5): p. 1291-8. Manjula, B.N., et al., Site-specific PEGylation of hemoglobin at Cys-93(beta): correlation between the colligative properties of the PEGylated protein and the length of the conjugated PEG chain. Bioconjug Chem, 2003. 14(2): p. 464-72). These approaches have some major drawbacks. First, positively charged lysines are often important for protein structure/function (Yoshioka, Y., et al., Optimal site-specific PEGylation of mutant TNF-alpha improves its antitumor potency. Biochem Biophys Res Commun, 2004. 315(4): p. 808-14). Second, adding cysteine residues creates serious problems with soluble expression and disulphide bond formation, and can even require moving to a mammalian expression host (Constantinou, A., et al., Site-specific polysialylation of an antitumor single-chain Fv fragment. Bioconjug Chem, 2009. 20(5): p. 924-31). Third, site-specific PEGylation severely limits the number of linked PEG molecules. GlycoPEGylation, involves conjugation of PEG to glycans that are already attached to specific residues within proteins. The advantages are that: (i) the process is site-specific, (ii) glycosylation sites can be engineered away from the active site(s), and (iii) the product can be highly active and relatively homogeneous.

The formulation herein may also contain more than one active compound as necessary for the particular indication being treated, preferably those with complementary activities that do not adversely affect each other. For instance, the formulation may further comprise another antibody or a chemotherapeutic agent. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes. Sustained-release preparations may be prepared. Suitable examples of sustained-release preparations include semipermeable matrices of solid hydrophobic polymers containing the glycoprotein, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

The pharmaceutical composition may be lyphilized. Lyophilized antibody formulations are described in U.S. Pat. No. 6,267,958. Stable aqueous antibody formulations are described in U.S. Pat. No. 6,171,586B1.

In certain aspects, the methods and compositions of the present invention can be used for non-therapeutic purposes, such as assays, diagnostics, reagents and kits.

### Kits

The disclosure further provides an article of manufacture and kit containing oligosaccharide materials. The article of manufacture comprises a container with a label. Suitable containers include, for example, bottles, vials, and test tubes. The containers may be formed from a variety of materials such as glass or plastic. The container holds a composition comprising the oligosaccharide preparations described herein. In other embodiments, the kit includes the glycoprotein. The label on the container indicates that the composition is used for the treatment or prevention of a particular disease or disorder, and may also indicate directions for *in vivo,* such as those described above. The kit of the disclosure comprises the container described above and a second container comprising a buffer. It may further include other materials desirable from a commercial and user standpoint, including other buffers, diluents, filters, needles, syringes, and package inserts with instructions for use.

Ultimately, synthesis of the various glycoforms in prokaryotes (e.g., *E. coli*) facilitates attachment to a protein, incorporation into a glycan array, and utilization as a substrate to produce other human-like, *N*-linked glycans, diagnostics, kits or reagents.

The above disclosure generally describes the present invention. A more specific description is provided below in the following examples. The examples are described solely for the purpose of illustration and are not intended to limit the scope of the present invention. Changes in form and substitution of equivalents are contemplated as circumstances suggest or render expedient. Although specific terms have been employed herein, such terms are intended in a descriptive sense and not for purposes of limitation.

### EXAMPLES

### Example 1 - Plasmid Construction

Vaderrama-Rincon et al. recently disclosed a biosynthetic pathway for the biosynthesis and assembly of Man₃GlcNAc₂ on Und-PP in the cytoplasmic membrane of *E. coli.* The pathway, which comprises Alg13 Alg14 Alg1 and Alg2 activities with either wild-type nucleotide sequences or codon optimized sequences confers eukaryotic glycosyltransferase activity to the prokaryotic host cell. This pathway serves to add GlcNAc and mannose units to undecaprenol-linked carrier substrate yielding a trimannosyl core oligosaccharide structure. *E. coli* possesses an integral membrane protein WecA that mediates the transfer of GlcNAc-1-phosphate from UDP-GlcNAc onto undecaprenyl phosphate (Und-P) to form Und-PP-GlcNAc (Rick, P.D. & Silver, R.P. in Escherichia coli and Salmonella: Cellular and Molecular Biology. (ed. F.C.a.o. Neidhardt) 104-122 (American Society for Microbiology, Washingtion, D.C.; 1996). Thus, natively produced Und-PP-GlcNAc exists as a candidate precursor for the desired Man₃GlcNAc₂ glycan. For the addition of the second GlcNAc residue, the *Saccharomyces cerevisiae* β1,4-GlcNAc transferase that is comprised of two subunits, Alg13 and Alg14 was expressed. In yeast, Alg14 is an integral membrane protein that functions as a membrane anchor to recruit soluble Alg13 to the cytosolic face of the ER, where catalysis to Dol-PP-GlcNAc₂ occurs (Bickel, T. et al., Biosynthesis of lipid-linked oligosaccharides in Saccharomyces cerevisiae: Alg13p and Alg14p form a complex required for the formation of GlcNAc(2)-PP-dolichol. J Biol Chem 280, 34500-34506 (2005)). When co-expressed in *E*. *coli,* Alg14 was observed to localize in the membrane fraction while Alg13 was found in both the cytoplasm and membrane fractions, consistent with the situation in yeast. For the subsequent steps, *S. cerevisiae* β1,4-mannosyltransferase Alg1, which attaches the first mannose to the glycan, and the bifunctional mannosyltransferase Alg2, which catalyzes the addition of both the α1,3- and α1,6-mannose residues to the glycan was expressed (O'Reilly, M.K., et al., In vitro evidence for the dual function of Alg2 and Alg11: essential mannosyltransferases in N-linked glycoprotein biosynthesis. Biochemistry 45, 9593-9603 (2006)). Following expression in *E. coli,* both Alg1 and Alg2 localized in cell membranes. To determine if the correctly localized Alg enzymes were capable of producing Man₃GlcNAc₂ on Und-PP, a plasmid pYCG (Valderrama-Rincon et al.) that permits simultaneous expression of Alg13, Alg14, Alg1 and Alg2 was constructed.

Plasmid pMW07 was constructed (Valderrama-Rincon et. al.) Plasmid pMQ70 (Shanks et. al., 2006 AEM. 72(7)5027-5036.) was linearized with Ahd1 which is an isoschizomer of Eam11051. The p15a ori and *cat* gene were amplified from pBAD33 and used to co-transform yeast with the linearized vector pMQ70. Homologous recombination in yeast resulted in replacement of the colE1 ori and *bla* gene generating vector pMW07 (Valderrama-Rincon et al.). Table 3 lists the construction and genotype of various strains.

### Example 2 - Analytical Protocols

The method for extraction and purification of the *N*-linked oligosaccharide was followed as described in Gao et al. (Gao et al., "Non-radioactive analysis of lipid linked oligosaccharide composition by fluorophore-assisted carbohydrate electrophoresis," Method Enzymol 415: 3-20). The purified oligosaccharides were analyzed by MALDI-TOF mass spectrometry using dihydroxybenzoic acid (DHB) as the matrix (AB Sciex TOF/TOF 5800).

The glycan figures are in standard CFG (Consortium for Functional Genomics) black and white notation, which were generated in GlycoWorkbench 2.0.

### Example 3 - Production of human-like N-linked Man₅GlcNAc₂ high mannose oligosaccharide in E. coli

In humans, and other eukaryotes, the Man₅GlcNAc₂ glycoform is a key intermediate in glycan synthesis. In eukaryotes, this key glycoform is synthesized on the cytosolic side of the endoplasmic reticulum membrane. The enzyme Alg11 catalyzes the addition of two, α1,2-mannose residues to the α 1,3 mannose of the Man₃GlcNAc₂ glycan core. The gene encoding Alg11 from *Saccharomyces cerevisiae* was cloned as a fusion to the gene (*gst*) encoding glutathione S-transferase into plasmid pMW07-YCG-PglB.CO which is used for production of the Man₃GlcNAc₂ trimannosyl core (Valderrama-Rincon et al.) The resulting plasmid was transformed into *E. coli* MC4100 Δ*waaL gmd*::*kan* by electroporation (Gly02). Gly02 was grown in 100 mL of Luria-Bertani (LB) broth and induced by adding 0.2% (v/v) arabinose once the culture reached an optical density of 3.0.

Analysis of the purified oligosaccharides by mass spectrometry revealed a predominant peak (m/z 1257.6 Na+) consistent with the desired Man₅GlcNAc₂ glycoform (**FIG. 1A**). In some samples, a minor peak appeared, which was consistent with the Man₃GlcNAc₂ glycoform (m/z 933.5 Na+). In other examples, minor peaks including glycans consistent with Man₂GlcNAc₂, Man₄GlcNAc, Man₃GlcNAc₂, HexMan₃GlcNAc₂, HexMan₅GlcNAc Man₆GlcNAc appeared. To confirm the addition of the expected α1,2 mannose residues to the Man₃GlcNAc₂ glycan core, purified glycans were treated with a α1,2 mannosidase (Prozyme) according to manufacturer's protocol. Following incubation with the enzyme, glycans were labeled and analyzed by mass spectrometry and a FACE gel in the method of Gao et al. In the untreated sample, a predominant peak consistent with the Man₅GlcNAc₂ glycoform was observed (not shown). In the treated sample, a predominant peak (m/z 933.4 Na+) consistent with a Man₃GlcNAc₂ glycoform was observed (**FIG. 1B**). This confirms the expected addition of two α1,2-mannose residues to the Man₃GlcNAc₂ glycan core. As a result, the human-like Man₅GlcNAc₂ glycoform can be produced by expression of Alg11 in *E. coli.* Isolation of the Man₅GlcNAc₂ glycoform is challenging by other means since, in eukaryotes, it is a transient oligosaccharide. Synthesis of Man₅GlcNAc₂ in this system was also challenging due to difficulty in expression of a sufficient amount of active enzyme. Various fusion partners, along with Alg11 alone, were explored and resulted in the lack of efficient product formation for majority of the Alg11 moieties examined. The gst and mstX fused to Alg11 produced the Man₅GlcNAc₂ glycoform in this system.

### Example 4 - Production of Hybrid N-linked GlcNAcMan₃GlcNAc₂ oligosaccharide in E. coli

In humans, and other eukaryotes, the GlcNAcMan₃GlcNAc₂ glycoform is a key intermediate in glycan synthesis. This glycoform is typically only found on N-linked glycans attached to proteins in the Golgi of eukaryotes. Here the glycan was assembled on a lipid carrier in *E. coli.* To accomplish this, the gene encoding a truncated form (residues 30-446) of *Nicotiana tabaccum N-*acetylglucosaminyltransferase I (GnTI) was synthesized. The GnTI gene was amplified by PCR and subcloned into the plasmid pMQ70 as a fusion to the gene (malE) encoding *E. coli* maltose binding protein (MBP) lacking its native signal sequence. The resulting pMQ70-MBP-NtGnTI was transformed into *E. coli* MC4100 Δ*waaL gmd*::*kan* (Gly03) and Origami2 *gmd*::*kan* (Gly03.1) by electroporation along with a second plasmid pMW07-YCG-PglB.CO for production of the Man₃GlcNAc₂ trimannosyl core (Valderrama-Rincon et al.) and grown in 100 mL of Luria-Bertani (LB) broth. Glycosyltransferase expression was induced by adding 0.2% (v/v) arabinose once the culture reached an optical density of 3.0.

Analysis of the purified oligosaccharides by mass spectrometry revealed a predominant peak (m/z 1136.5 Na+) consistent with the desired GlcNAcMan₃GlcNAc₂ glycoform (**FIG. 2A**). A minor peak was consistent with the Man₃GlcNAc₂ glycoform (m/z 933.4 Na+). To confirm the addition of the expected GlcNAc to the Man₃GlcNAc₂ glycan core, purified glycans were treated with a β-N-acetylglucosaminidase (New England Biolabs) according to manufacturer's protocol. Following incubation with the enzyme, glycans were labeled and analyzed by mass spectrometry, and a FACE gel in the method of Gao et al. In the untreated sample, a predominant peak consistent with the GlcNAcMan₃GlcNAc₂ glycoform was observed (not shown). In the treated sample, the predominant peak is consistent with a Man₃GlcNAc₂ glycoform (**FIG. 2B**). This confirms the expected addition of a β-GlcNAc to the Man₃GlcNAc₂ glycan core. As a result, the human-like GlcNAcMan₃GlcNAc₂ glycoform can be produced by expression of GnTI in *E. coli.* Isolation of the GlcNAcMan₃GlcNAc₂ glycoform is challenging by other means since, in eukaryotes, it is a transient oligosaccharide. Obstacles were also encountered using this system, where expression of human GnTI alone, or fused to mstX, in *E. coli* was first attempted and did not efficiently produce the desired GlcNAcMan₃GlcNAc₂ glycoform (figure not shown). Moreover, when not fused to MBP, the *N. tabaccum* GnTI failed to produce the desired product (figure not shown).

### Example 5 - Production of N-linked GlcNAc₂Man₃GlcNAc₂ complex oligosaccharide in E. coli

In humans, and other eukaryotes, the GlcNAc₂Man₃GlcNAc₂ complex glycoform ("G0") is a key intermediate in glycan synthesis, as it is the "core" by which the glycan is fully decorated. This glycoform is typically only found on *N-*linked glycans attached to proteins in the Golgi of eukaryotes. Here the glycan was assembled on a lipid carrier in *E. coli.* To accomplish this, the gene encoding a truncated form (residues 30-447) of human N-acetylglucosaminyltransferase II (GnTII) was synthesized. The GnTII gene was amplified by PCR and subcloned into the plasmid pMQ70 as a fusion to MBP lacking its native signal sequence. The resulting pMQ70-MBP-hGnTII was transformed into *E. coli* MC4100 Δ*waaL gmd*::*kan,* (gly06) Origami2 *gmd*::*kan* (Gly06.1), DR473 *gmd*::*kan* (gly06.2) and Shuffle Δ*waaL gmd*::*kan* (Gly06.3) by electroporation along with a second plasmid pMW07-YCG-MBP-NtGnTI for production of the GlcNAcMan₃GlcNAc₂ substrate oligosaccharide. Glycosyltransferase expression was induced with 0.2% (v/v) arabinose, added immediately upon inoculation into 1 L of Luria-Bertani (LB) broth.

Analysis of the purified oligosaccharides by mass spectrometry revealed a predominant peak (m/z 1339.8 Na+) consistent with the desired GlcNAc₂Man₃GlcNAc₂ glycoform (**FIG. 3**). A minor peak was consistent with the Man₃GlcNAc₂ glycoform (m/z 933.5 Na+). A second minor peak consistent with GlcNAcMan₃GlcNAc₂ (m/z 1136.6 Na+) was also observed in the spectrum. Expression of GnTII in the glycoengineered E. coli proved to be challenging, where GnTII from three organisms were examined by expression alone, or when fused to mstX or MBP. Additionally, GnTII expression was examined in both oxidative and non-oxidative bacterial strains. Of the six GnTII moieties and four bacterial strains examined, efficient production of the GlcNAc₂Man₃GlcNAc₂ glycan was seen with MBP-fused, human GnTII in one of the four bacterial strains (figure not shown).

### Example 6 - Production of Branched N-linked GlcNAc₂Man₃GlcNAc₂ hybrid oligosaccharide in E. coli

Synthesis of multiantennary, *N*-linked glycans is a common feature in humans and other eukaryotes. Production of triantennary oligosaccharides is accomplished by the addition of a GlcNAc residue to GlcNAc₂Man₃GlcNAc₂ by *N-*acetylglucosaminyltransferase IV (GnTIV). GnTIV can also act on GlcNAcMan₃GlcNAc₂, producing a biantennary, hybrid oligosaccharide that is a structural isomer of the GlcNAc₂Man₃GlcNAc₂ complex glycan. The bacterial codon optimized gene encoding a truncated form (residues 93-535) of bovine GnTIV was synthesized. The GnTIV gene was amplified by PCR and subcloned into the plasmid pMQ70 as a fusion to MBP lacking its native signal sequence. The resulting pMQ70-MBP-hGnTIV was transformed into *E. coli* MC4100 Δ*waaL gmd*::*kan* (Gly05) and Origami2 *gmd*::*kan* (Gly05.1) by electroporation along with a second plasmid pMW07-YCG-MBP-NtGnTI for production of the GlcNAcMan₃GlcNAc₂ substrate oligosaccharide. Glycosyltransferase expression was induced with 0.2% (v/v) arabinose, added immediately upon inoculation into 1 L of Luria-Bertani (LB) broth.

Analysis of the purified oligosaccharides by mass spectrometry revealed a predominant peak (m/z 1339.7 Na+) consistent with the desired GlcNAc₂Man₃GlcNAc₂ glycoform (**FIG. 4A**). In some samples, a minor peak was consistent with the Man₃GlcNAc₂ glycoform (m/z 933.5 Na+). To confirm the addition of the expected GlcNAc to the GlcNAcMan₃GlcNAc₂ glycan, purified glycans were treated with a β-N-acetylglucosaminidase (New England Biolabs) according to manufacturer's protocol. Following incubation with the enzyme, glycans were labeled and analyzed by mass spectrometry. In the untreated sample, a predominant peak consistent with the GlcNAc₂Man₃GlcNAc₂ glycoform was observed (not shown). In the treated sample, the predominant peak is consistent with a Man₃GlcNAc₂ glycoform (**FIG. 4B**). This confirms the expected addition of a β-GlcNAc to the GlcNAcMan₃GlcNAc₂ glycan core. Expression of GnTIV in the glycoengineered *E. coli* proved to be challenging, where GnTIV expression was examined in both oxidative and non-oxidative bacterial strains. Efficient production of the GlcNAc₂Man₃GlcNAc₂ glycan was only seen in the oxidative bacterial strain (figure not shown).

### Example 7 - Production of Multiple Antennary N-linked GlcNAc₃Man₃GlcNAc₂ hybrid oligosaccharide in E. coli

Synthesis of triantennary, *N*-linked glycans is a feature found in humans and other eukaryotes. Production of one such triantennary oligosaccharide is accomplished by the addition of a UDP-GlcNAc residue to GlcNAc₂Man₃GlcNAc₂ by *N*-acetylglucosaminyltransferase IV (GnTIV). The codon optimized gene encoding bovine GnTIV was synthesized. The GnTIV gene was amplified by PCR and subcloned past the 3'-end of the human GnTII gene in the plasmid pMQ70-MBP-hGnTII. The resulting construct was transformed into *E. coli* cells (Origami2 *gmd*::*kan*) by electroporation along with a second plasmid pMW07-YCG-MBP-NtGnTI for production of the GlcNAcMan₃GlcNAc₂ substrate oligosaccharide to create strain GLY06.1. Glycosyltransferase expression was induced with 0.2% (v/v) arabinose, added immediately upon inoculation into 1 L of Luria-Bertani (LB) broth. The method for extraction and purification of the *N*-linked oligosaccharide was followed as described in Gao et al. The purified oligosaccharides were analyzed by MALDI-TOF mass spectrometry using DHB as the matrix (AB Sciex TOF/TOF 5800).

Analysis of the purified oligosaccharides by mass spectrometry revealed two predominant peaks with m/z values of 1339.9 (Na+) and 1543.1 (Na+) consistent with the substrate GlcNAc₂Man₃GlcNAc₂ and the GlcNAc₃Man₃GlcNAc₂ product glycoform, respectively (**FIG. 5B**).

To generate the substrate oligosaccharide G0(1), a 1 L dense culture of GLY06 was induced with 0.2% v/v arabinose for 20 hr at 30 °C. The G0 oligosaccharide was isolated by following the methods described in Gao et al. The glycosyltransferases were expressed in a separate, 100 mL culture by induction with 0.2% v/v arabinose for 16 hr at 25°C. This culture was pelleted by centrifugation and resuspended in 2 ml of GnTIV activity buffer (50 mM tris, 10 mM MnCl₂, pH 7.5) and sonicated. The lysate was clarified by centrifugation and 20 uL was added to the dried trimannosyl core substrate (∼5µg). An excess of nucleotide-sugar (20 µg) was added to the reaction and subsequently incubated at 30°C. The reaction was monitored by MALDI-TOF mass spectrometry at various time points over a 24 hr period.

Analysis of the purified oligosaccharides by mass spectrometry revealed a peak (m/z 1542.9 Na+) consistent with the desired GlcNAc₃Man₃GlcNAc₂ glycoform (**FIG. 5A**).

### Example 7 - Production of Hybrid N-linked GalGlcNAc₂Man₃GlcNAc₂ hybrid oligosaccharide in E. coli

In humans, and other eukaryotes, GalGlcNAc₂Man₃GlcNAc₂ glycoform is an intermediate in glycan synthesis. This glycoform is somewhat atypical in healthy adults, but has been seen in individuals with prostate cancer (Kyselova et al., "Alterations in the serum glycome due to metastatic prostate cancer," J. Proteome Res. (2007)). Here the glycan was assembled on a lipid carrier in *E. coli.* The gene encoding *Helicobacter pylori* β-1,4-galactosyltransferase (GalT) was synthesized, amplified by PCR, and subcloned into the plasmid pMQ70. The resulting pMQ70-HpGalT was transformed into MC4100 Δ*waaL gmd*::*kan* (Gly04) and Origami2 *gmd*::*kan* (Gly04.1) by electroporation along with a second plasmid pMW07-YCG-MBP-NtGnTI for production of the GlcNAcMan₃GlcNAc₂ substrate oligosaccharide. Glycosyltransferase expression was induced with 0.2% (v/v) arabinose, added immediately upon inoculation into 1 L of Luria-Bertani (LB) broth.

Analysis of the purified oligosaccharides by mass spectrometry revealed a predominant peak (m/z 1298.7 Na+) consistent with the desired GalGlcNAcMan₃GlcNAc₂ glycoform (**FIG. 6**). In some samples, a minor peak was consistent with the Man₃GlcNAc₂ glycoform (m/z 933.5 Na+) (figure not shown). Expression of GalT in the glycoengineered *E. coli* proved to be challenging, where the GalT from bovine and human, both unfused and fused to MBP and mstX, and *Neisseria meningitides* did not produce the desired oligosaccharide in *E. coli* (not shown). Moreover, expression of *H. pylori* GalT was examined in both oxidative and non-oxidative bacterial strains and efficient galactosylation by was only seen in the oxidative bacterial strain.

### Example 8 - Production of N-linked Gal₂GlcNAc₂Man₃GlcNAc₂ complex oligosaccharide in E. coli

In humans, and other eukaryotes, Gal₂GlcNAc₂Man₃GlcNAc₂ glycoform is a key intermediate in glycan synthesis. This glycoform is typically only found on *N*-linked glycans attached to proteins in eukaryotes.

In one sample, the glycan was produced *ex vivo* using the G0 oligosaccharides produced from Gly06 and the methods as described in **Example 7**. Analysis of the purified oligosaccharides by mass spectrometry revealed a predominant peak (m/z 1664.1 Na+) consistent with the desired GalGlcNAc₂Man₃GlcNAc₂ glycoform (**FIG. 7A**).

For *in vivo* synthesis of terminally galactosylated glycans, the gene encoding *Helicobacter pylori* β-1,4-galactosyltransferase (GalT) was synthesized, amplified by PCR, and subcloned into the plasmid pMQ132. The resulting pMQ132-HpGalT was transformed into Origami2 *gmd*::*kan* (Gly04.2) by electroporation along with a second plasmid pMW07-YCG-MBP-NtGnTI and a third plasmid pMQ70-MBP-hGnTII for production of the GlcNAc₂Man₃GlcNAc₂ substrate oligosaccharide. Glycosyltransferase expression was induced with 0.2% (v/v) arabinose, added immediately upon inoculation into 1 L of Luria-Bertani (LB) broth.

Analysis of the purified oligosaccharides by mass spectrometry revealed a predominant peak (m/z 1664.1 Na+) consistent with the desired Gal₂GlcNAc₂Man₃GlcNAc₂ glycoform (**FIG. 7A**). Analysis of glycans synthesized in Gly04.2 revealed a peak (m/z 1662.2 Na+) consistent with G2 glycoform, a peak (m/z 1500.0 Na+) consistent with the G1 glycoform, and a peak (m/z 1337.9 Na+) consistent with G0 glycoform. The same challenges described in **Example 7** were encountered when producing the Gal₂GlcNAc₂Man₃GlcNAc₂ glycoform in *E. coli,* since the same enzyme was used to produce both products.

### Example 8 - Production of N-linked NANAGalGlcNAcMan₃GlcNAc₂ hybrid oligosaccharide in E. coli

To generate the substrate oligosaccharide GalGlcNAcMan₃GlcNAc₂, a 1 L dense culture of GLY04.1 was induced with 0.2% v/v arabinose for 20 hr at 30 °C. The substrate oligosaccharide was isolated by following the methods described in Gao et al. The ST6 was expressed in a separate, 100 mL culture by induction with 0.2% v/v arabinose for 16 hr at 25°C. This culture was pelleted by centrifugation and resuspended in 2 ml of ST6 activity buffer (50 mM tris, 10 mM MnCl₂, pH 7.5) and sonicated. The lysate was clarified by centrifugation and 20 uL was added to the dried trimannosyl core substrate (∼5gg). An excess of nucleotide-sugar (20 µg) was added to the reaction and subsequently incubated at 30°C. The reaction was monitored by MALDI-TOF mass spectrometry at various time points over a 24 hr period.

Analysis of the purified oligosaccharides by mass spectrometry revealed a peak (m/z 1565.7 Na+) consistent with the desired NANAGalGlcNAcMan₃GlcNAc₂ glycoform (**FIG. 8**).

### Example 9 - Optimization of N-linked glycan yield in E. coli

There are a number of methods to increasing the amount N-linked glycans produced in the glycoengineered *E. coli* that include: (i) increased glycoprotein production (ii) and facilitating the production of glycoanalytical tools, such as glycan arrays. Therefore, improvement to the yield of the trimannosyl core glycan, the Man₅GlcNAc₂ glycan, and addition of GlcNAc residues to the trimannosyl core were undertaken. Understanding that the nucleotide-sugar pool in *E. coli* may be limiting, enzymes in the nucleotide-sugar biosynthesis pathway were targeted for overexpression in the glycoengineered *E. coli.* Specifically phosphomannomutase (ManB), mannose-1-phosphate guanylyltransferase (ManC), and glutamine-fructose-6-phosphate transaminase (GlmS) were investigated, where ManB and ManC are involved in the formation of GDP-Mannose and GlmS is involved in formation of UDP-GlcNAc.

The genes encoding ManB and ManC from *E. coli* were bicistronically (ManC/ManB) cloned into the plasmid pMQ70 and transformed into *E. coli* MC4100 Δ*waaL gmd*::*kan* along with pMW07-YCG-PglB.CO for production of the Man₃GlcNAc₂ trimannosyl core (Valderrama-Rincon et al.) by electroporation (Gly01.2). The gene encoding GlmS from *E. coli* was cloned into the plasmid pTrc99Y (Valderrama-Rincon et al.) and transformed into *E. coli* MC4100 Δ*waaL gmd*::*kan* along with pMW07-YCG-MBP-NtGnTI by electroporation (Gly01.3). *E. coli* MC4100 Δ*waaL gmd*::*kan* containing pMW07-YCG-PglB.CO (Gly01) and *E. coli* MC4100 Δ*waaL gmd*::*kan* containing pMW07-YCG-MBP-NtGnTI (Gly01.1) were used as controls. Gly01 and Gly01.2 were grown in 100 mL of Luria-Bertani (LB) broth and expression was induced with 0.2% (v/v) arabinose at an optical density (O.D.) of 3. Gly01.1 and Gly01.3 were grown in 100 mL LB broth and expression was induced with 0.2% (v/v) arabinose and 1 mM IPTG (Gly01.3 only) at an O.D. of 3. The method for extraction and purification of the *N*-linked oligosaccharide was followed as described in Gao et al. The purified oligosaccharides were analyzed by *fluorophore-assisted carbohydrate electrophoresis* (*FACE*) using the methods described in Gao et al.

In the case of Gly01.2, a large increase in the production of the trimannosyl core was observed when compared to Gly01 (**FIG. 9A** left panel). However, difficulty lied within quantifying the difference in yield, since the Gly01 trimannosyl core band was virtually undetectable. Similarly, in the case of Gly01.3, a large increase in glycan yield was observed when compared to Gly01.1 (**FIG. 9A** right panel). Additionally, a large increase in GlcNAcMan₃GlcNAc₂ was observed when compared to Gly01.1, which was the goal of targeting this enzyme for overexpression. Since there are a number of enzymes involved in nucleotide-sugar biosynthesis, careful consideration was made in determining which enzymes to target for overexpression in the glycoengineered *E. coli,* where a number of the enzymes may have little effect on glycan yields.

Glycerol provides a carbon source alternative to glucose so as not to effect gene expression from plasmids via promoter repression, as cAMP levels remain high in *E. coli* with excess glycerol. Use of glycerol appears to increase glycan yield as shown in **FIG. 9B****.** Pyruvate plays a role in recycling GDP to GTP in the Krebs cycle. GTP is a substrate of GDP-mannose pyrophosphorylase that is required for GDP-mannose formation. Increased glycan yield is also contemplated with the addition of pyruvate **FIG. 9C****.**

Analysis of the purified oligosaccharides by mass spectrometry of host cells with overexpression of ManC/B revealed virtual elimination of the minor peaks as compared to the host cells without ManC/B overexpression. GLY01.4 produced a single predominant peak (m/z 933.5 Na+) consistent with the desired M3 glycoform (**Fig. 10D**). GLY02.1 produced a single predominant peak (m/z 1257.7 Na+) consistent with the desired M5 glycoform (**Fig. 10E**). GLY01.5 produced a single predominant peak (m/z 1136.9 Na+) consistent with the desired hybrid GlcNAcMan₃GlcNAc₂ glycoform (**Fig. 10F**).

### Example 10 - Glycosylated Glucagon Production in E. coli

The glucagon construct consists of glucagon with an *N*-linked glycosylation site (DQNAT) followed by a six-histidine tag at the C-terminus. Glucagon is expressed as a fusion to the C-terminus of MBP after three consecutive C-terminal TEV protease sites in the vector pTrc99Y. The genes encoding for ManC and ManB were also cloned into this vector past the 3' end of the glucagon coding region. The resulting plasmid was transformed into *E. coli* cells (Origami2Δ*waaL*, *gmd*::*kan*) cells by electroporation along with a corresponding glycosyltransferase plasmid. A 100mL culture of each strain was grown to an optical density at 600nm of ∼2.0 and induced with 0.2% v/v arabinose for 16 hr followed by induction with 0.1 mM IPTG for 8hr at 30°C. Cells were harvested by centrifugation and resuspended in lysis buffer (50 mM PO4 buffer, 300 mM NaCl, pH 8.0), sonicated, and spun to remove debris. The clarified cell lysate was loaded onto a pre-equilibrated Ni-NTA spin column (Qiagen) and washed with buffer containing 30 mM imidazole. The fusion protein was eluted with 200µL of 300 mM imidazole. Eluted protein was subsequently incubated with 1µg of TEV protease (Sigma Aldrich) at 30°C. Samples were analyzed by mass spectrometry at various time points over a 24hr period.

Analysis of MALDI-TOF MS of partially purified glucagon appended with a C-terminal glycosylation site was as follows: from strain (**FIG. 11A**) GLY01.6 consistent with the expected Man₃GlcNAc₂ glycopeptide (m/z 6283), (**FIG. 11B**) GLY02.2 consistent with the expected GlcNAcMan₅GlcNAc₂ glycopeptide (m/z 6611), (**FIG. 11C****)** GLY01.7 consistent with the expected GlcNAcMan₃GlcNAc₂ glycopeptide (m/z 6488), and (**FIG. 11D****)** GLY04.3 consistent with the expected GalGlcNAcMan₃GlcNAc₂ glycopeptide (m/z 6649). Asterisks indicate background signals present in all samples independent of glycosyltransferases.

### Example 11 - Mannosylated Vaccine Production in E. coli

**Cloning and expressing genes with candidate antigens.** Successful expression of candidate antigens in preparation for glycosylation studies requires that proteins encode an acceptor asparagine and are expressed in the periplasm. A GlycTag containing four iterations of an *N*-glycosylation sequon optimized for the bacterial OST PglB is employed. The signal peptide from *E. coli* maltose binding protein (MBP) which is exported via the Sec pathway and performs well in export of ectopic proteins for glycosylation is used (Fisher, A.C., et al., Production of Secretory and Extracellular N-Linked Glycoproteins in Escherichia coli. Applied and Environmental Microbiology, 2011. 77(3): p. 871-881.) Proteins are expressed from the isopropyl-β-D-thiogalactopyranoside (IPTG)-inducible TRC promoter to provide appropriate expression levels for use in glycosylation studies (Fisher et al.).

**Mannosylation of *E. coli* vaccine antigens**. Genes encoding candidate vaccine antigens c1275 and 3473 (Moriel, et. al., PNAS 2010) from pathogenic ExPEC *E. coli* were cloned as a fusion to the C-terminus of mature MBP and were modified at their C-terminus with four consecutive glycosylation sequons (4xGlycTag) and a hexahistidine tag. The signal peptide from DsbA was utilized to target proteins to the periplasm. The resulting plasmids were paired with pMW07-YCG-PglB in *E*. *coli* cells (MC4100 Δ*waaL*Δ*gma*::*kan*) by electroporation. After inoculation in to 10L cultures of each strain was grown to an approximate optical density at 600nm of 3.0 and induced with the addition of 0.2% (v/v) arabinose and 1mM IPTG. Glycoprotein was isolated by ConA affinity chromatography followed by Nickel affinity chromatography, as previously (Valerrama-Rincon, et. al.). The partially purified samples were analyzed Western blot using an anti-hexahistidine antibody and the ConA lectin (**FIG. 12**).

**Modify antigens with an asparagine-linked mannose-terminal glycan.** Following successful attachment of the *C*. *jejuni* heptasaccharide, mannose-terminal glycan is attached to the candidate antigens. The paucimannose oligosaccharide structure is present as normal human *N*-glycans, and it is currently in use in a human therapeutic (Van Patten, S.M., et al., Effect of mannose chain length on targeting of glucocerebrosidase for enzyme replacement therapy of Gaucher disease. Glycobiology, 2007. 17(5): p. 467-478.) suggesting the glycan itself is tolerated in humans. Plasmid (pYCG-PglB) expresses the OST PglB and four glycosyltransferases from *S. cerevisiae:* Alg13, Alg14, Alg1, and Alg2 (Valderrama et al.). These proteins coordinate the synthesis and conjugation of the Man₃GlcNAc₂ glycan and its derivatives, which forms the base of the human complex *N*-glycan.

Candidate antigens verified by glycosylation with the *C. jejuni* heptasaccharide is individually co-expressed with pYCG-PglB in glycosylation host strain MC4100 Δ*waaL gmd::kan.* Following induction of glycosylation pathway enzymes and antigen, cells are lysed and the target protein isolated with the ConA lectin which binds α-linked mannose residues. Because the engineered glycan terminates with α-mannose residues, purification with ConA favors the recovery of proteins modified with the complete desired glycan. Nickel-affinity chromatography is used to further purify the mannosylated antigen and a portion of the proteins is subjected to treatment with PNGase F to cleave off the glycan. Analysis by SDS-PAGE followed by immunoblotting with ConA and the αHis antibody verify recovery of the expected mannosylated protein. To confirm that the attached glycans are Man₃GlcNAc₂, PNGase F-released glycans are subject to mass spectrometry as described in (Valderrama-Rincon et al.). This process is expected to yield homogeneous, bacterially-derived mannosylated target antigens. Mannosylated antigen is detected by Western blot with the lectin ConA, and confirmation of the glycan identity by mass spectrometry.

**Evaluate immunogenicity of mannosylated antigens.** Mannosylated and aglycosylated vaccine antigens are purified and the immunogenic properties of the target antigens are assessed. Using a mouse model, markers of both the humoral and cell-mediated immune responses to mannosylated ExPEC antigens are examined.

**Purification of mannosylated antigen.** Mannosylated antigens are purified using lectin-affinity chromatography on a ConA column followed by nickel purification. Aglycosylated antigens are similarly purified by nickel-affinity chromatography. Preparations are compared to ensure similar purity by silver stain and endotoxin levels are determined for each sample. A suitable amount of mannosylated protein and aglycosylated protein of similar purity are recovered to conduct immunogenicity studies.

**Test binding of mannosylated antigens by human myeloid (mDC).** Mannosylated or aglycosylated antigens are incubated with mDCs to assess binding to the mannose receptor (Wieser, A., et al., A Multiepitope Subunit Vaccine Conveys Protection against Extraintestinal Pathogenic Escherichia coli in Mice. Infection and Immunity, 2010. 78(8): p. 3432-3442.). Following washing, cells are fixed and surface-bound antigen are detected with an αHis -FITC antibody using flow cytometry. Competition with free mannose or mannan validate the role of the mannose receptor in specific binding of mannosylated antigens (Wieser et al.). This step serves as preliminary validation of the mannosylated antigens prior to assessment of immunogenicity in a mouse model.

**Measure immunogenicity of mannosylated antigens.** The immune response of mice following subcutaneous administration of mannosylated antigens are evaluated compared to aglycosylated controls. This step is an important validation for the use of the Man₃GlcNAc₂ glycan as an enhancer of antigenicity for vaccine candidates. Groups of six CD1 mice (Charles River Laboratories) are immunized subcutaneously with e.g., 20 µg of antigen on day 1, 21, and 35. CD1 mice have been used previously as a sepsis model for ExPEC vaccine studies using the same immunization timeline (Moriel, D.G., et al.,) and thus, these experiments pave the way for future challenge studies. Serum collected two weeks after the final immunization and ELISA are used to quantify the humoral response including the titers of IgG and IgM (Park, S.-U., et al., Immunization with a DNA vaccine cocktail induces a Th1 response and protects mice against Mycobacterium avium subsp. paratuberculosis challenge. Vaccine, 2008. 26(34): p. 4329-4337.). The antigen-specific response are evaluated in comparison to an unrelated control protein bearing both a GlycTag and 6x-His tag. A CD8+ T cell assay are used to quantify the cellular response (Sivick, K.E. and H.L.T. Mobley, Waging War against Uropathogenic Escherichia coli: Winning Back the Urinary Tract. Infection and Immunity, 2010. 78(2): p. 568-585.) because both humoral and cell-mediated immunity may play a role in combating ExPEC infections (Thumbikat, P., et al., Antigen-Specific Responses Accelerate Bacterial Clearance in the Bladder. The Journal of Immunology, 2006. 176(5): p. 3080-3086. Nallamsetty, S. and D. Waugh, Solubility-enhancing proteins MBP and NusA play a passive role in the folding of their fusion partners. Protein Expr Purif, 2006. 45(1): p. 175-82.).

Model antigens are constructed as fusion proteins with the normal E*. coli* periplasmic resident MBP. An N-terminal MBP fusion can promote proper folding and export from the cytoplasm which in turn can improve glycosylation (Nallamsetty et al.). Testing alternate signal peptide sequences can address improper localization. Attachment of the *C. jejuni* heptasaccharide to a protein modified with a terminal GlycTag is reliably achieved in all cases where sufficient target protein is properly localized to the periplasm and serves as a predictive indicator for glycosylation success with the Man₃GlcNAc₂ glycan. However, glycosylation may also be improved by adjusting the position of the GlycTag, or utilizing a different mannose-terminal glycan such as the poly-mannose LPS from *E. coli* O9 which has previously been conjugated to proteins in the bacterial *N*-glycosylation reaction (Wacker, M., et al., Substrate specificity of bacterial oligosaccharyltransferase suggests a common transfer mechanism for the bacterial and eukaryotic systems. Proceedings of the National Academy of Sciences, 2006. 103(18): p. 7088-7093.).

The effect of mannosylation on antigen binding to mDC and immunogenicity are assessed using a mouse model. The kinetics of antigen internalization will influence our ability to visualize mDC binding and the surface-bound antigen and if necessary, an inhibitor of endosomal trafficking is employed or internalized antigen in permeabilized cells is assessed. Quantification of the cell-mediated and antigen-specific humoral immune response is used to determine whether mannosylation of vaccine antigen candidates has an impact on these indicators of immunogenicity. Various antigens glycosylated with an alternate mannose glycan such as the polymannose LPS from *E. coli* O9 are evaluated. Alternatively, antigens can be modified with additional glycosylation sites to promote attachment of multiple glycans.

**Table 3. Strain and Plasmid List.**

| **Strain name** | **Plasmid 1** | **Plasmid 2** | **Plasmid 3** | ***E. coli* strain** | **Product** |
|---|---|---|---|---|---|
| GLY01 | pMW07-YCG-PglB.CO | - | - | MC4100ΔΔ Δ*waaL gmd*::*kan* | Man₃GlcNAc₂ |
| GLY01.1 | YCG-MBP-NtGnTI-PglB.CO | - | - | MC4100ΔΔ Δ*waaL gmd*::*kan* | GlcNAcMan₃GlcNAc₂ |
| GLY01.2 | pMW07-YCG-PglB.CO | pMQ70-ManC/B | - | MC4100ΔΔ Δ*waaL gmd*::*kan* | Man₃GlcNAc₂ |
| GLY01.3 | YCG-MBP-NtGnTI-PglB.CO | pTrc99Y-GlmS | - | MC4100ΔΔ Δ*waaL gmd*::*kan* | GlcNAcMan₃GlcNAc₂ |
| GLY01.4 | pMW07-YCG-PglB.CO | pMQ70-ManC/B | - | Origami2 *gmd*::*kan* | GlcNAcMan₃GlcNAc₂ |
| GLY01.5 | YCG-MBP-NtGnTI-PglB.CO | pMQ70-ManC/B | - | Origami2 *gmd*::*kan* | GlcNAcMan₃GlcNAc₂ |
| GLY01.6 | pMW07-YCG-PglB.CO | pTrc99Y-MBP-Glucagon | - | Origami2 *gmd*::*kan* | Man₃GlcNAc₂-Glucagon |
| | | -ManC/B | | Δ*waaL* | |
| GLY01.7 | YCG-MBP-NtGnTI-PglB.CO | pTrc99Y-MBP-Glucagon | - | Origami2 *gmd*::*kan* | GlcNAcMan₃GlcNAc₂-Glucagon |
| | | -ManC/B | | Δ*waaL* | |
| GLY02 | pMW07-YCG-GST-Algll-PglB.CO | - | - | MC4100ΔΔ Δ*waaL gmd*::*kan* | Man₅GlcNAc₂ |
| GLY02.1 | pMW07-YCG-GST-Algll-PglB.CO | pMQ70-ManC/B | - | Origami2 *gmd*::*kan* | Man₅GlcNAc₂ |
| GLY02.2 | pMW07-YCG-GST-Algll-PglB.CO | pTrc99Y-MBP-Glucagon | - | Origami2 *gmd*::*kan* | Man₅GlcNAc₂-Glucagon |
| | | -ManC/B | | Δ*waaL* | |
| GLY03 | pMW07-YCG-PglB.CO | pMQ70-MBP-NtGnTI | - | MC4100ΔΔ Δ*waaL gmd*::*kan* | GlcNAcMan₃GlcNAc₂ |
| GLY03.1 | pMW07-YCG-PglB.CO | pMQ70-MBP-NtGnTI | - | Origami2 *gmd*::*kan* | GlcNAcMan₃GlcNAc₂ |
| GLY04 | YCG-MBP-NtGnTI-PglB.CO | pMQ70-HpGalT | - | MC4100ΔΔ Δ*waaL gmd*::*kan* | GalGlcNAc-Man₃GlcNAc₂ |
| GLY04.1 | YCG-MBP-NtGnTI-PglB.CO | pMQ70-HpGalT | - | Origami2 *gmd*::*kan* | GalGlcNAc-Man₃GlcNAc₂ |
| GLY04.2 | YCG-MBP-NtGnTI-PglB.CO | pMQ70-MBP-hGnTII | pMQ132-HpGalT | Origami2 *gmd*::*kan* | Gal₂GlcNAc₂-Man₃GlcNAc₂ |
| GLY04.3 | YCG-MBP-NtGnTI-HpGalT-PglB.CO | pTrc99Y-MBP-Glucagon -ManC/B | - | Origami2 *gmd*::*kan* Δ*waaL* | GalGlcNAc-Man₃GlcNAc₂-Glucagon |
| GLY05 | YCG-MBP-NtGnTI-PglB.CO | pMQ70-MBP-bGnTIV | - | Origami2 *gmd*::*kan* | GlcNAc₂-Man₃GlcNAc₂ |
| GLY06 | YCG-MBP-NtGnTI-PglB.CO | pMQ70-MBP-hGnTII | - | Origami2 *gmd*::*kan* | GlcNAc₂Man₃GlcNAc₂ |
| GLY06.1 | YCG-MBP-NtGnTI-PglB.CO | pMQ70-MBP-hGnTII-bGnTIV | - | Origami2 *gmd*::*kan* | GlcNAc₃Man₃GlcNAc₂ |

Although preferred embodiments have been depicted and described in detail herein, it will be apparent to those skilled in the relevant art that various modifications, additions, substitutions, and the like can be made without departing from the spirit of the invention and these are therefore, considered to be within the scope of the present invention as defined the claims which follow.

### Informal Sequence Listing

SEQ ID NO: 1 alg13 codon optimized
SEQ ID NO: 2 alg13
SEQ ID NO: 3 alg14 codon optimized
SEQ ID NO: 4 alg14
SEQ ID NO: 5 alg1 codon optimized
SEQ ID NO: 6 alg1
SEQ ID NO: 7 alg2 codon optimized
SEQ ID NO: 8 alg2
SEQ ID NO: 9 alg11
SEQ ID NO: 10 Alg11 protein
SEQ ID NO: 11 malE (MBP)
SEQ ID NO: 12 MalE protein (MBP)
SEQ ID NO: 13 mstX
SEQ ID NO: 14 MstX protein
SEQ ID NO: 15 GnTI (EC 2.4.1.101)
SEQ ID NO: 16 GnTI protein
SEQ ID NO: 17 GnT II (EC 2.4.1.143)
SEQ ID NO: 18 GnT II (EC 2.4.1.143)
SEQ ID NO: 19 GnTIV (EC 2.4.1.145)
SEQ ID NO: 20 GnTIV (EC 2.4.1.145)
SEQ ID NO: 21 GalT (EC 2.4.1.38)
SEQ ID NO: 22 GalT (EC 2.4.1.38)
SEQ ID NO: 23 manB (EC 5.4.2.8)
SEQ ID NO: 24 manB (EC 5.4.2.8)
SEQ ID NO: 25 manC (EC 2.7.7.13)
SEQ ID NO: 26 manC (EC 2.7.7.13)
SEQ ID NO: 27 glmS (EC 2.6.1.16)
SEQ ID NO: 28 glmS (EC 2.6.1.16)
SEQ ID NO: 29 (EC 2.4.99.1) ST6
SEQ ID NO: 30 (EC 2.4.99.1) ST6
SEQ ID NO: 31 MBP-GnTI fusion
SEQ ID NO: 32 MBP-GnTII fusion
SEQ ID NO: 33 MBP-GnTIV fusion
SEQ ID NO: 34 GST-alg11 fusion

## Claims

1. A method of producing an oligosaccharide composition, said method comprising:
culturing a recombinant prokaryotic host cell that produces an oligosaccharide composition having a terminal mannose residue to express one or more N-acetylglucosaminyl transferase enzyme activity (EC 2.4.1.101; EC 2.4.1.143; EC 2.4.1.145; EC 2.4.1.155; EC 2.4.1.201) that catalyzes the transfer of a UDP-GlcNAc residue onto said terminal mannose residue, said culturing step carried out under conditions effective to produce an oligosaccharide composition having a terminal GlcNAc residue.

2. The method of claim 1, wherein the culturing step further comprises the host cell to express one or more galactosyltransferase enzyme activity (EC 2.4.1.38) that catalyzes the transfer of a UDP-Galactose residue onto said terminal GlcNAc residue, said culturing step carried out under conditions effective to produce an oligosaccharide composition having a terminal galactose residue.

3. The method of claim 2, wherein the culturing step further comprises the host cell to express one or more sialyltransferase enzyme activity (EC 2.4.99.4 and EC 2.4.99.1) that catalyzes the transfer of a CMP-NANA residue onto said terminal galactose residue, said culturing step carried out under conditions effective to produce an oligosaccharide composition having a terminal sialic acid residue.

4. The method of claim 1, 2 or 3, wherein
(a) the culturing step comprises the host cell to express one or more eukaryotic UDP-GlcNAc transferase enzyme activity (EC 2.4.1.141 or EC 2.4.1.145) and one or more eukaryotic mannosyltransferase enzyme activity (EC 2.4.1.142, EC 2.4.1.132) wherein the enzymes confer eukaryotic glycosyltransferase activity to the host cell;
(b) the culturing step further comprises the host cell to express a fusion of one or more of the enzymes wherein said fusion comprises at least one of the following solubility enhancers selected from: DsbA, GlpE, GST, MBP, MstX, NusA and TrxA
(c) the culturing step is under oxidative conditions;
(d) the culturing step comprises the host cell to express one or more of the following: phosphomannomutase enzyme activity (ManB) (EC 5.4.2.8), mannose-1-phosphate guanylyltransferase enzyme activity (ManC) (EC 2.7.7.13) and glutamine-fructose-6-phosphate transaminase enzyme activity (GlmS) (EC 2.6.1.16), wherein ManB and ManC catalyze GDP-Mannose synthesis and wherein GlmS catalyzes UDP-GlcNAc synthesis;
(e) the culturing step comprises the host cell having an attenuation in GDP-D-mannose dehydratase enzyme activity (EC 4.2.1.47);
(f) the culturing step comprises the host cell to express a flippase enzyme activity;
(g) the culturing step comprises the host cell to express an oligosaccharyl transferase enzyme activity (EC 2.4.1.119);
(h) the method further comprises introducing into the host cell a gene encoding a protein of interest, whereby the host cell produces a glycosylated protein;
(i) the culturing step comprises the host cell to express at least one *N-*acetylglucosaminyl transferase, galactosyltransferaes or sialyltransferase is operably fused to MBP; or
(j) the host cell is cultured in the presence of glycercol or pyruvate.

5. The method of claim 4(f), wherein the oligosaccharide composition is *N*-linked to the protein.

6. The method of claim 4(h), wherein
(a) the culturing step comprises the recombinant prokaryotic host cell to express said glycosylated protein as an antibody comprising at least one of the following: Fv portion which binds to a native antigen and an Fc portion which is glycosylated at a conserved asparagine residue, diabody, scFv, scFv-Fc, scFv-CH, Fab and scFab; or
(b) the glycosylated protein comprises cytokines such as interferons, G-CSF, coagulation factors such as factor VIII, factor IX, and human protein C, soluble IgE receptor α-chain, IgG, IgG fragments, IgM, interleukins, urokinase, chymase, and urea trypsin inhibitor, IGF-binding protein, epidermal growth factor, growth hormone-releasing factor, annexin V fusion protein, angiostatin, vascular endothelial growth factor-2, myeloid progenitor inhibitory factor-1, osteoprotegerin, α-1 antitrypsin, DNase II, α-feto proteins, AAT, rhTBP-1 (aka TNF binding protein 1), TACI-Ig (transmembrane activator and calcium modulator and cyclophilin ligand interactor), FSH (follicle stimulating hormone), GM -CSF, glucagon, glucagon peptides, GLP-1 w/ and w/o FC (glucagon like protein 1) IL-1 receptor agonist, sTNFr (aka soluble TNF receptor Fc fusion), CTLA4-Ig (Cytotoxic T Lymphocyte associated Antigen 4-Ig), receptors, hormones such as human growth hormone, erythropoietin, peptides, stapled peptides, human vaccines, animal vaccines, serum albumin and enzymes such as ATIII, rhThrombin, glucocerebrosidase and asparaginase.

7. The method of claim 2, wherein the culturing step comprises an oxidative host cell to express said galactosyltransferase enzyme activity.

8. The method of claim 1, wherein
(a) the oligosaccharide compositions comprises GlcNAc₁₋₅Man₃GlcNAc₂ and Man₃GlcNAc₂
(b) the oligosaccharide composition is predominantly GlcNAcMan₃GlcNAc₂ or GlcNAc₂Man₃GlcNAc₂;
(c) the oligosaccharide compositions comprises Gal₁₋₅GlcNAc₁₋₅Man₃GlcNAc₂ and Man₃GlcNAc₂;
(d) the oligosaccharide composition is predominantly GalGlcNAcMan₃GlcNAc₂, GalGlcNAc₂Man₃GlcNAc₂ or Gal₂GlcNAc₂Man₃GlcNAc₂;
(e) the oligosaccharide compositions comprises NANA₁₋₅Gal₁₋₅GlcNAc₁₋₅Man₃GlcNAc₂; or
(f) the oligosaccharide composition is predominantly NANAGalGlcNAcMan₃GlcNAc₂ or NANA₂Gal₂GlcNAc₂Man₃GlcNAc₂.

9. A recombinant prokaryotic host cell that produces an oligosaccharide composition having a terminal mannose residue comprising: one or more *N*-acetylglucosaminyl transferase enzyme activity (EC 2.4.1.101; EC 2.4.1.143; EC 2.4.1.145; EC 2.4.1.155; EC 2.4.1.201) that catalyzes the transfer of a UDP-GlcNAc residue onto said terminal mannose residue, wherein said host cell produces an oligosaccharide composition having a terminal GlcNAc residue.

10. The host cell of claim 9, wherein said host cell further comprises one or more galactosyltransferase enzyme activity (EC 2.4.1.38) that catalyzes the transfer of a UDP-Galactose residue onto said terminal GlcNAc residue, wherein said host cell produces an oligosaccharide composition having a terminal galactose residue.

11. The host cell of claim 10, wherein said host cell further comprises one or more sialyltransferase enzyme activity SialylT (EC 2.4.99.4 and SialylT (EC 2.4.99.1) that catalyzes the transfer of a CMP-NANA residue onto said terminal galactose residue, wherein said host cell produces an oligosaccharide composition having a terminal sialic acid residue.

12. The host cell of claim 9, 10 or 11, wherein
(a) said host cell comprises one or more eukaryotic UDP-GlcNAc transferase enzyme activity (EC 2.4.1.141 or EC 2.4.1.145) and one or more eukaryotic mannosyltransferase enzyme activity (EC 2.4.1.142, EC 2.4.1.132), wherein said enzymes confer eukaryotic glycosyltransferase activity to the host cell;
(b) said host cell further comprises a fusion of one or more of the enzymes wherein said fusion comprises at least one of the following: DsbA, GlpE, GST, MBP, MstX, NusA and TrxA;
(c) said host cell is an oxidative host;
(d) said host cell comprises one or more of the following enzymes: phosphomannomutase enzyme activity (ManB) (EC 5.4.2.8), mannose-1-phosphate guanylyltransferase enzyme activity (ManC) (EC 2.7.7.13) and glutamine-fructose-6-phosphate transaminase enzyme activity (GlmS) (EC 2.6.1.16), wherein ManB and ManC catalyze GDP-Mannose synthesis and wherein GlmS catalyzes UDP-GlcNAc synthesis;
(e) the host cell further comprises an attenuation in GDP-D-mannose dehydratase enzyme activity (EC 4.2.1.47);
(f) the host cell further comprises a flippase enzyme activity;
(g) the host cell further comprises an oligosaccharyl transferase enzyme activity (EC 2.4.1.119); or
(h) the host cell further comprises a gene encoding a protein of interest, whereby the host cell produces a glycosylated protein.

13. The host cell of claim 12(h), wherein
(a) the host cell produces an oligosaccharide composition that is N-linked to the protein;
(b) the host cell expresses said glycosylated protein as an antibody comprising at least one of the following: Fv portion which binds to a native antigen and an Fc portion which is glycosylated at a conserved asparagine residue, diabody, scFv, scFv-Fc, scFv-CH, Fab and scFab; or
(c) the glycosylated protein comprises cytokines such as interferons, G-CSF, coagulation factors such as factor VIII, factor IX, and human protein C, soluble IgE receptor α-chain, IgG, IgG fragments, IgM, interleukins, urokinase, chymase, and urea trypsin inhibitor, IGF-binding protein, epidermal growth factor, growth hormone-releasing factor, annexin V fusion protein, angiostatin, vascular endothelial growth factor-2, myeloid progenitor inhibitory factor-1, osteoprotegerin, α-1 antitrypsin, DNase II, α-feto proteins, AAT, rhTBP-1 (aka TNF binding protein 1), TACI-Ig (transmembrane activator and calcium modulator and cyclophilin ligand interactor), FSH (follicle stimulating hormone), GM-CSF, glucagon, glucagon peptides, GLP-1 w/ and w/o FC (glucagon like protein 1) IL-1 receptor agonist, sTNFr (aka soluble TNF receptor Fc fusion), CTLA4-Ig (Cytotoxic T Lymphocyte associated Antigen 4-Ig), receptors, hormones such as human growth hormone, erythropoietin, peptides, stapled peptides, human vaccines, animal vaccines, serum albumin and enzymes such as ATIII, rhThrombin, glucocerebrosidase and asparaginase.

14. The host cell of claim 9, wherein
(a) the host cell expresses at least one mannosyltransferase, N-acetylglucosaminyl transferase, galactosyltransferaes or sialyltransferase is operably fused to MBP;
(b) an oxidative host cell is used to express the galactosyltransferase enzyme activity;
(c) the host cell produces oligosaccharide compositions comprises GlcNAc₁₋₅Man₃GlcNAc₂ and Man₃GlcNAc₂., optionallywherein the oligosaccharide composition is predominantly GlcNAcMan₃GlcNAc₂ or GlcNAc₂Man₃GlcNAc₂; or
(d) the host cell produces oligosaccharide compositions comprises Gal₁₋₅GlcNAc₁₋₅Man₃GlcNAc₂ and Man₃GlcNAc₂, optionally wherein the oligosaccharide composition is predominantly GalGlcNAcMan₃GlcNAc₂, GalGlcNAc₂Man₃GlcNAc₂ or Gal₂GlcNAc₂Man₃GlcNAc₂.

15. The host cell of claim 11, wherein the oligosaccharide composition comprises NANA₁₋₅Gal₁₋₅GlcNAc₁₋₅Man₃GlcNAc₂, optionally wherein the oligosaccharide composition is predominantly NANAGalGlcNAcMan₃GlcNAc₂ or NANA₂Gal₂GlcNAc₂Man₃GlcNAc₂.

## Patentansprüche

1. Verfahren zur Herstellung einer Oligosaccharidzusammensetzung, wobei das Verfahren folgendes umfasst:
Kultivieren einer rekombinanten prokaryotischen Wirtszelle, die eine Oligosaccharidzusammensetzung mit einem terminalen Mannoserest erzeugt, um eine oder mehrere N-Acetylglucosaminyltransferase-Enzymaktivitäten (EC 2.4.1.101; EC 2.4.1.143; EC 2.4.1.145; EC 2.4.1.155; EC 2.4.1.201) zu exprimieren, wodurch die Übertragung eines UDP-GlcNAc-Rests auf den terminalen Mannoserest katalysiert wird, wobei der Kultivierungsschritt unter Bedingungen ausgeführt wird, die wirksam eine Oligosaccharidzusammensetzung mit einem terminalen GlcNAc-Rest erzeugen.

2. Verfahren nach Anspruch 1, wobei der Kultivierungsschritt es ferner umfasst, dass die Wirtszelle eine oder mehrere Galactosyltransferase-Enzymaktivitäten (EC 2.4.1.38) exprimiert, wodurch die Übertragung eines UDP-Galactoserests auf den terminalen GlcNAc-Rest katalysiert wird, wobei der Kultivierungsschritt unter Bedingungen ausgeführt wird, die wirksam eine Oligosaccharidzusammensetzung mit einem terminalen Galactoserest erzeugen.

3. Verfahren nach Anspruch 2, wobei der Kultivierungsschritt es ferner umfasst, dass die Wirtszelle eine oder mehrere eine oder mehrere Sialyltransferase-Enzymaktivitäten (EC 2.4.99.4 und EAC 2.4.99.1) exprimiert, wodurch die Übertragung eines CMP-NANA-Rests auf den terminalen Galactoserest katalysiert wird, wobei der Kultivierungsschritt unter Bedingungen ausgeführt wird, die wirksam eine Oligosaccharidzusammensetzung mit einem terminalen Sialinsäurerest erzeugen.

4. Verfahren nach Anspruch 1, 2 oder 3, wobei:
(a) der Kultivierungsschritt es umfasst, dass die Wirtszelle eine oder mehrere eukaryotische UDP-GlcNAc-Transferase-Enzymaktivitäten (EC 2.4.1.141 oder EC 2.4.1.145) und eine oder mehrere eukaryotische Mannosyltransferase-Enzymaktivitäten (EC 2.4.1.142, EC 2.4.1.132) exprimiert, wobei die Enzyme eukaryotische Glycosyltransferaseaktivität auf die Wirtszelle übertragen;
(b) der Kultivierungsschritt es umfasst, dass die Wirtszelle eine Verschmelzung eines oder mehrerer der Enzyme exprimiert, wobei die Verschmelzung wenigstens einen der folgenden Lösungsvermittler umfasst, die ausgewählt sind aus: DsbA, GlpE, GST, MBP, MstX, NusA und TrxA;
(c) der Kultivierungsschritt unter oxidativen Bedingungen ausgeführt wird;
(d) der Kultivierungsschritt es umfasst, dass eines oder mehrere der folgenden exprimiert wird bzw. werden: Phosphomannomutase-Enzymaktivität (ManB) (EC 5.4.2.8), Mannose-1-Phosphat-Guanylyltransferase-Enzymaktivität (ManC) (EC 2.7.7.13) und Glutamin-Fructose-6-Phosphat-Transaminase-Enzymaktivität (GlmS) (EC 2.6.1.16), wobei ManB und ManC GDP-Mannose-Synthese katalysieren, und wobei GlmS UDP-GlcNAc-Synthese katalysiert;
(e) der Kultivierungsschritt es umfasst, dass die Wirtszelle eine Abschwächung der GDP-D-Mannose-Dehydratase-Enzymaktivität (EC 4.2.1.47) umfasst;
(f) der Kultivierungsschritt es umfasst, dass die Wirtszelle eine Flippase-Enzymaktivität exprimiert;
(g) der Kultivierungsschritt es umfasst, dass die Wirtszelle eine Oligosaccharyltransferase-Enzymaktivität (EC 2.4.1.119) exprimiert;
(h) wobei das Verfahren es ferner umfasst, dass in die Wirtszelle ein Gen eingeführt wird, das für ein relevantes Protein kodiert, wodurch die Wirtszelle ein glykolysiertes Protein erzeugt;
(i) der Kultivierungsschritt es umfasst, dass die Wirtszelle wenigstens eine N-Acetylglucosaminyl-Transferase, Galactosyltransferase oder Sialyltransferase exprimiert, die funktionsfähig mit MBP verschmolzen ist; oder
(j) die Wirtszelle in Gegenwart von Glycerol oder Pyruvat kultiviert wird.

5. Verfahren nach Anspruch 4(f), wobei die Oligosaccharidzusammensetzung eine *N*-Verknüpfung mit dem Protein aufweist.

6. Verfahren nach Anspruch 4(h), wobei:
(a) der Kultivierungsschritt es umfasst, dass die rekombinante prokaryotische Wirtszelle das glykolysierte Protein als einen Antikörper exprimiert, der wenigstens eines der folgenden umfasst: einen Fv-Teil, der an ein natürliches Antigen bindet, und einen Fc-Teil, der an einem konservierten Asparaginrest, einen Diabody, scFv, scFv-Fc, scFv-CH, Fab und scFab glykolisiert ist; oder
(b) das glykolysierte Protein Cytokine umfasst, wie etwa Interferone, G-CSF, Gerinnungsfaktoren wie etwa Faktor VIII, Faktor IX und menschliches Protein C, löslichen IgE-Rezeptor α-Kette, IgG, IgG-Fragmente, IgM, Interleukine, Urokinase, Chymase und Harnstoff-Trypsin-Inhibitor, IGF-Bindungsprotein, epidermalen Wachstumsfaktor, Wachstumshormon-Freisetzungsfaktor, Annexin-V-Fusionsprotein, Angiostatin, vaskulären endothelialen Wachstumsfaktor-2, myeloiden Vorläufer hemmenden Faktor-1, Osteoprotegerin, α-1-Antitrypsin, DNase II, α-Fetoproteine, AAT, rhTBP-1 (aka TNF-Bindungsprotein 1), TACI-IG (Transmembranaktivator und Calciummodulator und Cyclophilinligand-Interaktor, FSH (follikelstimulierendes Hormon), GM-GSF, Glucagon, Glucagonpeptide, GLP-1 mit und ohne FC (Glucagonartiges Protein 1) IL-1-Rezeptoragonist, sTNFr (aka lösliche TNF-Rezeptor-Fc-Fusion), CTLA4-IG (cytotoxisches T-Lymphozyt assoziiertes Antigen 4-Ig), Rezeptoren, Hormone wie etwa menschliches Wachstumshormon, Erythropoietin, Peptide, geheftete Peptide, menschliche Impfstoffe, tierische Impfstoffe, Serumalbumin und Enzyme wie etwa ATIII, rhThrombin, Glucocerebrosidase und Asparaginase.

7. Verfahren nach Anspruch 2, wobei der Kultivierungsschritt eine oxidative Wirtszelle zum Exprimieren der Galactosyltransferase-Enzymaktivität umfasst.

8. Verfahren nach Anspruch 1, wobei:
(a) die Oligosaccharidzusammensetzungen GlcNAc₁₋₅Man₃GlcNAc₂ und Man₃GlcNAc₂ umfasst;
(b) die Oligosaccharidzusammensetzung überwiegend GlcNAcMan₃GlcNAc₂ oder GlcNAc₂Man₃GlcNAc₂ ist;
(c) die Oligosaccharidzusammensetzung Gal₁₋₅GlcNAc₁₋₅Man₃GlcNAc₂ und Man₃GlcNAc₂ umfasst;
(d) die Oligosaccharidzusammensetzung überwiegend GalGlcNAcMan₃GlcANc₂, GalGlcNAc₂Man₃GlcNAc₂ oder Gal₂GlcNAc₂Man₃GlcNAc₂ ist;
(e) die Oligosaccharidzusammensetzung NANA₁₋₅Gal₁₋₅GlcNAc₁₋₅Man₃GlcNAc₂ umfasst; oder
(f) die Oligosaccharidzusammensetzung überwiegend NANAGalGlcNAcMan₃GlcNAc₂ oder NANA₂Gal₂GlcNAc₂Man₃GlcNAc₂ ist.

9. Rekombinante prokaryotische Wirtszelle, die eine Oligosaccharidzusammensetzung mit einem terminalen Mannoserest erzeugt, umfassend: eine oder mehrere *N*-Acetylglucosaminyltransferase-Enzymaktivitäten (EC 2.4.1.101; EC 2.4.1.143; EC 2.4.1.145; EC 2.4.1.155; EC 2.4.1.201), wodurch die Übertragung eines UDP-GlcNAc-Rests auf den terminalen Mannoserest katalysiert wird, wobei die Wirtszelle eine Oligosaccharidzusammensetzung mit einem terminalen GlcNAc-Rest erzeugt.

10. Wirtszelle nach Anspruch 9, wobei die Wirtszelle eine oder mehrere Galactosyltransferase-Enzymaktivitäten (EC 2.4.1.38) umfasst, wodurch die Übertragung eines UDP-Galactoserests auf den terminalen GlcNAc-Rest katalysiert wird, wobei die Wirtszelle eine Oligosaccharidzusammensetzung mit einem terminalen Galactoserest erzeugt.

11. Wirtszelle nach Anspruch 10, wobei die Wirtszelle eine oder mehrere Sialyltransferase-Enzymaktivitäten (EC 2.4.99.4 und EAC 2.4.99.1) umfasst, wodurch die Übertragung eines CMP-NANA-Rests auf den terminalen Galactoserest katalysiert wird, wobei die Wirtszelle eine Oligosaccharidzusammensetzung mit einem terminalen Sialinsäurerest erzeugt.

12. Wirtszelle nach Anspruch 9, 10 oder 11, wobei:
(a) die Wirtszelle eine oder mehrere eukaryotische UDP-GlcNAc-Transferase-Enzymaktivitäten (EC 2.4.1.141 oder EC 2.4.1.145) und eine oder mehrere eukaryotische Mannosyltransferase-Enzymaktivitäten (EC 2.4.1.142, EC 2.4.1.132) umfasst, wobei die Enzyme eukaryotische Glycosyltransferaseaktivität auf die Wirtszelle übertragen;
(b) die Wirtszelle ferner eine Verschmelzung eines oder mehrerer der Enzyme umfasst, wobei die Verschmelzung wenigstens eines der folgenden umfasst: DsbA, GlpE, GST, MBP, MstX, NusA und TrxA;
(c) die Wirtszelle ein oxidativer Host ist;
(d) die Wirtszelle eines oder mehrere der folgenden Enzyme umfasst: Phosphomannomutase-Enzymaktivität (ManB) (EC 5.4.2.8), Mannose-1-Phosphat-Guanylyltransferase-Enzymaktivität (ManC) (EC 2.7.7.13) und Glutamin-Fructose-6-Phosphat-Transaminase-Enzymaktivität (GlmS) (EC 2.6.1.16), wobei ManB und ManC GDP-Mannose-Synthese katalysieren, und wobei GlmS UDP-GlcNAc-Synthese katalysiert;
(e) die Wirtszelle ferner eine Abschwächung der GDP-D-Mannose-Dehydratase-Enzymaktivität (EC 4.2.1.47) umfasst;
(f) die Wirtszelle ferner eine Flippase-Enzymaktivität umfasst;
(g) die Wirtszelle ferner eine Oligosaccharyltransferase-Enzymaktivität (EC 2.4.1.119) umfasst; oder
(h) die Wirtszelle ferner ein Gen umfasst, das für ein relevantes Protein kodiert, wodurch die Wirtszelle ein glykolysiertes Protein erzeugt.

13. Wirtszelle nach Anspruch 12(h), wobei:
(a) die Wirtszelle eine Oligosaccharidzusammensetzung erzeugt, die eine N-Verknüpfung mit dem Protein aufweist;
(b) die Wirtszelle das glykolysierte Protein als einen Antikörper exprimiert, der wenigstens eines der folgenden umfasst: einen Fv-Teil, der an ein natürliches Antigen bindet, und einen Fc-Teil, der an einem konservierten Asparaginrest, einen Diabody, scFv, scFv-Fc, scFv-CH, Fab und scFab glykolisiert ist; oder
(c) das glykolysierte Protein Cytokine umfasst, wie etwa Interferone, G-CSF, Gerinnungsfaktoren wie etwa Faktor VIII, Faktor IX und menschliches Protein C, löslichen IgE-Rezeptor α-Kette, IgG, IgG-Fragmente, IgM, Interleukine, Urokinase, Chymase und Harnstoff-Trypsin-Inhibitor, IGF-Bindungsprotein, epidermalen Wachstumsfaktor, Wachstumshormon-Freisetzungsfaktor, Annexin-V-Fusionsprotein, Angiostatin, vaskulären endothelialen Wachstumsfaktor-2, myeloiden Vorläufer hemmenden Faktor-1, Osteoprotegerin, α-1-Antitrypsin, DNase II, α-Fetoproteine, AAT, rhTBP-1 (aka TNF-Bindungsprotein 1), TACI-IG (Transmembranaktivator und Calciummodulator und Cyclophilinligand-Interaktor, FSH (follikelstimulierendes Hormon), GM-GSF, Glucagon, Glucagonpeptide, GLP-1 mit und ohne FC (Glucagonartiges Protein 1) IL-1-Rezeptoragonist, sTNFr (aka lösliche TNF-Rezeptor-Fc-Fusion), CTLA4-IG (cytotoxisches T-Lymphozyt assoziiertes Antigen 4-lg), Rezeptoren, Hormone wie etwa menschliches Wachstumshormon, Erythropoietin, Peptide, geheftete Peptide, menschliche Impfstoffe, tierische Impfstoffe, Serumalbumin und Enzyme wie etwa ATIII, rhThrombin, Glucocerebrosidase und Asparaginase.

14. Wirtszelle nach Anspruch 9, wobei:
(a) die Wirtszelle wenigstens eine Mannosyltransferase, N-Acetylglucosaminyl-Transferase, Galactosyltransferase oder Sialyltransferase exprimiert, die funktionsfähig mit MBP verschmolzen ist;
(b) eine oxidative Wirtszelle verwendet wird, um die Galactosyltransferase-Enzymaktivität zu exprimieren;
(c) die Wirtszelle Oligosaccharidzusammensetzungen erzeugt, die GlcNAc₁₋₅Man₃GlcNAc₂ und Man₃GlcNAc₂ umfassen, wobei die Oligosaccharidzusammensetzung optional überwiegend GlcNAcMan₃GlcNAc₂ oder GlcNAc₂Man₃GlcNAc₂ ist; oder
(d) die Wirtszelle Oligosaccharidzusammensetzungen erzeugt, die Gal₁₋₅GlcNAc₁₋₅Man₃GlcNAc₂ und Man₃GlcNAc₂ umfassen wobei die Oligosaccharidzusammensetzung optional überwiegend GalGlcNAcMan₃GlcANc₂, GalGlcNAc₂Man₃GlcNAc₂ oder Gal₂GlcNAc₂Man₃GlcNAc₂ ist.

15. Wirtszelle nach Anspruch 11, wobei die Oligosaccharidzusammensetzung NANA₁₋₅Gal₁₋₅GlcNAc₁₋₅Man₃GlcNAc₂ umfasst, wobei die Oligosaccharidzusammensetzung optional überwiegend NANAGalGlcNAcMan₃GlcNAc₂ oder NANA₂Gal₂GlcNAc₂Man₃GlcNAc₂ ist.

## Revendications

1. Procédé de production d'une composition d'oligosaccharide, ledit procédé comprenant les étapes consistant à :
cultiver une cellule hôte procaryote recombinante qui produit une composition d'oligosaccharide ayant un résidu mannose terminal pour exprimer une ou plusieurs activités enzymatiques de la N-acétylglucosaminyl transférase (EC 2.4.1.101 ; EC 2.4.1.143 ; EC 2.4.1.145 ; EC 2.4.1.155 ; EC 2.4.1.1.201) qui catalyse le transfert d'un résidu UDP-GlcNAc sur ledit résidu mannose terminal, ladite culture étant effectuée dans des conditions efficaces pour produire une composition d'oligosaccharide ayant un résidu GlcNAc terminal.

2. Procédé selon la revendication 1, la culture comprenant en outre l'étape dans laquelle la cellule hôte exprime une ou plusieurs activités enzymatiques de la galactosyltransférase (EC 2.4.1.38) qui catalyse le transfert d'un résidu UDP-Galactose sur ledit résidu GlcNAc terminal, ladite culture étant effectuée dans des conditions efficaces pour produire une composition d'oligosaccharide ayant un résidu galactose terminal.

3. Procédé selon la revendication 2, la culture comprenant en outre une étape dans laquelle la cellule hôte exprime une ou plusieurs activités enzymatiques de la sialyltransférase (EC 2.4.99.4 et EC 2.4.99.1) qui catalyse le transfert d'un résidu CMP-NANA sur ledit résidu galactose terminal, ladite culture étant effectuée dans des conditions efficaces pour produire une composition d'oligosaccharide ayant un résidu acide sialique terminal.

4. Procédé selon la revendication 1, 2 ou 3,
(a) la culture comprenant l'étape dans laquelle la cellule hôte exprime une ou plusieurs activités enzymatiques de la transférase UDP-GlcNAc eucaryote (EC 2.4.1.141 ou EC 2.4.1.145) et une ou plusieurs activités enzymatiques de la mannosyltransférase eucaryote (EC 2.4.1.142, EC 2.4.1.132), les enzymes conférant une activité de la glycosyltransférase eucaryote à la cellule hôte ;
(b) la culture comprenant en outre l'étape dans laquelle la cellule hôte exprime une fusion d'une ou plusieurs des enzymes, ladite fusion comprenant au moins l'un des exhausteurs de solubilité suivants choisis parmi : DsbA, GlpE, GST, MBP, MstX, NusA et TrxA
(c) la culture se déroulant dans des conditions d'oxydation ;
(d) la culture comprenant l'étape dans laquelle la cellule hôte exprime une ou plusieurs des activités suivantes : activité enzymatique de la phosphomannomutase (ManB) (EC 5.4.2.8), l'activité enzymatique de la mannose-1-phosphate guanylyltransférase (ManC) (EC 2.7.7.13) et l'activité enzymatique de la glutamine-fructose-6-phosphate transaminase (GlmS) (EC 2.6.1.16), ManB et ManC catalysant la synthèse GDP-Mannose et GlmS catalysant la synthèse UDP-GlcNAc ;
(e) la culture comprenant l'étape dans laquelle la cellule hôte a une atténuation dans l'activité enzymatique de la GDP-D-mannose déshydratase (EC 4.2.1.47) ;
(f) la culture comprenant l'étape dans laquelle la cellule hôte exprime une activité enzymatique de la flippase ;
(g) la culture comprenant l'étape dans laquelle la cellule hôte exprime une activité enzymatique de l'oligosaccharyltransférase (EC 2.4.1.119) ;
(h) le procédé comprenant en outre l'étape consistant à introduire dans la cellule hôte d'un gène codant pour une protéine d'intérêt, la cellule hôte produisant une protéine glycosylée ;
(i) la culture comprenant l'étape dans laquelle la cellule hôte exprime au moins une *N*-acétylglucosaminyltransférase, des galactosyltransférases ou une sialyltransférase étant fusionnée de manière opérationnelle à MBP ; ou
(j) la cellule hôte étant cultivée en présence de glycercol ou de pyruvate.

5. Procédé selon la revendication 4(f), la composition d'oligosaccharide étant *N*-liée à la protéine.

6. Procédé selon la revendication 4(h),
(a) la culture comprenant l'étape dans laquelle la cellule hôte procaryote recombinante exprime ladite protéine glycosylée sous la forme d'un anticorps comprenant au moins l'un des éléments suivants : partie Fv qui se lie à un antigène natif et une partie Fc qui est glycosylée au niveau d'un résidu asparagine conservé, diabody, scFv, scFv-Fc, scFv-CH, Fab et scFab ; ou
(b) la protéine glycosylée comprenant des cytokines telles que des interférons, G-CSF, facteurs de coagulation tels que facteur VIII, facteur IX et protéine C humaine, récepteur IgE soluble α-chain, IgG, fragments IgG, IgM, interleukines, urokinase, chymase et inhibiteur de trypsine d'urée, protéine liant l'IGF, facteur de croissance épidermique, facteur de croissance de l'hormone de croissance, protéine de fusion de l'annexine V, angiostatine, facteur de croissance endothéliale vasculaire-2, facteur inhibiteur du progéniteur myéloïde-1, ostéoprotégérine, α-1 antitrypsine, DNase II, protéines α-feto, AAT, rhTBP-1 (également appelé protéine de liaison TNF 1), TACI-Ig (activateur transmembranaire et modulateur du calcium et interacteur de ligand cyclophiline), FSH (hormone folliculo-stimulante), GM -CSF, glucagon, glucagon, peptides de glucagon, GLP-1 avec et sans agoniste des récepteurs IL-1 FC (glucagon-like protéine 1), sTNFr (également appelé fusion Fc de récepteur TNF soluble), CTLA4-Ig (lymphocyte T cytotoxique associé à l'antigène 4-Ig), récepteurs, hormones telles que l'hormone de croissance humaine, érythropoïétine, peptides, peptides agrafés, vaccins humains, vaccins animaux, albumine sérique et enzymes telles que ATIII, rhThrombine, glucocerebrosidase et asparaginase.

7. Procédé selon la revendication 2, la culture comprenant l'étape dans laquelle une cellule hôte oxydante exprime ladite activité enzymatique de la galactosyltransférase.

8. Procédé selon la revendication 1,
(a) les compositions d'oligosaccharide comprenant GlcNAc₁₋₅Man₃GlcNAc₂ et Man₃GlcNAc₂ ;
(b) la composition d'oligosaccharide étant principalement GlcNAcMan₃GlcNAc₂ ou GlcNAc₂Man₃GlcNAc₂ ;
(c) les compositions d'oligosaccharides comprenant Gal₁₋₅GlcNAc₁₋₅Man₃GlcNAc₂ et Man₃GlcNAc₂ ;
(d) la composition d'oligosaccharide étant principalement GalGlcNAcMan₃GlcNAc₂, GalGlcNAc₂Man₃GlcNAc₂ ou Gal₂GlcNAc₂Man₃GlcNAc₂ ;
(e) les compositions d'oligosaccharide comprenant NANA₁₋₅Gal₁₋₅GlcNAc₁₋₅Man₃GlcNAc₂ ; ou
(f) la composition d'oligosaccharide est principalement NANAGalGlcNAcMan₃GlcNAc₂ ou NANA₂Gal₂GlcNAc₂Man₃GlcNAc₂.

9. Cellule hôte procaryote recombinante qui produit une composition d'oligosaccharide ayant un résidu mannose terminal comprenant : une ou plusieurs activités enzymatiques de la *N*-acétylglucosaminyltransférase (EC 2.4.1.101 ; EC 2.4.1.143 ; EC 2.4.1.145 ; EC 2.4.1.155 ; EC 2.4.1.1.201) qui catalyse le transfert d'un résidu UDP-GlcNAc sur ledit résidu mannose terminal, ladite cellule hôte produisant une composition d'oligosaccharide ayant un résidu GlcNAc terminal.

10. Cellule hôte selon la revendication 9, ladite cellule hôte comprenant en outre une ou plusieurs activités enzymatiques de la galactosyltransférase (EC 2.4.1.38) qui catalyse le transfert d'un résidu UDP-Galactose sur ledit résidu GlcNAc terminal, ladite cellule hôte produisant une composition d'oligosaccharide ayant un résidu galactose terminal.

11. Cellule hôte selon la revendication 10, ladite cellule hôte comprenant en outre une ou plusieurs activités enzymatiques de la sialyltransférase SialyIT (EC 2.4.99.4) et SialyIT (EC 2.4.99.1) qui catalyse le transfert d'un résidu CMP-NANA sur ledit résidu galactose terminal, ladite cellule hôte produisant une composition d'oligosaccharide ayant un résidu acide sialique terminal.

12. Cellule hôte selon la revendication 9, 10 ou 11,
(a) ladite cellule hôte comprenant une ou plusieurs activités enzymatiques de la transférase UDP-GlcNAc eucaryote (EC 2.4.1.141 ou EC 2.4.1.145) et une ou plusieurs activités enzymatiques de la mannosyltransférase eucaryote (EC 2.4.1.142, EC 2.4.1.132), lesdites enzymes conférant une activité de la glycosyltransférase eucaryote à la cellule hôte ;
(b) ladite cellule hôte comprenant en outre une fusion d'une ou plusieurs des enzymes, ladite fusion comprenant au moins l'un des éléments suivants : DsbA, GlpE, GST, MBP, MstX, NusA et TrxA ;
(c) ladite cellule hôte étant un hôte oxydatif ;
(d) ladite cellule hôte comprenant une ou plusieurs des enzymes suivantes : activité enzymatique de la phosphomannomutase (ManB) (EC 5.4.2.8), l'activité enzymatique de la mannose-1-phosphate guanylyltransférase (ManC) (EC 2.7.7.13) et l'activité enzymatique de la glutamine-fructose-6-phosphate transaminase (GlmS) (EC 2.6.1.16), ManB et ManC catalysant la synthèse GDP-Mannose et GlmS catalysant la synthèse UDP-GlcNAc ;
(e) la cellule hôte comprenant en outre une atténuation de l'activité de l'enzyme GDP-D-mannose déshydratase (EC 4.2.1.47) ;
(f) la cellule hôte comprenant une activité enzymatique de la flippase ;
(g) la cellule hôte comprenant une activité enzymatique de l'oligosaccharyltransférase (EC 2.4.1.119) ; ou
(h) la cellule hôte comprenant en outre un gène codant pour une protéine d'intérêt, la cellule hôte produisant une protéine glycosylée.

13. Cellule hôte selon la revendication 12(h),
(a) la cellule hôte produisant une composition d'oligosaccharide qui est N-liée à la protéine ;
(a) la cellule hôte exprimant ladite protéine glycosylée sous la forme d'un anticorps comprenant au moins l'un des éléments suivants : partie Fv qui se lie à un antigène natif et une partie Fc qui est glycosylée au niveau d'un résidu asparagine conservé, diabody, scFv, scFv-Fc, scFv-CH, Fab et scFab ; ou
(c) la protéine glycosylée comprenant des cytokines telles que des interférons, G-CSF, facteurs de coagulation tels que facteur VIII, facteur IX et protéine C humaine, récepteur IgE soluble α-chain, IgG, fragments IgG, IgM, interleukines, urokinase, chymase et inhibiteur de trypsine d'urée, protéine liant l'IGF, facteur de croissance épidermique, facteur de croissance de l'hormone de croissance, protéine de fusion de l'annexine V, angiostatine, facteur de croissance endothéliale vasculaire-2, facteur inhibiteur du progéniteur myéloïde-1, ostéoprotégérine, α-1 antitrypsine, DNase II, protéines α-feto, AAT, rhTBP-1 (également appelé protéine de liaison TNF 1), TACI-Ig (activateur transmembranaire et modulateur du calcium et interacteur de ligand cyclophiline), FSH (hormone folliculo-stimulante), GM -CSF, glucagon, glucagon, peptides de glucagon, GLP-1 avec et sans agoniste des récepteurs IL-1 FC (glucagon-like protéine 1), sTNFr (également appelé fusion Fc de récepteur TNF soluble), CTLA4-Ig (lymphocyte T cytotoxique associé à l'antigène 4-Ig), récepteurs, hormones telles que l'hormone de croissance humaine, érythropoïétine, peptides, peptides agrafés, vaccins humains, vaccins animaux, albumine sérique et enzymes telles que ATIII, rhThrombine, glucocerebrosidase et asparaginase.

14. Cellule hôte selon la revendication 9,
(a) la cellule hôte exprimant au moins une mannosyltransférase, une N-acétylglucosaminyltransférase, des galactosyltransférases ou une sialyltransférase étant fusionnée de manière opérationnelle à MBP ;
(b) une cellule hôte oxydative étant utilisée pour exprimer l'activité d'enzyme galactosyltransférase ;
(c) la cellule hôte produisant des compositions d'oligosaccharide comprenant GlcNAc₁₋₅Man₃GlcNAc₂ et Man₃GlcNAc₂, éventuellement la composition d'oligosaccharide étant principalement GlcNAcMan₃GlcNAc₂ ou GlcNAc₂Man₃GlcNAc₂ ; ou
(d) la cellule hôte produisant des compositions d'oligosaccharide comprenant Gal₁₋₅GlcNAc₁₋₅Man₃GlcNAc₂ et Man₃GlcNAc₂, éventuellement la composition d'oligosaccharide étant principalement GalGlcNAcMan₃GlcNAc₂, GalGlcNAc₂Man₃GlcNAc₂ ou Gal₂GlcNAc₂Man₃GlcNAc₂.

15. Cellule hôte selon la revendication 11, la composition d'oligosaccharide comprenant NANA₁₋₅Gal₁₋₅GlcNAc₁₋₅Man₃GlcNAc₂, éventuellement la composition d'oligosaccharide étant principalement NANAGalGlcNAcMan₃GlcNAc₂ ou NANA₂Gal₂GlcNAc₂Man₃GlcNAc₂.


### SEQUENCE LISTING

<110> GLYCOBIA, INC.
<120> OLIGOSACCHARIDE COMPOSITIONS, GLYCOPROTEINS AND METHODS TO PRODUCE THE SAME IN PROKARYOTES
<130> GLY-103 PCT
<140> PCT/US2014/026990
   <141> 2014-03-14
<150> 61/785,586
   <151> 2013-03-14
<160> 36
<170> PatentIn version 3.5
<210> 1
   <211> 609
   <212> DNA
   <213> Artificial Sequence
<220>
   <223> Description of Artificial Sequence: Synthetic polynucleotide
<400> 1
   atgggtatca tcgaagaaaa agctctgttc gttacctgcg gtgctaccgt tccgttcccg      60
   aaactggttt cttgcgttct gtctgacgaa ttctgccagg aactgatcca gtacggtttc     120
   gttcgtctga tcatccagtt cggtcgtaac tactcttctg aattcgaaca cctggttcag     180
   gaacgtggtg gtcagcgtga atctcagaaa atcccgatcg accagttcgg ttgcggtgac     240
   accgctcgtc agtacgttct gatgaacggt aaactgaaag ttatcggttt cgacttctct     300
   accaaaatgc agtctatcat ccgtgactac tctgacctgg ttatctctca cgctggtacc     360
   ggttctatcc tggactctct gcgtctgaac aaaccgctga tcgtttgcgt taacgactct     420
   ctgatggaca accaccagca gcagatcgct gacaaattcg ttgaactggg ttacgtttgg     480
   tcttgcgctc cgaccgaaac cggtctgatc gctggtctgc gtgcttctca gaccgaaaaa     540
   ctgaaaccgt tcccggtttc tcacaacccg tctttcgaac gtctgctggt tgaaaccatc     600
   tactcttaa                                                             609
<210> 2
   <211> 202
   <212> PRT
   <213> Saccharomyces cerevisiae
<400> 2
   Met Gly Ile Ile Glu Glu Lys Ala Leu Phe Val Thr Cys Gly Ala Thr
   1               5                   10                  15
   Val Pro Phe Pro Lys Leu Val Ser Cys Val Leu Ser Asp Glu Phe Cys
               20                  25                  30
   Gln Glu Leu Ile Gln Tyr Gly Phe Val Arg Leu Ile Ile Gln Phe Gly
           35                  40                  45
   Arg Asn Tyr Ser Ser Glu Phe Glu His Leu Val Gln Glu Arg Gly Gly
       50                  55                  60
   Gln Arg Glu Ser Gln Lys Ile Pro Ile Asp Gln Phe Gly Cys Gly Asp
   65                  70                  75                  80
   Thr Ala Arg Gln Tyr Val Leu Met Asn Gly Lys Leu Lys Val Ile Gly
                   85                  90                  95
   Phe Asp Phe Ser Thr Lys Met Gln Ser Ile Ile Arg Asp Tyr Ser Asp
               100                 105                 110
   Leu Val Ile Ser His Ala Gly Thr Gly Ser Ile Leu Asp Ser Leu Arg
           115                 120                 125
   Leu Asn Lys Pro Leu Ile Val Cys Val Asn Asp Ser Leu Met Asp Asn
       130                 135                 140
   His Gln Gln Gln Ile Ala Asp Lys Phe Val Glu Leu Gly Tyr Val Trp
   145                 150                 155                 160
   Ser Cys Ala Pro Thr Glu Thr Gly Leu Ile Ala Gly Leu Arg Ala Ser
                   165                 170                 175
   Gln Thr Glu Lys Leu Lys Pro Phe Pro Val Ser His Asn Pro Ser Phe
               180                 185                 190
   Glu Arg Leu Leu Val Glu Thr Ile Tyr Ser
           195                 200
<210> 3
   <211> 714
   <212> DNA
   <213> Artificial Sequence
<220>
   <223> Description of Artificial Sequence: Synthetic polynucleotide
<400> 3
   atgaaaaccg cttacctggc ttctctggtt ctgatcgttt ctaccgctta cgttatccgt     60
   ctgatcgcta tcctgccgtt cttccacacc caggctggta ccgaaaaaga caccaaagac    120
   ggtgttaacc tgctgaaaat ccgtaaatct tctaaaaaac cgctgaaaat cttcgttttc    180
   ctgggttctg gtggtcacac cggtgaaatg atccgtctgc tggaaaacta ccaggacctg    240
   ctgctgggta aatctatcgt ttacctgggt tactctgacg aagcttctcg tcagcgtttc    300
   gctcacttca tcaaaaaatt cggtcactgc aaagttaaat actacgaatt catgaaagct    360
   cgtgaagtta aagctaccct gctgcagtct gttaaaacca tcatcggtac cctggttcag    420
   tctttcgttc acgttgttcg tatccgtttc gctatgtgcg gttctccgca cctgttcctg    480
   ctgaacggtc cgggtacctg ctgcatcatc tctttctggc tgaaaatcat ggaactgctg    540
   ctgccgctgc tgggttcttc tcacatcgtt tacgttgaat ctctggctcg tatcaacacc    600
   ccgtctctga ccggtaaaat cctgtactgg gttgttgacg aattcatcgt tcagtggcag    660
   gaactgcgtg acaactacct gccgcgttct aaatggttcg gtatcctggt ttaa          714
<210> 4
   <211> 237
   <212> PRT
   <213> Saccharomyces cerevisiae
<400> 4
   Met Lys Thr Ala Tyr Leu Ala Ser Leu Val Leu Ile Val Ser Thr Ala
   1               5                   10                  15
   Tyr Val Ile Arg Leu Ile Ala Ile Leu Pro Phe Phe His Thr Gln Ala
               20                  25                  30
   Gly Thr Glu Lys Asp Thr Lys Asp Gly Val Asn Leu Leu Lys Ile Arg
           35                  40                  45
   Lys Ser Ser Lys Lys Pro Leu Lys Ile Phe Val Phe Leu Gly Ser Gly
       50                  55                  60
   Gly His Thr Gly Glu Met Ile Arg Leu Leu Glu Asn Tyr Gln Asp Leu
   65                  70                  75                  80
   Leu Leu Gly Lys Ser Ile Val Tyr Leu Gly Tyr Ser Asp Glu Ala Ser
                   85                  90                  95
   Arg Gln Arg Phe Ala His Phe Ile Lys Lys Phe Gly His Cys Lys Val
               100                 105                 110
   Lys Tyr Tyr Glu Phe Met Lys Ala Arg Glu Val Lys Ala Thr Leu Leu
           115                 120                 125
   Gln Ser Val Lys Thr Ile Ile Gly Thr Leu Val Gln Ser Phe Val His
       130                 135                 140
   Val Val Arg Ile Arg Phe Ala Met Cys Gly Ser Pro His Leu Phe Leu
   145                 150                 155                 160
   Leu Asn Gly Pro Gly Thr Cys Cys Ile Ile Ser Phe Trp Leu Lys Ile
                   165                 170                 175
   Met Glu Leu Leu Leu Pro Leu Leu Gly Ser Ser His Ile Val Tyr Val
               180                 185                 190
   Glu Ser Leu Ala Arg Ile Asn Thr Pro Ser Leu Thr Gly Lys Ile Leu
           195                 200                 205
   Tyr Trp Val Val Asp Glu Phe Ile Val Gln Trp Gln Glu Leu Arg Asp
       210                 215                 220
   Asn Tyr Leu Pro Arg Ser Lys Trp Phe Gly Ile Leu Val
   225                 230                 235
<210> 5
   <211> 1350
   <212> DNA
   <213> Artificial Sequence
<220>
   <223> Description of Artificial Sequence: Synthetic polynucleotide
<400> 5
   atgttcctgg aaatcccgcg ttggctgctg gctctgatca tcctgtacct gtctatcccg      60
   ctggttgttt actacgttat cccgtacctg ttctacggta acaaatctac caaaaaacgt     120
   atcatcatct tcgttctggg tgacgttggt cactctccgc gtatctgcta ccacgctatc     180
   tctttctcta aactgggttg gcaggttgaa ctgtgcggtt acgttgaaga caccctgccg     240
   aaaatcatct cttctgaccc gaacatcacc gttcaccaca tgtctaacct gaaacgtaaa     300
   ggtggtggta cctctgttat cttcatggtt aaaaaagttc tgttccaggt tctgtctatc     360
   ttcaaactgc tgtgggaact gcgtggttct gactacatcc tggttcagaa cccgccgtct     420
   atcccgatcc tgccgatcgc tgttctgtac aaactgaccg gttgcaaact gatcatcgac     480
   tggcacaacc tggcttactc tatcctgcag ctgaaattca aaggtaactt ctaccacccg     540
   ctggttctga tctcttacat ggttgaaatg atcttctcta aattcgctga ctacaacctg     600
   accgttaccg aagctatgcg taaatacctg atccagtctt tccacctgaa cccgaaacgt     660
   tgcgctgttc tgtacgaccg tccggcttct cagttccagc cgctggctgg tgacatctct     720
   cgtcagaaag ctctgaccac caaagctttc atcaaaaact acatccgtga cgacttcgac     780
   accgaaaaag gtgacaaaat catcgttacc tctacctctt tcaccccgga cgaagacatc     840
   ggtatcctgc tgggtgctct gaaaatctac gaaaactctt acgttaaatt cgactcttct     900
   ctgccgaaaa tcctgtgctt catcaccggt aaaggtccgc tgaaagaaaa atacatgaaa     960
   caggttgaag aatacgactg gaaacgttgc cagatcgaat tcgtttggct gtctgctgaa    1020
   gactacccga aactgctgca gctgtgcgac tacggtgttt ctctgcacac ctcttcttct    1080
   ggtctggacc tgccgatgaa aatcctggac atgttcggtt ctggtctgcc ggttatcgct    1140
   atgaactacc cggttctgga cgaactggtt cagcacaacg ttaacggtct gaaattcgtt    1200
   gaccgtcgtg aactgcacga atctctgatc ttcgctatga aagacgctga cctgtaccag    1260
   aaactgaaaa aaaacgttac ccaggaagct gaaaaccgtt ggcagtctaa ctgggaacgt    1320
   accatgcgtg acctgaaact gatccactaa                                     1350
<210> 6
   <211> 449
   <212> PRT
   <213> Saccharomyces cerevisiae
<400> 6
   Met Phe Leu Glu Ile Pro Arg Trp Leu Leu Ala Leu Ile Ile Leu Tyr
   1               5                   10                  15
   Leu Ser Ile Pro Leu Val Val Tyr Tyr Val Ile Pro Tyr Leu Phe Tyr
               20                  25                  30
   Gly Asn Lys Ser Thr Lys Lys Arg Ile Ile Ile Phe Val Leu Gly Asp
           35                  40                  45
   Val Gly His Ser Pro Arg Ile Cys Tyr His Ala Ile Ser Phe Ser Lys
       50                  55                  60
   Leu Gly Trp Gln Val Glu Leu Cys Gly Tyr Val Glu Asp Thr Leu Pro
   65                  70                  75                  80
   Lys Ile Ile Ser Ser Asp Pro Asn Ile Thr Val His His Met Ser Asn
                   85                  90                  95
   Leu Lys Arg Lys Gly Gly Gly Thr Ser Val Ile Phe Met Val Lys Lys
               100                 105                 110
   Val Leu Phe Gln Val Leu Ser Ile Phe Lys Leu Leu Trp Glu Leu Arg
           115                 120                 125
   Gly Ser Asp Tyr Ile Leu Val Gln Asn Pro Pro Ser Ile Pro Ile Leu
       130                 135                 140
   Pro Ile Ala Val Leu Tyr Lys Leu Thr Gly Cys Lys Leu Ile Ile Asp
   145                 150                 155                 160
   Trp His Asn Leu Ala Tyr Ser Ile Leu Gln Leu Lys Phe Lys Gly Asn
   165 170 175
   Phe Tyr His Pro Leu Val Leu Ile Ser Tyr Met Val Glu Met Ile Phe
   180 185 190
   Ser Lys Phe Ala Asp Tyr Asn Leu Thr Val Thr Glu Ala Met Arg Lys
   195 200 205
   Tyr Leu Ile Gln Ser Phe His Leu Asn Pro Lys Arg Cys Ala Val Leu
   210 215 220
   Tyr Asp Arg Pro Ala Ser Gln Phe Gln Pro Leu Ala Gly Asp Ile Ser
   225 230 235 240
   Arg Gln Lys Ala Leu Thr Thr Lys Ala Phe Ile Lys Asn Tyr Ile Arg
   245 250 255
   Asp Asp Phe Asp Thr Glu Lys Gly Asp Lys Ile Ile Val Thr Ser Thr
   260 265 270
   Ser Phe Thr Pro Asp Glu Asp Ile Gly Ile Leu Leu Gly Ala Leu Lys
   275 280 285
   Ile Tyr Glu Asn Ser Tyr Val Lys Phe Asp Ser Ser Leu Pro Lys Ile
   290 295 300
   Leu Cys Phe Ile Thr Gly Lys Gly Pro Leu Lys Glu Lys Tyr Met Lys
   305 310 315 320
   Gln Val Glu Glu Tyr Asp Trp Lys Arg Cys Gln Ile Glu Phe Val Trp
   325 330 335
   Leu Ser Ala Glu Asp Tyr Pro Lys Leu Leu Gln Leu Cys Asp Tyr Gly
   340 345 350
   Val Ser Leu His Thr Ser Ser Ser Gly Leu Asp Leu Pro Met Lys Ile
   355 360 365
   Leu Asp Met Phe Gly Ser Gly Leu Pro Val Ile Ala Met Asn Tyr Pro
   370 375 380
   Val Leu Asp Glu Leu Val Gln His Asn Val Asn Gly Leu Lys Phe Val
   385 390 395 400
   Asp Arg Arg Glu Leu His Glu Ser Leu Ile Phe Ala Met Lys Asp Ala
   405 410 415
   Asp Leu Tyr Gln Lys Leu Lys Lys Asn Val Thr Gln Glu Ala Glu Asn
               420                 425                 430
   Arg Trp Gln Ser Asn Trp Glu Arg Thr Met Arg Asp Leu Lys Leu Ile
           435                 440                 445
   His
<210> 7
   <211> 1512
   <212> DNA
   <213> Artificial Sequence
<220>
   <223> Description of Artificial Sequence: Synthetic polynucleotide
<400> 7
   atgatcgaaa aagacaaacg taccatcgct ttcatccacc cggacctggg tatcggtggt 60
   gctgaacgtc tggttgttga cgctgctctg ggtctgcagc agcagggtca ctctgttatc 120
   atctacacct ctcactgcga caaatctcac tgcttcgaag aagttaaaaa cggtcagctg 180
   aaagttgaag tttacggtga cttcctgccg accaacttcc tgggtcgttt cttcatcgtt 240
   ttcgctacca tccgtcagct gtacctggtt atccagctga tcctgcagaa aaaagttaac 300
   gcttaccagc tgatcatcat cgaccagctg tctacctgca tcccgctgct gcacatcttc 360
   tcttctgcta ccctgatgtt ctactgccac ttcccggacc agctgctggc tcagcgtgct 420
   ggtctgctga aaaaaatcta ccgtctgccg ttcgacctga tcgaacagtt ctctgtttct 480
   gctgctgaca ccgttgttgt taactctaac ttcaccaaaa acaccttcca ccagaccttc 540
   aaatacctgt ctaacgaccc ggacgttatc tacccgtgcg ttgacctgtc taccatcgaa 600
   atcgaagaca tcgacaaaaa attcttcaaa accgttttca acgaaggtga ccgtttctac 660
   ctgtctatca accgtttcga aaaaaaaaaa gacgttgctc tggctatcaa agctttcgct 720
   ctgtctgaag accagatcaa cgacaacgtt aaactggtta tctgcggtgg ttacgacgaa 780
   cgtgttgctg aaaacgttga atacctgaaa gaactgcagt ctctggctga cgaatacgaa 840
   ctgtctcaca ccaccatcta ctaccaggaa atcaaacgtg tttctgacct ggaatctttc 900
   aaaaccaaca actctaaaat catcttcctg acctctatct cttcttctct gaaagaactg 960
   ctgctggaac gtaccgaaat gctgctgtac accccggctt acgaacactt cggtatcgtt 1020
   ccgctggaag ctatgaaact gggtaaaccg gttctggctg ttaacaacgg tggtccgctg 1080
   gaaaccatca aatcttacgt tgctggtgaa aacgaatctt ctgctaccgg ttggctgaaa 1140
   ccggctgttc cgatccagtg ggctaccgct atcgacgaat ctcgtaaaat cctgcagaac 1200
   ggttctgtta acttcgaacg taacggtccg ctgcgtgtta aaaaatactt ctctcgtgaa 1260
   gctatgaccc agtctttcga agaaaacgtt gaaaaagtta tctggaaaga aaaaaaatac 1320
   tacccgtggg aaatcttcgg tatctctttc tctaacttca tcctgcacat ggctttcatc 1380
   aaaatcctgc cgaacaaccc gtggccgttc ctgttcatgg ctaccttcat ggttctgtac 1440
   ttcaaaaact acctgtgggg tatctactgg gctttcgttt tcgctctgtc ttacccgtac 1500
   gaagaaatct aa 1512
<210> 8
   <211> 503
   <212> PRT
   <213> Saccharomyces cerevisiae
<400> 8
   Met Ile Glu Lys Asp Lys Arg Thr Ile Ala Phe Ile His Pro Asp Leu
   1               5                   10                  15
   Gly Ile Gly Gly Ala Glu Arg Leu Val Val Asp Ala Ala Leu Gly Leu
               20                  25                  30
   Gln Gln Gln Gly His Ser Val Ile Ile Tyr Thr Ser His Cys Asp Lys
           35                  40                  45
   Ser His Cys Phe Glu Glu Val Lys Asn Gly Gln Leu Lys Val Glu Val
       50                  55                  60
   Tyr Gly Asp Phe Leu Pro Thr Asn Phe Leu Gly Arg Phe Phe Ile Val
   65                  70                  75                  80
   Phe Ala Thr Ile Arg Gln Leu Tyr Leu Val Ile Gln Leu Ile Leu Gln
                   85                  90                  95
   Lys Lys Val Asn Ala Tyr Gln Leu Ile Ile Ile Asp Gln Leu Ser Thr
               100                 105                 110
   Cys Ile Pro Leu Leu His Ile Phe Ser Ser Ala Thr Leu Met Phe Tyr
           115                 120                 125
   Cys His Phe Pro Asp Gln Leu Leu Ala Gln Arg Ala Gly Leu Leu Lys
       130                 135                 140
   Lys Ile Tyr Arg Leu Pro Phe Asp Leu Ile Glu Gln Phe Ser Val Ser
   145                 150                 155                 160
   Ala Ala Asp Thr Val Val Val Asn Ser Asn Phe Thr Lys Asn Thr Phe
                   165                 170                 175
   His Gln Thr Phe Lys Tyr Leu Ser Asn Asp Pro Asp Val Ile Tyr Pro
               180                 185                 190
   Cys Val Asp Leu Ser Thr Ile Glu Ile Glu Asp Ile Asp Lys Lys Phe
           195                 200                 205
   Phe Lys Thr Val Phe Asn Glu Gly Asp Arg Phe Tyr Leu Ser Ile Asn
       210                 215                 220
   Arg Phe Glu Lys Lys Lys Asp Val Ala Leu Ala Ile Lys Ala Phe Ala
   225                 230                 235                 240
   Leu Ser Glu Asp Gln Ile Asn Asp Asn Val Lys Leu Val Ile Cys Gly
                   245                 250                 255
   Gly Tyr Asp Glu Arg Val Ala Glu Asn Val Glu Tyr Leu Lys Glu Leu
               260                 265                 270
   Gln Ser Leu Ala Asp Glu Tyr Glu Leu Ser His Thr Thr Ile Tyr Tyr
           275                 280                 285
   Gln Glu Ile Lys Arg Val Ser Asp Leu Glu Ser Phe Lys Thr Asn Asn
       290                 295                 300
   Ser Lys Ile Ile Phe Leu Thr Ser Ile Ser Ser Ser Leu Lys Glu Leu
   305                 310                 315                 320
   Leu Leu Glu Arg Thr Glu Met Leu Leu Tyr Thr Pro Ala Tyr Glu His
                   325                 330                 335
   Phe Gly Ile Val Pro Leu Glu Ala Met Lys Leu Gly Lys Pro Val Leu
               340                 345                 350
   Ala Val Asn Asn Gly Gly Pro Leu Glu Thr Ile Lys Ser Tyr Val Ala
           355                 360                 365
   Gly Glu Asn Glu Ser Ser Ala Thr Gly Trp Leu Lys Pro Ala Val Pro
       370                 375                 380
   Ile Gln Trp Ala Thr Ala Ile Asp Glu Ser Arg Lys Ile Leu Gln Asn
   385                 390                 395                 400
   Gly Ser Val Asn Phe Glu Arg Asn Gly Pro Leu Arg Val Lys Lys Tyr
                   405                 410                 415
   Phe Ser Arg Glu Ala Met Thr Gln Ser Phe Glu Glu Asn Val Glu Lys
               420                 425                 430
   Val Ile Trp Lys Glu Lys Lys Tyr Tyr Pro Trp Glu Ile Phe Gly Ile
   435 440 445
   Ser Phe Ser Asn Phe Ile Leu His Met Ala Phe Ile Lys Ile Leu Pro
   450 455 460
   Asn Asn Pro Trp Pro Phe Leu Phe Met Ala Thr Phe Met Val Leu Tyr
   465 470 475 480
   Phe Lys Asn Tyr Leu Trp Gly Ile Tyr Trp Ala Phe Val Phe Ala Leu
   485 490 495
   Ser Tyr Pro Tyr Glu Glu Ile
   500
<210> 9
   <211> 1647
   <212> DNA
   <213> Saccharomyces cerevisiae
<400> 9
   atgggcagtg cttggacaaa ctacaatttt gaagaggtta agtctcattt tgggttcaaa 60
   aaatatgttg tatcatcttt agtactagtg tatggactaa ttaaggttct cacgtggatc 120
   ttccgtcaat gggtgtattc cagcttgaat ccgttctcca aaaaatcttc attactgaac 180
   agagcagttg cctcctgtgg tgagaagaat gtgaaagttt ttggtttttt tcatccgtat 240
   tgtaatgctg gtggtggtgg ggaaaaagtg ctctggaaag ctgtagatat cactttgaga 300
   aaagatgcta agaacgttat tgtcatttat tcaggggatt ttgtgaatgg agagaatgtt 360
   actccggaga atattctaaa taatgtgaaa gcgaagttcg attacgactt ggattcggat 420
   agaatatttt tcatttcatt gaagctaaga tacttggtgg attcttcaac atggaagcat 480
   ttcacgttga ttggacaagc aattggatca atgattctcg catttgaatc cattattcag 540
   tgtccacctg atatatggat tgatacaatg gggtaccctt tcagctatcc tattattgct 600
   aggtttttga ggagaattcc tatcgtcaca tatacgcatt atccgataat gtcaaaagac 660
   atgttaaata agctgttcaa aatgcccaag aagggtatca aagtttacgg taaaatatta 720
   tactggaaag tttttatgtt aatttatcaa tccattggtt ctaaaattga tattgtaatc 780
   acaaactcaa catggacaaa taaccacata aagcaaattt ggcaatccaa tacgtgtaaa 840
   attatatatc ctccatgctc tactgagaaa ttagtagatt ggaagcaaaa gtttggtact 900
   gcaaagggtg agagattaaa tcaagcaatt gtgttggcac aatttcgtcc tgagaaacgt 960
   cataagttaa tcattgagtc ctttgcaact ttcttgaaaa atttaccgga ttctgtatcg 1020
   ccaattaaat tgataatggc ggggtccact agatccaagc aagatgaaaa ttatgttaaa 1080
   agtttacaag actggtcaga aaatgtatta aaaattccta aacatttgat atcattcgaa 1140
   aaaaatctgc ccttcgataa gattgaaata ttactaaaca aatctacttt cggtgttaat 1200
   gccatgtgga atgagcactt tggaattgca gttgtagagt atatggcttc cggtttgatc 1260
   cccatagttc atgcctcggc gggcccattg ttagatatag ttactccatg ggatgccaac 1320
   gggaatatcg gaaaagctcc accacaatgg gagttacaaa agaaatattt tgcaaaactc 1380
   gaagatgatg gtgaaactac tggatttttc tttaaagagc cgagtgatcc tgattataac 1440
   acaaccaaag atcctctgag ataccctaat ttgtccgacc ttttcttaca aattacgaaa 1500
   ctggactatg actgcctaag ggtgatgggc gcaagaaacc agcagtattc attgtataaa 1560
   ttctctgatt tgaagtttga taaagattgg gaaaactttg tactgaatcc tatttgtaaa 1620
   ttattagaag aggaggaaag gggctga 1647
<210> 10
   <211> 548
   <212> PRT
   <213> Saccharomyces cerevisiae
<400> 10
   Met Gly Ser Ala Trp Thr Asn Tyr Asn Phe Glu Glu Val Lys Ser His
   1               5                   10                  15
   Phe Gly Phe Lys Lys Tyr Val Val Ser Ser Leu Val Leu Val Tyr Gly
               20                  25                  30
   Leu Ile Lys Val Leu Thr Trp Ile Phe Arg Gln Trp Val Tyr Ser Ser
           35                  40                  45
   Leu Asn Pro Phe Ser Lys Lys Ser Ser Leu Leu Asn Arg Ala Val Ala
       50                  55                  60
   Ser Cys Gly Glu Lys Asn Val Lys Val Phe Gly Phe Phe His Pro Tyr
   65                  70                  75                  80
   Cys Asn Ala Gly Gly Gly Gly Glu Lys Val Leu Trp Lys Ala Val Asp
                   85                  90                  95
   Ile Thr Leu Arg Lys Asp Ala Lys Asn Val Ile Val Ile Tyr Ser Gly
               100                 105                 110
   Asp Phe Val Asn Gly Glu Asn Val Thr Pro Glu Asn Ile Leu Asn Asn
           115                 120                 125
   Val Lys Ala Lys Phe Asp Tyr Asp Leu Asp Ser Asp Arg Ile Phe Phe
       130                 135                 140
   Ile Ser Leu Lys Leu Arg Tyr Leu Val Asp Ser Ser Thr Trp Lys His
   145 150 155 160
   Phe Thr Leu Ile Gly Gln Ala Ile Gly Ser Met Ile Leu Ala Phe Glu
   165 170 175
   Ser Ile Ile Gln Cys Pro Pro Asp Ile Trp Ile Asp Thr Met Gly Tyr
   180 185 190
   Pro Phe Ser Tyr Pro Ile Ile Ala Arg Phe Leu Arg Arg Ile Pro Ile
   195 200 205
   Val Thr Tyr Thr His Tyr Pro Ile Met Ser Lys Asp Met Leu Asn Lys
   210 215 220
   Leu Phe Lys Met Pro Lys Lys Gly Ile Lys Val Tyr Gly Lys Ile Leu
   225 230 235 240
   Tyr Trp Lys Val Phe Met Leu Ile Tyr Gln Ser Ile Gly Ser Lys Ile
   245 250 255
   Asp Ile Val Ile Thr Asn Ser Thr Trp Thr Asn Asn His Ile Lys Gln
   260 265 270
   Ile Trp Gln Ser Asn Thr Cys Lys Ile Ile Tyr Pro Pro Cys Ser Thr
   275 280 285
   Glu Lys Leu Val Asp Trp Lys Gln Lys Phe Gly Thr Ala Lys Gly Glu
   290 295 300
   Arg Leu Asn Gln Ala Ile Val Leu Ala Gln Phe Arg Pro Glu Lys Arg
   305 310 315 320
   His Lys Leu Ile Ile Glu Ser Phe Ala Thr Phe Leu Lys Asn Leu Pro
   325 330 335
   Asp Ser Val Ser Pro Ile Lys Leu Ile Met Ala Gly Ser Thr Arg Ser
   340 345 350
   Lys Gln Asp Glu Asn Tyr Val Lys Ser Leu Gln Asp Trp Ser Glu Asn
   355 360 365
   Val Leu Lys Ile Pro Lys His Leu Ile Ser Phe Glu Lys Asn Leu Pro
   370 375 380
   Phe Asp Lys Ile Glu Ile Leu Leu Asn Lys Ser Thr Phe Gly Val Asn
   385 390 395 400
   Ala Met Trp Asn Glu His Phe Gly Ile Ala Val Val Glu Tyr Met Ala
                   405                 410                 415
   Ser Gly Leu Ile Pro Ile Val His Ala Ser Ala Gly Pro Leu Leu Asp
               420                 425                 430
   Ile Val Thr Pro Trp Asp Ala Asn Gly Asn Ile Gly Lys Ala Pro Pro
           435                 440                 445
   Gln Trp Glu Leu Gln Lys Lys Tyr Phe Ala Lys Leu Glu Asp Asp Gly
       450                 455                 460
   Glu Thr Thr Gly Phe Phe Phe Lys Glu Pro Ser Asp Pro Asp Tyr Asn
   465                 470                 475                 480
   Thr Thr Lys Asp Pro Leu Arg Tyr Pro Asn Leu Ser Asp Leu Phe Leu
                   485                 490                 495
   Gln Ile Thr Lys Leu Asp Tyr Asp Cys Leu Arg Val Met Gly Ala Arg
               500                 505                 510
   Asn Gln Gln Tyr Ser Leu Tyr Lys Phe Ser Asp Leu Lys Phe Asp Lys
           515                 520                 525
   Asp Trp Glu Asn Phe Val Leu Asn Pro Ile Cys Lys Leu Leu Glu Glu
       530                 535                 540
   Glu Glu Arg Gly
   545
<210> 11
   <211> 1113
   <212> DNA
   <213> Escherichia coli
<400> 11
   aaaatcgaag aaggtaaact ggtaatctgg attaacggcg ataaaggcta taacggtctc      60
   gctgaagtcg gtaagaaatt cgagaaagat accggaatta aagtcaccgt tgagcatccg     120
   gataaactgg aagagaaatt cccacaggtt gcggcaactg gcgatggccc tgacattatc     180
   ttctgggcac acgaccgctt tggtggctac gctcaatctg gcctgttggc tgaaatcacc     240
   ccggacaaag cgttccagga caagctgtat ccgtttacct gggatgccgt acgttacaac     300
   ggcaagctga ttgcttaccc gatcgctgtt gaagcgttat cgctgattta taacaaagat     360
   ctgctgccga acccgccaaa aacctgggaa gagatcccgg cgctggataa agaactgaaa     420
   gcgaaaggta agagcgcgct gatgttcaac ctgcaagaac cgtacttcac ctggccgctg     480
   attgctgctg acgggggtta tgcgttcaag tatgaaaacg gcaagtacga cattaaagac     540
   gtgggcgtgg ataacgctgg cgcgaaagcg ggtctgacct tcctggttga cctgattaaa     600
   aacaaacaca tgaatgcaga caccgattac tccatcgcag aagctgcctt taataaaggc     660
   gaaacagcga tgaccatcaa cggcccgtgg gcatggtcca acatcgacac cagcaaagtg     720
   aattatggtg taacggtact gccgaccttc aagggtcaac catccaaacc gttcgttggc     780
   gtgctgagcg caggtattaa cgccgccagt ccgaacaaag agctggcgaa agagttcctc     840
   gaaaactatc tgctgactga tgaaggtctg gaagcggtta ataaagacaa accgctgggt     900
   gccgtagcgc tgaagtctta cgaggaagag ttggcgaaag atccacgtat tgccgccacc     960
   atggaaaacg cccagaaagg tgaaatcatg ccgaacatcc cgcagatgtc cgctttctgg    1020
   tatgccgtgc gtactgcggt gatcaacgcc gccagcggtc gtcagactgt cgatgaagcc    1080
   ctgaaagacg cgcagactcg tatcaccaag taa                                1113
<210> 12
   <211> 370
   <212> PRT
   <213> Escherichia coli
<400> 12
<210> 13
   <211> 330
   <212> DNA
   <213> Bacillus sp.
<400> 13
   atgttttgta cattttttga aaaacatcac cggaagtggg acatactgtt agaaaaaagc        60
   acgggtgtga tggaagctat gaaagtgacg agtgaggaaa aggaacagct gagcacagca       120
   atcgaccgaa tgaatgaagg actggacgcg tttatccagc tgtataatga atcggaaatt       180
   gatgaaccgc ttattcagct tgatgatgat acagccgagt taatgaagca ggcccgagat       240
   atgtacggcc aggaaaagct aaatgagaaa ttaaatacaa ttattaaaca gattttatcc       300
   atctcagtat ctgaagaagg agaaaaagaa                                        330
<210> 14
   <211> 110
   <212> PRT
   <213> Bacillus sp.
<400> 14
   Met Phe Cys Thr Phe Phe Glu Lys His His Arg Lys Trp Asp Ile Leu
   1               5                   10                  15
   Leu Glu Lys Ser Thr Gly Val Met Glu Ala Met Lys Val Thr Ser Glu
               20                  25                  30
   Glu Lys Glu Gln Leu Ser Thr Ala Ile Asp Arg Met Asn Glu Gly Leu
           35                  40                  45
   Asp Ala Phe Ile Gln Leu Tyr Asn Glu Ser Glu Ile Asp Glu Pro Leu
       50                  55                  60
   Ile Gln Leu Asp Asp Asp Thr Ala Glu Leu Met Lys Gln Ala Arg Asp
   65                  70                  75                  80
   Met Tyr Gly Gln Glu Lys Leu Asn Glu Lys Leu Asn Thr Ile Ile Lys
                   85                  90                  95
   Gln Ile Leu Ser Ile Ser Val Ser Glu Glu Gly Glu Lys Glu
               100                 105                 110
<210> 15
   <211> 1254
   <212> DNA
   <213> Nicotiana tabacum
<400> 15
   gcgacacagt cagaatatgc agatcgcctt gctgctgcaa ttgaagcaga aaatcattgt 60
   acaagccaga ccagattgct tattgaccag attagcctgc agcaaggaag aatagttgct 120
   cttgaagaac aaatgaagcg tcaggaccag gagtgccgac aattaagggc tcttgttcag 180
   gatcttgaaa gtaagggcat aaaaaagttg atcggaaatg tacagatgcc agtggctgct 240
   gtagttgtta tggcttgcaa tcgggctgat tacctggaaa agactattaa atccatctta 300
   aaataccaaa tatctgttgc gtcaaaatat cctcttttca tatcccagga tggatcacat 360
   cctgatgtca ggaagcttgc tttgagctat gatcagctga cgtatatgca gcacttggat 420
   tttgaacctg tgcatactga aagaccaggg gagctgattg catactacaa aattgcacgt 480
   cattacaagt gggcattgga tcagctgttt tacaagcata attttagccg tgttatcata 540
   ctagaagatg atatggaaat tgcccctgat ttttttgact tttttgaggc tggagctact 600
   cttcttgaca gagacaagtc gattatggct atttcttctt ggaatgacaa tggacaaatg 660
   cagtttgtcc aagatcctta tgctctttac cgctcagatt tttttcccgg tcttggatgg 720
   atgctttcaa aatctacttg ggacgaatta tctccaaagt ggccaaaggc ttactgggac 780
   gactggctaa gactcaaaga gaatcacaga ggtcgacaat ttattcgccc agaagtttgc 840
   agaacatata attttggtga gcatggttct agtttggggc agtttttcaa gcagtatctt 900
   gagccaatta aactaaatga tgtccaggtt gattggaagt caatggacct tagttacctt 960
   ttggaggaca attacgtgaa acactttggt gacttggtta aaaaggctaa gcccatccat 1020
   ggagctgatg ctgtcttgaa agcatttaac atagatggtg atgtgcgtat tcagtacaga 1080
   gatcaactag actttgaaaa tatcgcacgg caatttggca tttttgaaga atggaaggat 1140
   ggtgtaccac gtgcagcata taaaggaata gtagttttcc ggtaccaaac gtccagacgt 1200
   gtattccttg ttggccatga ttcgcttcaa caactcggaa ttgaagatac ttaa 1254
<210> 16
   <211> 417
   <212> PRT
   <213> Nicotiana tabacum
<400> 16
   Ala Thr Gln Ser Glu Tyr Ala Asp Arg Leu Ala Ala Ala Ile Glu Ala
   1               5                   10                  15
   Glu Asn His Cys Thr Ser Gln Thr Arg Leu Leu Ile Asp Gln Ile Ser
               20                  25                  30
   Leu Gln Gln Gly Arg Ile Val Ala Leu Glu Glu Gln Met Lys Arg Gln
           35                  40                  45
   Asp Gln Glu Cys Arg Gln Leu Arg Ala Leu Val Gln Asp Leu Glu Ser
       50                  55                  60
   Lys Gly Ile Lys Lys Leu Ile Gly Asn Val Gln Met Pro Val Ala Ala
   65                  70                  75                  80
   Val Val Val Met Ala Cys Asn Arg Ala Asp Tyr Leu Glu Lys Thr Ile
                   85                  90                  95
   Lys Ser Ile Leu Lys Tyr Gln Ile Ser Val Ala Ser Lys Tyr Pro Leu
               100                 105                 110
   Phe Ile Ser Gln Asp Gly Ser His Pro Asp Val Arg Lys Leu Ala Leu
           115                 120                 125
   Ser Tyr Asp Gln Leu Thr Tyr Met Gln His Leu Asp Phe Glu Pro Val
       130                 135                 140
   His Thr Glu Arg Pro Gly Glu Leu Ile Ala Tyr Tyr Lys Ile Ala Arg
   145                 150                 155                 160
   His Tyr Lys Trp Ala Leu Asp Gln Leu Phe Tyr Lys His Asn Phe Ser
                   165                 170                 175
   Arg Val Ile Ile Leu Glu Asp Asp Met Glu Ile Ala Pro Asp Phe Phe
               180                 185                 190
   Asp Phe Phe Glu Ala Gly Ala Thr Leu Leu Asp Arg Asp Lys Ser Ile
           195                 200                 205
   Met Ala Ile Ser Ser Trp Asn Asp Asn Gly Gln Met Gln Phe Val Gln
       210                 215                 220
   Asp Pro Tyr Ala Leu Tyr Arg Ser Asp Phe Phe Pro Gly Leu Gly Trp
   225                 230                 235                 240
   Met Leu Ser Lys Ser Thr Trp Asp Glu Leu Ser Pro Lys Trp Pro Lys
                   245                 250                 255
   Ala Tyr Trp Asp Asp Trp Leu Arg Leu Lys Glu Asn His Arg Gly Arg
               260                 265                 270
   Gln Phe Ile Arg Pro Glu Val Cys Arg Thr Tyr Asn Phe Gly Glu His
           275                 280                 285
   Gly Ser Ser Leu Gly Gln Phe Phe Lys Gln Tyr Leu Glu Pro Ile Lys
       290                 295                 300
   Leu Asn Asp Val Gln Val Asp Trp Lys Ser Met Asp Leu Ser Tyr Leu
   305                 310                 315                 320
   Leu Glu Asp Asn Tyr Val Lys His Phe Gly Asp Leu Val Lys Lys Ala
                   325                 330                 335
   Lys Pro Ile His Gly Ala Asp Ala Val Leu Lys Ala Phe Asn Ile Asp
               340                 345                 350
   Gly Asp Val Arg Ile Gln Tyr Arg Asp Gln Leu Asp Phe Glu Asn Ile
           355                 360                 365
   Ala Arg Gln Phe Gly Ile Phe Glu Glu Trp Lys Asp Gly Val Pro Arg
       370                 375                 380
   Ala Ala Tyr Lys Gly Ile Val Val Phe Arg Tyr Gln Thr Ser Arg Arg
   385                 390                 395                 400
   Val Phe Leu Val Gly His Asp Ser Leu Gln Gln Leu Gly Ile Glu Asp
                   405                 410                 415
   Thr
<210> 17
   <211> 1344
   <212> DNA
   <213> Homo sapiens
<400> 17
   atgcgctttc gtatctataa acgtaaagtg ctgatcctga cactggttgt tgccgcttgt      60
   ggttttgttc tgtggagcag taatggtcgt cagcgtaaaa atgaagccct ggcacctcct     120
   ctgctggatg ctgaaccggc acgtggtgct ggcggtcgtg gtggtgatca tccgtctgtt     180
   gccgttggta ttcgtcgtgt gagcaatgtt tcggctgcct ctctggtccc ggctgttcct     240
   caacctgaag ctgataacct gaccctgcgc tatcgctctc tggtgtatca actgaacttc     300
   gatcaaactc tgcgtaacgt ggataaagca ggcacatggg ctcctcgtga actggtactg     360
   gtagtccagg tccataatcg tccggaatat ctgcgtctgc tgctggattc tctgcgcaaa     420
   gctcaaggca tcgataatgt cctggtcatc ttctctcatg atttctggag cacggagatt     480
   aaccagctga ttgccggcgt gaatttttgt cctgtgctgc aggtgttttt tccgttttct     540
   atccaactgt atccgaacga atttccgggt tctgatcctc gtgattgtcc tcgtgatctg     600
   cctaaaaatg ccgctctgaa actgggctgt attaatgccg agtatcctga ttcttttggc     660
   cactatcgtg aggcgaaatt ttctcagacc aaacatcatt ggtggtggaa actgcatttc     720
   gtgtgggaac gtgtgaaaat cctgcgcgac tatgctggcc tgattctgtt tctggaagaa     780
   gatcactatc tggctccgga cttttatcat gtgttcaaaa aaatgtggaa actgaaacag     840
   caggaatgtc cagaatgtga tgtgctgtca ctgggcacct atagtgcttc tcgctccttc     900
   tatggtatgg ccgacaaagt ggacgttaaa acatggaaat ccaccgagca caacatgggt     960
   ctggcactga ctcgtaatgc ctatcaaaaa ctgattgagt gtaccgacac cttttgtacg     1020
   tatgatgact ataactggga ctggaccctg caatatctga ccgtgagctg tctgccaaaa     1080
   ttttggaaag ttctggtgcc tcagattcct cgtatctttc atgctggcga ctgtggtatg     1140
   caccataaaa aaacttgccg tccgtcaaca caatctgctc agatcgagtc gctgctgaat     1200
   aataacaaac agtatatgtt cccggagact ctgacaattt ctgaaaaatt caccgtggtc     1260
   gccatttctc cgcctcgtaa aaatggaggt tggggcgata tccgtgacca tgaactgtgt     1320
   aaaagctatc gtcgtctgca gtga                                            1344
<210> 18
   <211> 447
   <212> PRT
   <213> Homo sapiens
<400> 18
   Met Arg Phe Arg Ile Tyr Lys Arg Lys Val Leu Ile Leu Thr Leu Val
   1               5                   10                  15
   Val Ala Ala Cys Gly Phe Val Leu Trp Ser Ser Asn Gly Arg Gln Arg
               20                  25                  30
   Lys Asn Glu Ala Leu Ala Pro Pro Leu Leu Asp Ala Glu Pro Ala Arg
           35                  40                  45
   Gly Ala Gly Gly Arg Gly Gly Asp His Pro Ser Val Ala Val Gly Ile
       50                  55                  60
   Arg Arg Val Ser Asn Val Ser Ala Ala Ser Leu Val Pro Ala Val Pro
   65                  70                  75                  80
   Gln Pro Glu Ala Asp Asn Leu Thr Leu Arg Tyr Arg Ser Leu Val Tyr
                   85                  90                  95
   Gln Leu Asn Phe Asp Gln Thr Leu Arg Asn Val Asp Lys Ala Gly Thr
               100                 105                 110
   Trp Ala Pro Arg Glu Leu Val Leu Val Val Gln Val His Asn Arg Pro
           115                 120                 125
   Glu Tyr Leu Arg Leu Leu Leu Asp Ser Leu Arg Lys Ala Gln Gly Ile
       130                 135                 140
   Asp Asn Val Leu Val Ile Phe Ser His Asp Phe Trp Ser Thr Glu Ile
   145                 150                 155                 160
   Asn Gln Leu Ile Ala Gly Val Asn Phe Cys Pro Val Leu Gln Val Phe
                   165                 170                 175
   Phe Pro Phe Ser Ile Gln Leu Tyr Pro Asn Glu Phe Pro Gly Ser Asp
   180 185 190
   Pro Arg Asp Cys Pro Arg Asp Leu Pro Lys Asn Ala Ala Leu Lys Leu
   195 200 205
   Gly Cys Ile Asn Ala Glu Tyr Pro Asp Ser Phe Gly His Tyr Arg Glu
   210 215 220
   Ala Lys Phe Ser Gln Thr Lys His His Trp Trp Trp Lys Leu His Phe
   225 230 235 240
   Val Trp Glu Arg Val Lys Ile Leu Arg Asp Tyr Ala Gly Leu Ile Leu
   245 250 255
   Phe Leu Glu Glu Asp His Tyr Leu Ala Pro Asp Phe Tyr His Val Phe
   260 265 270
   Lys Lys Met Trp Lys Leu Lys Gln Gln Glu Cys Pro Glu Cys Asp Val
   275 280 285
   Leu Ser Leu Gly Thr Tyr Ser Ala Ser Arg Ser Phe Tyr Gly Met Ala
   290 295 300
   Asp Lys Val Asp Val Lys Thr Trp Lys Ser Thr Glu His Asn Met Gly
   305 310 315 320
   Leu Ala Leu Thr Arg Asn Ala Tyr Gln Lys Leu Ile Glu Cys Thr Asp
   325 330 335
   Thr Phe Cys Thr Tyr Asp Asp Tyr Asn Trp Asp Trp Thr Leu Gln Tyr
   340 345 350
   Leu Thr Val Ser Cys Leu Pro Lys Phe Trp Lys Val Leu Val Pro Gln
   355 360 365
   Ile Pro Arg Ile Phe His Ala Gly Asp Cys Gly Met His His Lys Lys
   370 375 380
   Thr Cys Arg Pro Ser Thr Gln Ser Ala Gln Ile Glu Ser Leu Leu Asn
   385 390 395 400
   Asn Asn Lys Gln Tyr Met Phe Pro Glu Thr Leu Thr Ile Ser Glu Lys
   405 410 415
   Phe Thr Val Val Ala Ile Ser Pro Pro Arg Lys Asn Gly Gly Trp Gly
   420 425 430
   Asp Ile Arg Asp His Glu Leu Cys Lys Ser Tyr Arg Arg Leu Gln
   435 440 445
<210> 19
   <211> 1329
   <212> DNA
   <213> Bos taurus
<400> 19
   ttgaaagaac tgacgtccaa aaagagcttg caagtcccgt ccatctacta tcacttgccg      60
   cacttgctgc aaaacgaggg ctctttgcaa ccggcagttc agatcggcaa tggtcgcacc     120
   ggcgtgagca ttgttatggg tatcccgacc gtgaaacgtg aagtgaaaag ctatctgatt     180
   gaaacgctgc atagcctgat cgataacctg tacccggaag aaaaactgga ctgcgtgatt     240
   gtcgttttca ttggtgaaac cgacacggat tatgtgaatg gcgttgttgc caatctggaa     300
   aaagagttca gcaaagagat cagcagcggc ctggttgaga tcatttctcc gccggagagc     360
   tattacccgg atctgacgaa cctgaaagaa accttcggtg atagcaaaga gcgtgtccgt     420
   tggcgcacta agcagaacct ggactattgt tttctgatga tgtacgcgca agaaaagggt     480
   acgtattaca tccaactgga ggacgacatt attgtgaagc aaaactactt caacaccatt     540
   aagaacttcg cgctgcagct gagcagcgaa gagtggatga ttctggagtt cagccagctg     600
   ggcttcattg gcaagatgtt tcaggcaccg gacttgaccc tgatcgtgga gtttatcttt     660
   atgttctaca aagagaaacc gatcgattgg ctgctggatc atatcctgtg ggtcaaggtc     720
   tgcaatccgg aaaaagatgc caagcattgt gaccgccaga aagcgaatct gcgtattcgt     780
   tttcgtccta gcctgttcca acacgtgggt ctgcacagct ctctgaccgg taagatccaa     840
   aagctgaccg acaaagatta catgaaaccg ctgctgctga agatccatgt caacccgcca     900
   gcagaggtga gcacctcgct gaaagtctac cagggtcaca ctctggagaa aacctatatg     960
   ggcgaggact tcttttgggc gattacgcct gttgcgggtg actatatctt gtttaagttt    1020
   gacaagccgg ttaatgtaga gagctacttg tttcatagcg gtaaccagga tcacccaggt    1080
   gacattctgc tgaacaccac cgttgaagtg ttgccgctga aaagcgaagg tctggatatt    1140
   tcgaaagaaa cgaaggataa gcgtctggag gatggttact tccgtatcgg caagttcgag    1200
   aatggcgtgg ctgaaggtat ggtcgacccg agcctgaacc cgatttccgc atttcgcctg    1260
   tccgtcatcc agaatagcgc ggtttgggct atcctgaatg agattcacat caaaaaggtt    1320
   acgaattaa                                                            1329
<210> 20
   <211> 443
   <212> PRT
   <213> Bos taurus
<400> 20
   Ile Leu Lys Glu Leu Thr Ser Lys Lys Ser Leu Gln Val Pro Ser Ile
   1               5                   10                  15
   Tyr Tyr His Leu Pro His Leu Leu Gln Asn Glu Gly Ser Leu Gln Pro
               20                  25                  30
   Ala Val Gln Ile Gly Asn Gly Arg Thr Gly Val Ser Ile Val Met Gly
           35                  40                  45
   Ile Pro Thr Val Lys Arg Glu Val Lys Ser Tyr Leu Ile Glu Thr Leu
       50                  55                  60
   His Ser Leu Ile Asp Asn Leu Tyr Pro Glu Glu Lys Leu Asp Cys Val
   65                  70                  75                  80
   Ile Val Val Phe Ile Gly Glu Thr Asp Thr Asp Tyr Val Asn Gly Val
                   85                  90                  95
   Val Ala Asn Leu Glu Lys Glu Phe Ser Lys Glu Ile Ser Ser Gly Leu
               100                 105                 110
   Val Glu Ile Ile Ser Pro Pro Glu Ser Tyr Tyr Pro Asp Leu Thr Asn
           115                 120                 125
   Leu Lys Glu Thr Phe Gly Asp Ser Lys Glu Arg Val Arg Trp Arg Thr
       130                 135                 140
   Lys Gln Asn Leu Asp Tyr Cys Phe Leu Met Met Tyr Ala Gln Glu Lys
   145                 150                 155                 160
   Gly Thr Tyr Tyr Ile Gln Leu Glu Asp Asp Ile Ile Val Lys Gln Asn
                   165                 170                 175
   Tyr Phe Asn Thr Ile Lys Asn Phe Ala Leu Gln Leu Ser Ser Glu Glu
               180                 185                 190
   Trp Met Ile Leu Glu Phe Ser Gln Leu Gly Phe Ile Gly Lys Met Phe
           195                 200                 205
   Gln Ala Pro Asp Leu Thr Leu Ile Val Glu Phe Ile Phe Met Phe Tyr
       210                 215                 220
   Lys Glu Lys Pro Ile Asp Trp Leu Leu Asp His Ile Leu Trp Val Lys
   225                 230                 235                 240
   Val Cys Asn Pro Glu Lys Asp Ala Lys His Cys Asp Arg Gln Lys Ala
                   245                 250                 255
   Asn Leu Arg Ile Arg Phe Arg Pro Ser Leu Phe Gln His Val Gly Leu
               260                 265                 270
   His Ser Ser Leu Thr Gly Lys Ile Gln Lys Leu Thr Asp Lys Asp Tyr
           275                 280                 285
   Met Lys Pro Leu Leu Leu Lys Ile His Val Asn Pro Pro Ala Glu Val
       290                 295                 300
   Ser Thr Ser Leu Lys Val Tyr Gln Gly His Thr Leu Glu Lys Thr Tyr
   305                 310                 315                 320
   Met Gly Glu Asp Phe Phe Trp Ala Ile Thr Pro Val Ala Gly Asp Tyr
                   325                 330                 335
   Ile Leu Phe Lys Phe Asp Lys Pro Val Asn Val Glu Ser Tyr Leu Phe
               340                 345                 350
   His Ser Gly Asn Gln Asp His Pro Gly Asp Ile Leu Leu Asn Thr Thr
           355                 360                 365
   Val Glu Val Leu Pro Leu Lys Ser Glu Gly Leu Asp Ile Ser Lys Glu
       370                 375                 380
   Thr Lys Asp Lys Arg Leu Glu Asp Gly Tyr Phe Arg Ile Gly Lys Phe
   385                 390                 395                 400
   Glu Asn Gly Val Ala Glu Gly Met Val Asp Pro Ser Leu Asn Pro Ile
                   405                 410                 415
   Ser Ala Phe Arg Leu Ser Val Ile Gln Asn Ser Ala Val Trp Ala Ile
               420                 425                 430
   Leu Asn Glu Ile His Ile Lys Lys Val Thr Asn
           435                 440
<210> 21
   <211> 822
   <212> DNA
   <213> Helicobacter pylori
<400> 21
   atgcgtgtct ttattatcag tctgaaccag aaagtgtgtg acaaattcgg cctggtgttt 60
   cgtgatacca caaccctgct gaataacatc aatgccaccc gccacaaagc acagattttt 120
   gacgccgtct atagcaaaac gttcgaaggt gggctgcatc cactggtgaa aaaacatctg 180
   cacccgtatt tcattaccca gaacatcaaa gacatgggca ttaccaccaa cctgattagc 240
   ggtgtatcca aattctatta tgctctgaaa tatcacgcca aattcatgag cctgggcgaa 300
   ctgggctgtt atgccagcca ttatagcctg tgggagaaat gtattgagct gaacgaggcc 360
   atttgtatcc tggaagatga cattacgctg aaagaagatt tcaaagaggg cctggatttc 420
   ctggaaaaac acattcagga gctgggctat gttcgtctga tgcatctgct gtatgatgcc 480
   tccgttaaaa gcgaacctct gtcccataaa aaccacgaga ttcaagagcg tgtcgggatc 540
   attaaagctt atagtcacgg tgttggcact cagggatatg tgattactcc gaaaattgcc 600
   aaagtgttca aaaaatgctc ccgtaaatgg gttgttccgg tggatacgat catggatgcc 660
   acgtttattc atggggtgaa aaacctggta ctgcaaccgt ttgtgattgc cgatgatgag 720
   caaatttcca cgattgtccg taaagaggag ccgtattccc ctaaaattgc cctgatgcgc 780
   gaactgcact tcaaatatct gaaatattgg cagtttgtgt ga 822
<210> 22
   <211> 273
   <212> PRT
   <213> Helicobacter pylori
<400> 22
   Met Arg Val Phe Ile Ile Ser Leu Asn Gln Lys Val Cys Asp Lys Phe
   1               5                   10                  15
   Gly Leu Val Phe Arg Asp Thr Thr Thr Leu Leu Asn Asn Ile Asn Ala
               20                  25                  30
   Thr Arg His Lys Ala Gln Ile Phe Asp Ala Val Tyr Ser Lys Thr Phe
           35                  40                  45
   Glu Gly Gly Leu His Pro Leu Val Lys Lys His Leu His Pro Tyr Phe
       50                  55                  60
   Ile Thr Gln Asn Ile Lys Asp Met Gly Ile Thr Thr Asn Leu Ile Ser
   65                  70                  75                  80
   Gly Val Ser Lys Phe Tyr Tyr Ala Leu Lys Tyr His Ala Lys Phe Met
                   85                  90                  95
   Ser Leu Gly Glu Leu Gly Cys Tyr Ala Ser His Tyr Ser Leu Trp Glu
               100                 105                 110
   Lys Cys Ile Glu Leu Asn Glu Ala Ile Cys Ile Leu Glu Asp Asp Ile
           115                 120                 125
   Thr Leu Lys Glu Asp Phe Lys Glu Gly Leu Asp Phe Leu Glu Lys His
           130                 135                 140
   Ile Gln Glu Leu Gly Tyr Val Arg Leu Met His Leu Leu Tyr Asp Ala
   145                 150                 155                 160
   Ser Val Lys Ser Glu Pro Leu Ser His Lys Asn His Glu Ile Gln Glu
                   165                 170                 175
   Arg Val Gly Ile Ile Lys Ala Tyr Ser His Gly Val Gly Thr Gln Gly
               180                 185                 190
   Tyr Val Ile Thr Pro Lys Ile Ala Lys Val Phe Lys Lys Cys Ser Arg
           195                 200                 205
   Lys Trp Val Val Pro Val Asp Thr Ile Met Asp Ala Thr Phe Ile His
       210                 215                 220
   Gly Val Lys Asn Leu Val Leu Gln Pro Phe Val Ile Ala Asp Asp Glu
   225                 230                 235                 240
   Gln Ile Ser Thr Ile Val Arg Lys Glu Glu Pro Tyr Ser Pro Lys Ile
                   245                 250                 255
   Ala Leu Met Arg Glu Leu His Phe Lys Tyr Leu Lys Tyr Trp Gln Phe
               260                 265                 270
   Val
<210> 23
   <211> 1371
   <212> DNA
   <213> Escherichia coli
<400> 23
   atgaaaaaat taacctgctt taaagcctat gatattcgcg ggaaattagg cgaagaactg 60
   aatgaagata tcgcctggcg cattggtcgc gcctatggcg aatttctcaa accgaaaacc 120
   attgtgttag gcggtgatgt ccgcctcacc agcgaaacct taaaactggc gctggcgaaa 180
   ggtttacagg atgcgggcgt tgacgtgctg gatattggta tgtccggcac cgaagagatc 240
   tatttcgcca cgttccatct cggcgtggat ggcggcattg aagttaccgc cagccataat 300
   ccgatggatt ataacggcat gaagctggtt cgcgaggggg ctcgcccgat cagcggagat 360
   accggactgc gcgacgtcca gcgtctggct gaagccaacg actttcctcc cgtcgatgaa 420
   accaaacgcg gtcgctatca gcaaatcaac ctgcgtgacg cttacgttga tcacctgttc 480
   ggttatatca atgtcaaaaa cctcacgccg ctcaagctgg tgatcaactc cgggaacggc 540
   gcagcgggtc cggtggtgga cgccattgaa gcccgcttta aagccctcgg cgcgcccgtg 600
   gaattaatca aagtgcacaa cacgccggac ggcaatttcc ccaacggtat tcctaaccca 660
   ctactgccgg aatgccgcga cgacacccgc aatgcggtca tcaaacacgg cgcggatatg 720
   ggcattgctt ttgatggcga ttttgaccgc tgtttcctgt ttgacgaaaa agggcagttt 780
   attgagggct actacattgt cggcctgttg gcagaagcat tcctcgaaaa aaatcccggc 840
   gcgaagatca tccacgatcc acgtctctcc tggaacaccg ttgatgtggt gactgccgca 900
   ggtggcacgc cggtaatgtc gaaaaccgga cacgccttta ttaaagaacg tatgcgcaag 960
   gaagacgcca tctatggtgg cgaaatgagc gcccaccatt acttccgtga tttcgcttac 1020
   tgcgacagcg gcatgatccc gtggctgctg gtcgccgaac tggtgtgcct gaaagataaa 1080
   acgctgggcg aactggtacg cgaccggatg gcggcgtttc cggcaagcgg tgagatcaac 1140
   agcaaactgg cgcaacccgt tgaggcgatt aaccgcgtgg aacagcattt tagccgtgag 1200
   gcgctggcgg tggatcgcac cgatggcatc agcatgacct ttgccgactg gcgctttaac 1260
   ctgcgcacct ccaataccga accggtggtg cgcctgaatg tggaatcgcg cggtgatgtg 1320
   ccgctgatgg aagcgcgaac gcgaactctg ctgacgttgc tgaacgagta a 1371
<210> 24
   <211> 456
   <212> PRT
   <213> Escherichia coli
<400> 24
   Met Lys Lys Leu Thr Cys Phe Lys Ala Tyr Asp Ile Arg Gly Lys Leu
   1               5                   10                  15
   Gly Glu Glu Leu Asn Glu Asp Ile Ala Trp Arg Ile Gly Arg Ala Tyr
               20                  25                  30
   Gly Glu Phe Leu Lys Pro Lys Thr Ile Val Leu Gly Gly Asp Val Arg
           35                  40                  45
   Leu Thr Ser Glu Thr Leu Lys Leu Ala Leu Ala Lys Gly Leu Gln Asp
       50                  55                  60
   Ala Gly Val Asp Val Leu Asp Ile Gly Met Ser Gly Thr Glu Glu Ile
   65                  70                  75                  80
   Tyr Phe Ala Thr Phe His Leu Gly Val Asp Gly Gly Ile Glu Val Thr
                   85                  90                  95
   Ala Ser His Asn Pro Met Asp Tyr Asn Gly Met Lys Leu Val Arg Glu
               100                 105                 110
   Gly Ala Arg Pro Ile Ser Gly Asp Thr Gly Leu Arg Asp Val Gln Arg
           115                 120                 125
   Leu Ala Glu Ala Asn Asp Phe Pro Pro Val Asp Glu Thr Lys Arg Gly
       130                 135                 140
   Arg Tyr Gln Gln Ile Asn Leu Arg Asp Ala Tyr Val Asp His Leu Phe
   145                 150                 155                 160
   Gly Tyr Ile Asn Val Lys Asn Leu Thr Pro Leu Lys Leu Val Ile Asn
                   165                 170                 175
   Ser Gly Asn Gly Ala Ala Gly Pro Val Val Asp Ala Ile Glu Ala Arg
               180                 185                 190
   Phe Lys Ala Leu Gly Ala Pro Val Glu Leu Ile Lys Val His Asn Thr
           195                 200                 205
   Pro Asp Gly Asn Phe Pro Asn Gly Ile Pro Asn Pro Leu Leu Pro Glu
       210                 215                 220
   Cys Arg Asp Asp Thr Arg Asn Ala Val Ile Lys His Gly Ala Asp Met
   225                 230                 235                 240
   Gly Ile Ala Phe Asp Gly Asp Phe Asp Arg Cys Phe Leu Phe Asp Glu
                   245                 250                 255
   Lys Gly Gln Phe Ile Glu Gly Tyr Tyr Ile Val Gly Leu Leu Ala Glu
               260                 265                 270
   Ala Phe Leu Glu Lys Asn Pro Gly Ala Lys Ile Ile His Asp Pro Arg
           275                 280                 285
   Leu Ser Trp Asn Thr Val Asp Val Val Thr Ala Ala Gly Gly Thr Pro
       290                 295                 300
   Val Met Ser Lys Thr Gly His Ala Phe Ile Lys Glu Arg Met Arg Lys
   305                 310                 315                 320
   Glu Asp Ala Ile Tyr Gly Gly Glu Met Ser Ala His His Tyr Phe Arg
                   325                 330                 335
   Asp Phe Ala Tyr Cys Asp Ser Gly Met Ile Pro Trp Leu Leu Val Ala
               340                 345                 350
   Glu Leu Val Cys Leu Lys Asp Lys Thr Leu Gly Glu Leu Val Arg Asp
           355                 360                 365
   Arg Met Ala Ala Phe Pro Ala Ser Gly Glu Ile Asn Ser Lys Leu Ala
       370                 375                 380
   Gln Pro Val Glu Ala Ile Asn Arg Val Glu Gln His Phe Ser Arg Glu
   385                 390                 395                 400
   Ala Leu Ala Val Asp Arg Thr Asp Gly Ile Ser Met Thr Phe Ala Asp
                   405                 410                 415
   Trp Arg Phe Asn Leu Arg Thr Ser Asn Thr Glu Pro Val Val Arg Leu
               420                 425                 430
   Asn Val Glu Ser Arg Gly Asp Val Pro Leu Met Glu Ala Arg Thr Arg
           435                 440                 445
   Thr Leu Leu Thr Leu Leu Asn Glu
       450                 455
<210> 25
   <211> 1437
   <212> DNA
   <213> Escherichia coli
<400> 25
   atggcgcagt cgaaactcta tccagttgtg atggcaggtg gctccggtag ccgcttatgg 60
   ccgctttccc gcgtacttta tcccaagcag tttttatgcc tgaaaggcga tctcaccatg 120
   ctgcaaacca ccatctgccg cctgaacggc gtggagtgcg aaagcccggt ggtgatttgc 180
   aatgagcagc accgctttat tgtcgcggaa cagctgcgtc aactgaacaa acttaccgag 240
   aacattattc tcgaaccggc agggcgaaac acggcacctg ccattgcgct ggcggcgctg 300
   gcggcaaaac gtcatagccc ggagagcgac ccgttaatgc tggtattggc ggcggatcat 360
   gtgattgccg atgaagacgc gttccgtgcc gccgtgcgta atgccatgcc atatgccgaa 420
   gcgggcaagc tggtgacctt cggcattgtg ccggatctac cagaaaccgg ttatggctat 480
   attcgtcgcg gtgaagtgtc tgcgggtgag caggatatgg tggcctttga agtggcgcag 540
   tttgtcgaaa aaccgaatct ggaaaccgct caggcctatg tggcaagcgg cgaatattac 600
   tggaacagcg gtatgttcct gttccgcgcc ggacgctatc tcgaagaact gaaaaaatat 660
   cgcccggata tcctcgatgc ctgtgaaaaa gcgatgagcg ccgtcgatcc ggatctcaat 720
   tttattcgcg tggatgaaga agcgtttctc gcctgcccgg aagagtcggt ggattacgcg 780
   gtcatggaac gtacggcaga tgctgttgtg gtgccgatgg atgcgggctg gagcgatgtt 840
   ggctcctggt cttcattatg ggagatcagc gcccacaccg ccgagggcaa cgtttgccac 900
   ggcgatgtga ttaatcacaa aactgaaaac agctatgtgt atgctgaatc tggcctggtc 960
   accaccgtcg gggtgaaaga tctggtagtg gtgcagacca aagatgcggt gctgattgcc 1020
   gaccgtaacg cggtacagga tgtgaaaaaa gtggtcgagc agatcaaagc cgatggtcgc 1080
   catgagcatc gggtgcatcg cgaagtgtat cgtccgtggg gcaaatatga ctctatcgac 1140
   gcgggcgacc gctaccaggt gaaacgcatc accgtgaaac cgggcgaggg cttgtcggta 1200
   cagatgcacc atcaccgcgc ggaacactgg gtggttgtcg cgggaacggc aaaagtcacc 1260
   attgatggtg atatcaaact gcttggtgaa aacgagtcca tttatattcc gctgggggcg 1320
   acgcattgcc tggaaaaccc ggggaaaatt ccgctcgatt taattgaagt gcgctccggc 1380
   tcttatctcg aagaggatga tgtggtgcgt ttcgcggatc gctacggacg ggtgtaa 1437
<210> 26
   <211> 478
   <212> PRT
   <213> Escherichia coli
<400> 26
   Met Ala Gln Ser Lys Leu Tyr Pro Val Val Met Ala Gly Gly Ser Gly
   1               5                   10                  15
   Ser Arg Leu Trp Pro Leu Ser Arg Val Leu Tyr Pro Lys Gln Phe Leu
               20                  25                  30
   Cys Leu Lys Gly Asp Leu Thr Met Leu Gln Thr Thr Ile Cys Arg Leu
           35                  40                  45
   Asn Gly Val Glu Cys Glu Ser Pro Val Val Ile Cys Asn Glu Gln His
       50                  55                  60
   Arg Phe Ile Val Ala Glu Gln Leu Arg Gln Leu Asn Lys Leu Thr Glu
   65                  70                  75                  80
   Asn Ile Ile Leu Glu Pro Ala Gly Arg Asn Thr Ala Pro Ala Ile Ala
                   85                  90                  95
   Leu Ala Ala Leu Ala Ala Lys Arg His Ser Pro Glu Ser Asp Pro Leu
               100                 105                 110
   Met Leu Val Leu Ala Ala Asp His Val Ile Ala Asp Glu Asp Ala Phe
           115                 120                 125
   Arg Ala Ala Val Arg Asn Ala Met Pro Tyr Ala Glu Ala Gly Lys Leu
       130                 135                 140
   Val Thr Phe Gly Ile Val Pro Asp Leu Pro Glu Thr Gly Tyr Gly Tyr
   145                 150                 155                 160
   Ile Arg Arg Gly Glu Val Ser Ala Gly Glu Gln Asp Met Val Ala Phe
   165 170 175
   Glu Val Ala Gln Phe Val Glu Lys Pro Asn Leu Glu Thr Ala Gln Ala
   180 185 190
   Tyr Val Ala Ser Gly Glu Tyr Tyr Trp Asn Ser Gly Met Phe Leu Phe
   195 200 205
   Arg Ala Gly Arg Tyr Leu Glu Glu Leu Lys Lys Tyr Arg Pro Asp Ile
   210 215 220
   Leu Asp Ala Cys Glu Lys Ala Met Ser Ala Val Asp Pro Asp Leu Asn
   225 230 235 240
   Phe Ile Arg Val Asp Glu Glu Ala Phe Leu Ala Cys Pro Glu Glu Ser
   245 250 255
   Val Asp Tyr Ala Val Met Glu Arg Thr Ala Asp Ala Val Val Val Pro
   260 265 270
   Met Asp Ala Gly Trp Ser Asp Val Gly Ser Trp Ser Ser Leu Trp Glu
   275 280 285
   Ile Ser Ala His Thr Ala Glu Gly Asn Val Cys His Gly Asp Val Ile
   290 295 300
   Asn His Lys Thr Glu Asn Ser Tyr Val Tyr Ala Glu Ser Gly Leu Val
   305 310 315 320
   Thr Thr Val Gly Val Lys Asp Leu Val Val Val Gln Thr Lys Asp Ala
   325 330 335
   Val Leu Ile Ala Asp Arg Asn Ala Val Gln Asp Val Lys Lys Val Val
   340 345 350
   Glu Gln Ile Lys Ala Asp Gly Arg His Glu His Arg Val His Arg Glu
   355 360 365
   Val Tyr Arg Pro Trp Gly Lys Tyr Asp Ser Ile Asp Ala Gly Asp Arg
   370 375 380
   Tyr Gln Val Lys Arg Ile Thr Val Lys Pro Gly Glu Gly Leu Ser Val
   385 390 395 400
   Gln Met His His His Arg Ala Glu His Trp Val Val Val Ala Gly Thr
   405 410 415
   Ala Lys Val Thr Ile Asp Gly Asp Ile Lys Leu Leu Gly Glu Asn Glu
               420                 425                 430
   Ser Ile Tyr Ile Pro Leu Gly Ala Thr His Cys Leu Glu Asn Pro Gly
           435                 440                 445
   Lys Ile Pro Leu Asp Leu Ile Glu Val Arg Ser Gly Ser Tyr Leu Glu
       450                 455                 460
   Glu Asp Asp Val Val Arg Phe Ala Asp Arg Tyr Gly Arg Val
   465                 470                 475
<210> 27
   <211> 1830
   <212> DNA
   <213> Escherichia coli
<400> 27
   atgtgtggaa ttgttggcgc gatcgcgcaa cgtgatgtag cagaaatcct tcttgaaggt 60
   ttacgtcgtc tggaataccg cggatatgac tctgccggtc tggccgttgt tgatgcagaa 120
   ggtcatatga cccgcctgcg tcgcctcggt aaagtccaga tgctggcaca ggcagcggaa 180
   gaacatcctc tgcatggcgg cactggtatt gctcacactc gctgggcgac ccacggtgaa 240
   ccttcagaag tgaatgcgca tccgcatgtt tctgaacaca ttgtggtggt gcataacggc 300
   atcatcgaaa accatgaacc gctgcgtgaa gagctaaaag cgcgtggcta taccttcgtt 360
   tctgaaaccg acaccgaagt gattgcccat ctggtgaact gggagctgaa acaaggcggg 420
   actctgcgtg aggccgttct gcgtgctatc ccgcagctgc gtggtgcgta cggtacagtg 480
   atcatggact cccgtcaccc ggataccctg ctggcggcac gttctggtag tccgctggtg 540
   attggcctgg ggatgggcga aaactttatc gcttctgacc agctggcgct gttgccggtg 600
   acccgtcgct ttatcttcct tgaagagggc gatattgcgg aaatcactcg ccgttcggta 660
   aacatcttcg ataaaactgg cgcggaagta aaacgtcagg atatcgaatc caatctgcaa 720
   tatgacgcgg gcgataaagg catttaccgt cactacatgc agaaagagat ctacgaacag 780
   ccgaacgcga tcaaaaacac ccttaccgga cgcatcagcc acggtcaggt tgatttaagc 840
   gagctgggac cgaacgccga cgaactgctg tcgaaggttg agcatattca gatcctcgcc 900
   tgtggtactt cttataactc cggtatggtt tcccgctact ggtttgaatc gctagcaggt 960
   attccgtgcg acgtcgaaat cgcctctgaa ttccgctatc gcaaatctgc cgtgcgtcgt 1020
   aacagcctga tgatcacctt gtcacagtct ggcgaaaccg cggataccct ggctggcctg 1080
   cgtctgtcga aagagctggg ttaccttggt tcactggcaa tctgtaacgt tccgggttct 1140
   tctctggtgc gcgaatccga tctggcgcta atgaccaacg cgggtacaga aatcggcgtg 1200
   gcatccacta aagcattcac cactcagtta actgtgctgt tgatgctggt ggcgaagctg 1260
   tctcgcctga aaggtctgga tgcctccatt gaacatgaca tcgtgcatgg tctgcaggcg 1320
   ctgccgagcc gtattgagca gatgctgtct caggacaaac gcattgaagc gctggcagaa 1380
   gatttctctg acaaacatca cgcgctgttc ctgggccgtg gcgatcagta cccaatcgcg 1440
   ctggaaggcg cattgaagtt gaaagagatc tcttacattc acgctgaagc ctacgctgct 1500
   ggcgaactga aacacggtcc gctggcgcta attgatgccg atatgccggt tattgttgtt 1560
   gcaccgaaca acgaattgct ggaaaaactg aaatccaaca ttgaagaagt tcgcgcgcgt 1620
   ggcggtcagt tgtatgtctt cgccgatcag gatgcgggtt ttgtaagtag cgataacatg 1680
   cacatcatcg agatgccgca tgtggaagag gtgattgcac cgatcttcta caccgttccg 1740
   ctgcagctgc tggcttacca tgtcgcgctg atcaaaggca ccgacgttga ccagccgcgt 1800
   aacctggcaa aatcggttac ggttgagtaa 1830
<210> 28
   <211> 609
   <212> PRT
   <213> Escherichia coli
<400> 28
   Met Cys Gly Ile Val Gly Ala Ile Ala Gln Arg Asp Val Ala Glu Ile
   1               5                   10                  15
   Leu Leu Glu Gly Leu Arg Arg Leu Glu Tyr Arg Gly Tyr Asp Ser Ala
               20                  25                  30
   Gly Leu Ala Val Val Asp Ala Glu Gly His Met Thr Arg Leu Arg Arg
           35                  40                  45
   Leu Gly Lys Val Gln Met Leu Ala Gln Ala Ala Glu Glu His Pro Leu
       50                  55                  60
   His Gly Gly Thr Gly Ile Ala His Thr Arg Trp Ala Thr His Gly Glu
   65                  70                  75                  80
   Pro Ser Glu Val Asn Ala His Pro His Val Ser Glu His Ile Val Val
                   85                  90                  95
   Val His Asn Gly Ile Ile Glu Asn His Glu Pro Leu Arg Glu Glu Leu
               100                 105                 110
   Lys Ala Arg Gly Tyr Thr Phe Val Ser Glu Thr Asp Thr Glu Val Ile
           115                 120                 125
   Ala His Leu Val Asn Trp Glu Leu Lys Gln Gly Gly Thr Leu Arg Glu
       130                 135                 140
   Ala Val Leu Arg Ala Ile Pro Gln Leu Arg Gly Ala Tyr Gly Thr Val
   145                 150                 155                 160
   Ile Met Asp Ser Arg His Pro Asp Thr Leu Leu Ala Ala Arg Ser Gly
                   165                 170                 175
   Ser Pro Leu Val Ile Gly Leu Gly Met Gly Glu Asn Phe Ile Ala Ser
               180                 185                 190
   Asp Gln Leu Ala Leu Leu Pro Val Thr Arg Arg Phe Ile Phe Leu Glu
           195                 200                 205
   Glu Gly Asp Ile Ala Glu Ile Thr Arg Arg Ser Val Asn Ile Phe Asp
       210                 215                 220
   Lys Thr Gly Ala Glu Val Lys Arg Gln Asp Ile Glu Ser Asn Leu Gln
   225                 230                 235                 240
   Tyr Asp Ala Gly Asp Lys Gly Ile Tyr Arg His Tyr Met Gln Lys Glu
                   245                 250                 255
   Ile Tyr Glu Gln Pro Asn Ala Ile Lys Asn Thr Leu Thr Gly Arg Ile
               260                 265                 270
   Ser His Gly Gln Val Asp Leu Ser Glu Leu Gly Pro Asn Ala Asp Glu
           275                 280                 285
   Leu Leu Ser Lys Val Glu His Ile Gln Ile Leu Ala Cys Gly Thr Ser
       290                 295                 300
   Tyr Asn Ser Gly Met Val Ser Arg Tyr Trp Phe Glu Ser Leu Ala Gly
   305                 310                 315                 320
   Ile Pro Cys Asp Val Glu Ile Ala Ser Glu Phe Arg Tyr Arg Lys Ser
                   325                 330                 335
   Ala Val Arg Arg Asn Ser Leu Met Ile Thr Leu Ser Gln Ser Gly Glu
               340                 345                 350
   Thr Ala Asp Thr Leu Ala Gly Leu Arg Leu Ser Lys Glu Leu Gly Tyr
           355                 360                 365
   Leu Gly Ser Leu Ala Ile Cys Asn Val Pro Gly Ser Ser Leu Val Arg
       370                 375                 380
   Glu Ser Asp Leu Ala Leu Met Thr Asn Ala Gly Thr Glu Ile Gly Val
   385                 390                 395                 400
   Ala Ser Thr Lys Ala Phe Thr Thr Gln Leu Thr Val Leu Leu Met Leu
                   405                 410                 415
   Val Ala Lys Leu Ser Arg Leu Lys Gly Leu Asp Ala Ser Ile Glu His
               420                 425                 430
   Asp Ile Val His Gly Leu Gln Ala Leu Pro Ser Arg Ile Glu Gln Met
           435                 440                 445
   Leu Ser Gln Asp Lys Arg Ile Glu Ala Leu Ala Glu Asp Phe Ser Asp
       450                 455                 460
   Lys His His Ala Leu Phe Leu Gly Arg Gly Asp Gln Tyr Pro Ile Ala
   465                 470                 475                 480
   Leu Glu Gly Ala Leu Lys Leu Lys Glu Ile Ser Tyr Ile His Ala Glu
                   485                 490                 495
   Ala Tyr Ala Ala Gly Glu Leu Lys His Gly Pro Leu Ala Leu Ile Asp
               500                 505                 510
   Ala Asp Met Pro Val Ile Val Val Ala Pro Asn Asn Glu Leu Leu Glu
           515                 520                 525
   Lys Leu Lys Ser Asn Ile Glu Glu Val Arg Ala Arg Gly Gly Gln Leu
       530                 535                 540
   Tyr Val Phe Ala Asp Gln Asp Ala Gly Phe Val Ser Ser Asp Asn Met
   545                 550                 555                 560
   His Ile Ile Glu Met Pro His Val Glu Glu Val Ile Ala Pro Ile Phe
                   565                 570                 575
   Tyr Thr Val Pro Leu Gln Leu Leu Ala Tyr His Val Ala Leu Ile Lys
               580                 585                 590
   Gly Thr Asp Val Asp Gln Pro Arg Asn Leu Ala Lys Ser Val Thr Val
           595                 600                 605
   Glu
<210> 29
   <211> 2028
   <212> DNA
   <213> Photobacterium damselae
<400> 29
   atgaaaaaaa tcctgaccgt gctgtccatc tttatcctgt ctgcctgtaa tagcgacaat 60
   accagcctga aagagactgt tagcagcaat tcagcggatg ttgtggaaac cgaaacttat 120
   caactgacgc cgatcgatgc tccttcttcg ttcctgagcc attcttggga acagacctgt 180
   ggtacaccaa ttctgaacga gtccgacaaa caggccattt ccttcgattt tgttgccccg 240
   gaactgaaac aagacgagaa atattgcttc accttcaaag gcattaccgg tgatcatcgt 300
   tatatcacga acaccactct gactgtcgta gcaccgacac tggaagtgta tatcgaccat 360
   gccagcctgc ctagtctgca gcaactgatc catattatcc aggcgaaaga cgaatatccg 420
   agcaaccagc gttttgtgag ctggaaacgt gttactgtgg atgccgacaa cgccaataaa 480
   ctgaacattc acacctatcc tctgaaaggc aataacacca gccctgagat ggtagcggcg 540
   attgatgagt atgcccagag caaaaaccgt ctgaacattg agttctatac caatacggcc 600
   cacgtgttta ataacctgcc gccaatcatt caacctctgt ataacaacga gaaagtgaaa 660
   atcagccaca tttcgctgta tgatgatggc agtagcgagt atgttagcct gtatcagtgg 720
   aaagacaccc cgaataaaat cgagactctg gagggtgaag tttctctgct ggccaactat 780
   ctggccggta caagtcctga tgctccgaaa gggatgggta accgctataa ttggcacaaa 840
   ctgtatgaca ccgactatta ttttctgcgc gaggattatc tggacgtgga agccaatctg 900
   catgatctgc gcgattatct gggttctagc gccaaacaaa tgccgtggga tgaatttgct 960
   aaactgtccg attctcagca aaccctgttc ctggacatcg ttggctttga taaagagcag 1020
   ctgcaacagc agtatagcca gtcaccgctg ccgaacttca tttttactgg caccaccaca 1080
   tgggcagggg gtgagacaaa agagtattat gctcaacaac aggtgaacgt catcaacaat 1140
   gccattaacg aaacctcccc atattatctg ggtaaagact atgacctgtt ctttaaaggc 1200
   catccggctg gaggagtgat taatgatatt atcctgggct cctttcctga catgattaac 1260
   attccggcga aaatctcatt tgaggtgctg atgatgactg atatgctgcc ggataccgtt 1320
   gctggaattg cctcttccct gtatttcacc attcctgccg acaaagtgaa cttcatcgtg 1380
   ttcaccagca gtgataccat tacagaccgt gaagaagcgc tgaaatctcc tctggttcag 1440
   gtgatgctga cactgggtat cgtgaaagaa aaagacgtcc tgttttgggc cgaccataaa 1500
   gtgaatagca tggaggtggc catcgacgaa gcgtgtactc gtattatcgc caaacgtcag 1560
   cctaccgctt cagatctgcg tctggttatc gccattatca aaacgatcac cgatctggag 1620
   cgtattggag atgttgccga aagcattgcc aaagttgccc tggagagctt ttctaacaaa 1680
   cagtataatc tgctggtcag cctggaatct ctgggtcaac acaccgttcg tatgctgcat 1740
   gaagtgctgg atgcttttgc ccgtatggat gtgaaagcag ccattgaagt ctatcaggag 1800
   gatgaccgta tcgatcagga atatgagagc attgtccgtc aactgatggc ccatatgatg 1860
   gaagatccgt ctagcattcc gaatgtgatg aaagtgatgt gggcagctcg tagtattgaa     1920
   cgtgtgggtg accgctgcca gaacatttgt gagtatatca tctatttcgt aaaaggcaaa     1980
   gatgttcgcc acaccaaacc ggatgacttc ggtactatgc tggactga                  2028
<210> 30
   <211> 675
   <212> PRT
   <213> Photobacterium damselae
<400> 30
   Met Lys Lys Ile Leu Thr Val Leu Ser Ile Phe Ile Leu Ser Ala Cys
   1               5                   10                  15
   Asn Ser Asp Asn Thr Ser Leu Lys Glu Thr Val Ser Ser Asn Ser Ala
               20                  25                  30
   Asp Val Val Glu Thr Glu Thr Tyr Gln Leu Thr Pro Ile Asp Ala Pro
           35                  40                  45
   Ser Ser Phe Leu Ser His Ser Trp Glu Gln Thr Cys Gly Thr Pro Ile
       50                  55                  60
   Leu Asn Glu Ser Asp Lys Gln Ala Ile Ser Phe Asp Phe Val Ala Pro
   65                  70                  75                  80
   Glu Leu Lys Gln Asp Glu Lys Tyr Cys Phe Thr Phe Lys Gly Ile Thr
                   85                  90                  95
   Gly Asp His Arg Tyr Ile Thr Asn Thr Thr Leu Thr Val Val Ala Pro
               100                 105                 110
   Thr Leu Glu Val Tyr Ile Asp His Ala Ser Leu Pro Ser Leu Gln Gln
           115                 120                 125
   Leu Ile His Ile Ile Gln Ala Lys Asp Glu Tyr Pro Ser Asn Gln Arg
       130                 135                 140
   Phe Val Ser Trp Lys Arg Val Thr Val Asp Ala Asp Asn Ala Asn Lys
   145                 150                 155                 160
   Leu Asn Ile His Thr Tyr Pro Leu Lys Gly Asn Asn Thr Ser Pro Glu
                   165                 170                 175
   Met Val Ala Ala Ile Asp Glu Tyr Ala Gln Ser Lys Asn Arg Leu Asn
               180                 185                 190
   Ile Glu Phe Tyr Thr Asn Thr Ala His Val Phe Asn Asn Leu Pro Pro
   195 200 205
   Ile Ile Gln Pro Leu Tyr Asn Asn Glu Lys Val Lys Ile Ser His Ile
   210 215 220
   Ser Leu Tyr Asp Asp Gly Ser Ser Glu Tyr Val Ser Leu Tyr Gln Trp
   225 230 235 240
   Lys Asp Thr Pro Asn Lys Ile Glu Thr Leu Glu Gly Glu Val Ser Leu
   245 250 255
   Leu Ala Asn Tyr Leu Ala Gly Thr Ser Pro Asp Ala Pro Lys Gly Met
   260 265 270
   Gly Asn Arg Tyr Asn Trp His Lys Leu Tyr Asp Thr Asp Tyr Tyr Phe
   275 280 285
   Leu Arg Glu Asp Tyr Leu Asp Val Glu Ala Asn Leu His Asp Leu Arg
   290 295 300
   Asp Tyr Leu Gly Ser Ser Ala Lys Gln Met Pro Trp Asp Glu Phe Ala
   305 310 315 320
   Lys Leu Ser Asp Ser Gln Gln Thr Leu Phe Leu Asp Ile Val Gly Phe
   325 330 335
   Asp Lys Glu Gln Leu Gln Gln Gln Tyr Ser Gln Ser Pro Leu Pro Asn
   340 345 350
   Phe Ile Phe Thr Gly Thr Thr Thr Trp Ala Gly Gly Glu Thr Lys Glu
   355 360 365
   Tyr Tyr Ala Gln Gln Gln Val Asn Val Ile Asn Asn Ala Ile Asn Glu
   370 375 380
   Thr Ser Pro Tyr Tyr Leu Gly Lys Asp Tyr Asp Leu Phe Phe Lys Gly
   385 390 395 400
   His Pro Ala Gly Gly Val Ile Asn Asp Ile Ile Leu Gly Ser Phe Pro
   405 410 415
   Asp Met Ile Asn Ile Pro Ala Lys Ile Ser Phe Glu Val Leu Met Met
   420 425 430
   Thr Asp Met Leu Pro Asp Thr Val Ala Gly Ile Ala Ser Ser Leu Tyr
   435 440 445
   Phe Thr Ile Pro Ala Asp Lys Val Asn Phe Ile Val Phe Thr Ser Ser
       450                 455                 460
   Asp Thr Ile Thr Asp Arg Glu Glu Ala Leu Lys Ser Pro Leu Val Gln
   465                 470                 475                 480
   Val Met Leu Thr Leu Gly Ile Val Lys Glu Lys Asp Val Leu Phe Trp
                   485                 490                 495
   Ala Asp His Lys Val Asn Ser Met Glu Val Ala Ile Asp Glu Ala Cys
               500                 505                 510
   Thr Arg Ile Ile Ala Lys Arg Gln Pro Thr Ala Ser Asp Leu Arg Leu
           515                 520                 525
   Val Ile Ala Ile Ile Lys Thr Ile Thr Asp Leu Glu Arg Ile Gly Asp
       530                 535                 540
   Val Ala Glu Ser Ile Ala Lys Val Ala Leu Glu Ser Phe Ser Asn Lys
   545                 550                 555                 560
   Gln Tyr Asn Leu Leu Val Ser Leu Glu Ser Leu Gly Gln His Thr Val
                   565                 570                 575
   Arg Met Leu His Glu Val Leu Asp Ala Phe Ala Arg Met Asp Val Lys
               580                 585                 590
   Ala Ala Ile Glu Val Tyr Gln Glu Asp Asp Arg Ile Asp Gln Glu Tyr
           595                 600                 605
   Glu Ser Ile Val Arg Gln Leu Met Ala His Met Met Glu Asp Pro Ser
       610                 615                 620
   Ser Ile Pro Asn Val Met Lys Val Met Trp Ala Ala Arg Ser Ile Glu
   625                 630                 635                 640
   Arg Val Gly Asp Arg Cys Gln Asn Ile Cys Glu Tyr Ile Ile Tyr Phe
                   645                 650                 655
   Val Lys Gly Lys Asp Val Arg His Thr Lys Pro Asp Asp Phe Gly Thr
               660                 665                 670
   Met Leu Asp
           675
<210> 31
   <211> 2367
   <212> DNA
   <213> Artificial Sequence
<220>
   <223> Description of Artificial Sequence: Synthetic polynucleotide
<400> 31
   atgaaaatcg aagaaggtaa actggtaatc tggattaacg gcgataaagg ctataacggt 60
   ctcgctgaag tcggtaagaa attcgagaaa gataccggaa ttaaagtcac cgttgagcat 120
   ccggataaac tggaagagaa attcccacag gttgcggcaa ctggcgatgg ccctgacatt 180
   atcttctggg cacacgaccg ctttggtggc tacgctcaat ctggcctgtt ggctgaaatc 240
   accccggaca aagcgttcca ggacaagctg tatccgttta cctgggatgc cgtacgttac 300
   aacggcaagc tgattgctta cccgatcgct gttgaagcgt tatcgctgat ttataacaaa 360
   gatctgctgc cgaacccgcc aaaaacctgg gaagagatcc cggcgctgga taaagaactg 420
   aaagcgaaag gtaagagcgc gctgatgttc aacctgcaag aaccgtactt cacctggccg 480
   ctgattgctg ctgacggggg ttatgcgttc aagtatgaaa acggcaagta cgacattaaa 540
   gacgtgggcg tggataacgc tggcgcgaaa gcgggtctga ccttcctggt tgacctgatt 600
   aaaaacaaac acatgaatgc agacaccgat tactccatcg cagaagctgc ctttaataaa 660
   ggcgaaacag cgatgaccat caacggcccg tgggcatggt ccaacatcga caccagcaaa 720
   gtgaattatg gtgtaacggt actgccgacc ttcaagggtc aaccatccaa accgttcgtt 780
   ggcgtgctga gcgcaggtat taacgccgcc agtccgaaca aagagctggc gaaagagttc 840
   ctcgaaaact atctgctgac tgatgaaggt ctggaagcgg ttaataaaga caaaccgctg 900
   ggtgccgtag cgctgaagtc ttacgaggaa gagttggcga aagatccacg tattgccgcc 960
   accatggaaa acgcccagaa aggtgaaatc atgccgaaca tcccgcagat gtccgctttc 1020
   tggtatgccg tgcgtactgc ggtgatcaac gccgccagcg gtcgtcagac tgtcgatgaa 1080
   gccctgaaag acgcgcagac tcgtatcacc aaggcgacac agtcagaata tgcagatcgc 1140
   cttgctgctg caattgaagc agaaaatcat tgtacaagcc agaccagatt gcttattgac 1200
   cagattagcc tgcagcaagg aagaatagtt gctcttgaag aacaaatgaa gcgtcaggac 1260
   caggagtgcc gacaattaag ggctcttgtt caggatcttg aaagtaaggg cataaaaaag 1320
   ttgatcggaa atgtacagat gccagtggct gctgtagttg ttatggcttg caatcgggct 1380
   gattacctgg aaaagactat taaatccatc ttaaaatacc aaatatctgt tgcgtcaaaa 1440
   tatcctcttt tcatatccca ggatggatca catcctgatg tcaggaagct tgctttgagc 1500
   tatgatcagc tgacgtatat gcagcacttg gattttgaac ctgtgcatac tgaaagacca 1560
   ggggagctga ttgcatacta caaaattgca cgtcattaca agtgggcatt ggatcagctg 1620
   ttttacaagc ataattttag ccgtgttatc atactagaag atgatatgga aattgcccct 1680
   gatttttttg acttttttga ggctggagct actcttcttg acagagacaa gtcgattatg 1740
   gctatttctt cttggaatga caatggacaa atgcagtttg tccaagatcc ttatgctctt 1800
   taccgctcag atttttttcc cggtcttgga tggatgcttt caaaatctac ttgggacgaa 1860
   ttatctccaa agtggccaaa ggcttactgg gacgactggc taagactcaa agagaatcac 1920
   agaggtcgac aatttattcg cccagaagtt tgcagaacat ataattttgg tgagcatggt 1980
   tctagtttgg ggcagttttt caagcagtat cttgagccaa ttaaactaaa tgatgtccag 2040
   gttgattgga agtcaatgga ccttagttac cttttggagg acaattacgt gaaacacttt 2100
   ggtgacttgg ttaaaaaggc taagcccatc catggagctg atgctgtctt gaaagcattt 2160
   aacatagatg gtgatgtgcg tattcagtac agagatcaac tagactttga aaatatcgca 2220
   cggcaatttg gcatttttga agaatggaag gatggtgtac cacgtgcagc atataaagga 2280
   atagtagttt tccggtacca aacgtccaga cgtgtattcc ttgttggcca tgattcgctt 2340
   caacaactcg gaattgaaga tacttaa 2367
<210> 32
   <211> 2370
   <212> DNA
   <213> Artificial Sequence
<220>
   <223> Description of Artificial Sequence: Synthetic polynucleotide
<400> 32
   atgaaaatcg aagaaggtaa actggtaatc tggattaacg gcgataaagg ctataacggt      60
   ctcgctgaag tcggtaagaa attcgagaaa gataccggaa ttaaagtcac cgttgagcat     120
   ccggataaac tggaagagaa attcccacag gttgcggcaa ctggcgatgg ccctgacatt     180
   atcttctggg cacacgaccg ctttggtggc tacgctcaat ctggcctgtt ggctgaaatc     240
   accccggaca aagcgttcca ggacaagctg tatccgttta cctgggatgc cgtacgttac     300
   aacggcaagc tgattgctta cccgatcgct gttgaagcgt tatcgctgat ttataacaaa     360
   gatctgctgc cgaacccgcc aaaaacctgg gaagagatcc cggcgctgga taaagaactg     420
   aaagcgaaag gtaagagcgc gctgatgttc aacctgcaag aaccgtactt cacctggccg     480
   ctgattgctg ctgacggggg ttatgcgttc aagtatgaaa acggcaagta cgacattaaa     540
   gacgtgggcg tggataacgc tggcgcgaaa gcgggtctga ccttcctggt tgacctgatt     600
   aaaaacaaac acatgaatgc agacaccgat tactccatcg cagaagctgc ctttaataaa     660
   ggcgaaacag cgatgaccat caacggcccg tgggcatggt ccaacatcga caccagcaaa     720
   gtgaattatg gtgtaacggt actgccgacc ttcaagggtc aaccatccaa accgttcgtt     780
   ggcgtgctga gcgcaggtat taacgccgcc agtccgaaca aagagctggc gaaagagttc     840
   ctcgaaaact atctgctgac tgatgaaggt ctggaagcgg ttaataaaga caaaccgctg 900
   ggtgccgtag cgctgaagtc ttacgaggaa gagttggcga aagatccacg tattgccgcc 960
   accatggaaa acgcccagaa aggtgaaatc atgccgaaca tcccgcagat gtccgctttc 1020
   tggtatgccg tgcgtactgc ggtgatcaac gccgccagcg gtcgtcagac tgtcgatgaa 1080
   gccctgaaag acgcgcagac tcgtatcacc aagcgtcagc gtaaaaatga agccctggca 1140
   cctcctctgc tggatgctga accggcacgt ggtgctggcg gtcgtggtgg tgatcatccg 1200
   tctgttgccg ttggtattcg tcgtgtgagc aatgtttcgg ctgcctctct ggtcccggct 1260
   gttcctcaac ctgaagctga taacctgacc ctgcgctatc gctctctggt gtatcaactg 1320
   aacttcgatc aaactctgcg taacgtggat aaagcaggca catgggctcc tcgtgaactg 1380
   gtactggtag tccaggtcca taatcgtccg gaatatctgc gtctgctgct ggattctctg 1440
   cgcaaagctc aaggcatcga taatgtcctg gtcatcttct ctcatgattt ctggagcacg 1500
   gagattaacc agctgattgc cggcgtgaat ttttgtcctg tgctgcaggt gttttttccg 1560
   ttttctatcc aactgtatcc gaacgaattt ccgggttctg atcctcgtga ttgtcctcgt 1620
   gatctgccta aaaatgccgc tctgaaactg ggctgtatta atgccgagta tcctgattct 1680
   tttggccact atcgtgaggc gaaattttct cagaccaaac atcattggtg gtggaaactg 1740
   catttcgtgt gggaacgtgt gaaaatcctg cgcgactatg ctggcctgat tctgtttctg 1800
   gaagaagatc actatctggc tccggacttt tatcatgtgt tcaaaaaaat gtggaaactg 1860
   aaacagcagg aatgtccaga atgtgatgtg ctgtcactgg gcacctatag tgcttctcgc 1920
   tccttctatg gtatggccga caaagtggac gttaaaacat ggaaatccac cgagcacaac 1980
   atgggtctgg cactgactcg taatgcctat caaaaactga ttgagtgtac cgacaccttt 2040
   tgtacgtatg atgactataa ctgggactgg accctgcaat atctgaccgt gagctgtctg 2100
   ccaaaatttt ggaaagttct ggtgcctcag attcctcgta tctttcatgc tggcgactgt 2160
   ggtatgcacc ataaaaaaac ttgccgtccg tcaacacaat ctgctcagat cgagtcgctg 2220
   ctgaataata acaaacagta tatgttcccg gagactctga caatttctga aaaattcacc 2280
   gtggtcgcca tttctccgcc tcgtaaaaat ggaggttggg gcgatatccg tgaccatgaa 2340
   ctgtgtaaaa gctatcgtcg tctgcagtga 2370
<210> 33
   <211> 2445
   <212> DNA
   <213> Artificial Sequence
<220>
   <223> Description of Artificial Sequence: Synthetic polynucleotide
<400> 33
   atgaaaatcg aagaaggtaa actggtaatc tggattaacg gcgataaagg ctataacggt 60
   ctcgctgaag tcggtaagaa attcgagaaa gataccggaa ttaaagtcac cgttgagcat 120
   ccggataaac tggaagagaa attcccacag gttgcggcaa ctggcgatgg ccctgacatt 180
   atcttctggg cacacgaccg ctttggtggc tacgctcaat ctggcctgtt ggctgaaatc 240
   accccggaca aagcgttcca ggacaagctg tatccgttta cctgggatgc cgtacgttac 300
   aacggcaagc tgattgctta cccgatcgct gttgaagcgt tatcgctgat ttataacaaa 360
   gatctgctgc cgaacccgcc aaaaacctgg gaagagatcc cggcgctgga taaagaactg 420
   aaagcgaaag gtaagagcgc gctgatgttc aacctgcaag aaccgtactt cacctggccg 480
   ctgattgctg ctgacggggg ttatgcgttc aagtatgaaa acggcaagta cgacattaaa 540
   gacgtgggcg tggataacgc tggcgcgaaa gcgggtctga ccttcctggt tgacctgatt 600
   aaaaacaaac acatgaatgc agacaccgat tactccatcg cagaagctgc ctttaataaa 660
   ggcgaaacag cgatgaccat caacggcccg tgggcatggt ccaacatcga caccagcaaa 720
   gtgaattatg gtgtaacggt actgccgacc ttcaagggtc aaccatccaa accgttcgtt 780
   ggcgtgctga gcgcaggtat taacgccgcc agtccgaaca aagagctggc gaaagagttc 840
   ctcgaaaact atctgctgac tgatgaaggt ctggaagcgg ttaataaaga caaaccgctg 900
   ggtgccgtag cgctgaagtc ttacgaggaa gagttggcga aagatccacg tattgccgcc 960
   accatggaaa acgcccagaa aggtgaaatc atgccgaaca tcccgcagat gtccgctttc 1020
   tggtatgccg tgcgtactgc ggtgatcaac gccgccagcg gtcgtcagac tgtcgatgaa 1080
   gccctgaaag acgcgcagac tcgtatcacc aagattttga aagaactgac gtccaaaaag 1140
   agcttgcaag tcccgtccat ctactatcac ttgccgcact tgctgcaaaa cgagggctct 1200
   ttgcaaccgg cagttcagat cggcaatggt cgcaccggcg tgagcattgt tatgggtatc 1260
   ccgaccgtga aacgtgaagt gaaaagctat ctgattgaaa cgctgcatag cctgatcgat 1320
   aacctgtacc cggaagaaaa actggactgc gtgattgtcg ttttcattgg tgaaaccgac 1380
   acggattatg tgaatggcgt tgttgccaat ctggaaaaag agttcagcaa agagatcagc 1440
   agcggcctgg ttgagatcat ttctccgccg gagagctatt acccggatct gacgaacctg 1500
   aaagaaacct tcggtgatag caaagagcgt gtccgttggc gcactaagca gaacctggac 1560
   tattgttttc tgatgatgta cgcgcaagaa aagggtacgt attacatcca actggaggac 1620
   gacattattg tgaagcaaaa ctacttcaac accattaaga acttcgcgct gcagctgagc 1680
   agcgaagagt ggatgattct ggagttcagc cagctgggct tcattggcaa gatgtttcag 1740
   gcaccggact tgaccctgat cgtggagttt atctttatgt tctacaaaga gaaaccgatc 1800
   gattggctgc tggatcatat cctgtgggtc aaggtctgca atccggaaaa agatgccaag 1860
   cattgtgacc gccagaaagc gaatctgcgt attcgttttc gtcctagcct gttccaacac 1920
   gtgggtctgc acagctctct gaccggtaag atccaaaagc tgaccgacaa agattacatg 1980
   aaaccgctgc tgctgaagat ccatgtcaac ccgccagcag aggtgagcac ctcgctgaaa 2040
   gtctaccagg gtcacactct ggagaaaacc tatatgggcg aggacttctt ttgggcgatt 2100
   acgcctgttg cgggtgacta tatcttgttt aagtttgaca agccggttaa tgtagagagc 2160
   tacttgtttc atagcggtaa ccaggatcac ccaggtgaca ttctgctgaa caccaccgtt 2220
   gaagtgttgc cgctgaaaag cgaaggtctg gatatttcga aagaaacgaa ggataagcgt 2280
   ctggaggatg gttacttccg tatcggcaag ttcgagaatg gcgtggctga aggtatggtc 2340
   gacccgagcc tgaacccgat ttccgcattt cgcctgtccg tcatccagaa tagcgcggtt 2400
   tgggctatcc tgaatgagat tcacatcaaa aaggttacga attaa 2445
<210> 34
   <211> 2247
   <212> DNA
   <213> Artificial Sequence
<220>
   <223> Description of Artificial Sequence: Synthetic polynucleotide
<400> 34
   atgaaattgt tctacaaacc gggtgcctgc tctctcgctt cccatatcac cctgcgtgag      60
   agcggaaagg attttaccct cgtcagtgtg gatttaatga aaaaacgtct cgaaaacggt     120
   gacgattact ttgccgttaa ccctaagggg caggtgcctg cattgctgct ggatgacggt     180
   actttgctga cggaaggcgt agcgattatg cagtatcttg ccgacagcgt ccccgaccgc     240
   cagttgctgg caccggtaaa cagtatttcc cgctataaaa ccatcgaatg gctgaattac     300
   atcgccaccg agctgcataa aggtttcaca cctctgtttc gccctgatac accggaagag     360
   tacaaaccga cagttcgcgc gcagctggag aagaagctgc aatatgtgaa cgaggcactg     420
   aaggatgagc actggatctg cgggcaaaga tttacaattg ctgatgccta tctgtttacg     480
   gttctgcgct gggcatacgc ggtgaaactg aatctggaag ggttagagca cattgcagca     540
   tttatgcaac gtatggctga acgtccggaa gtacaagacg cgctgtcagc ggaaggctta     600
   aagggcagtg cttggacaaa ctacaatttt gaagaggtta agtctcattt tgggttcaaa     660
   aaatatgttg tatcatcttt agtactagtg tatggactaa ttaaggttct cacgtggatc     720
   ttccgtcaat gggtgtattc cagcttgaat ccgttctcca aaaaatcttc attactgaac     780
   agagcagttg cctcctgtgg tgagaagaat gtgaaagttt ttggtttttt tcatccgtat     840
   tgtaatgctg gtggtggtgg ggaaaaagtg ctctggaaag ctgtagatat cactttgaga     900
   aaagatgcta agaacgttat tgtcatttat tcaggggatt ttgtgaatgg agagaatgtt     960
   actccggaga atattctaaa taatgtgaaa gcgaagttcg attacgactt ggattcggat    1020
   agaatatttt tcatttcatt gaagctaaga tacttggtgg attcttcaac atggaagcat      1080
   ttcacgttga ttggacaagc aattggatca atgattctcg catttgaatc cattattcag      1140
   tgtccacctg atatatggat tgatacaatg gggtaccctt tcagctatcc tattattgct      1200
   aggtttttga ggagaattcc tatcgtcaca tatacgcatt atccgataat gtcaaaagac      1260
   atgttaaata agctgttcaa aatgcccaag aagggtatca aagtttacgg taaaatatta      1320
   tactggaaag tttttatgtt aatttatcaa tccattggtt ctaaaattga tattgtaatc      1380
   acaaactcaa catggacaaa taaccacata aagcaaattt ggcaatccaa tacgtgtaaa      1440
   attatatatc ctccatgctc tactgagaaa ttagtagatt ggaagcaaaa gtttggtact      1500
   gcaaagggtg agagattaaa tcaagcaatt gtgttggcac aatttcgtcc tgagaaacgt      1560
   cataagttaa tcattgagtc ctttgcaact ttcttgaaaa atttaccgga ttctgtatcg      1620
   ccaattaaat tgataatggc ggggtccact agatccaagc aagatgaaaa ttatgttaaa      1680
   agtttacaag actggtcaga aaatgtatta aaaattccta aacatttgat atcattcgaa      1740
   aaaaatctgc ccttcgataa gattgaaata ttactaaaca aatctacttt cggtgttaat      1800
   gccatgtgga atgagcactt tggaattgca gttgtagagt atatggcttc cggtttgatc      1860
   cccatagttc atgcctcggc gggcccattg ttagatatag ttactccatg ggatgccaac      1920
   gggaatatcg gaaaagctcc accacaatgg gagttacaaa agaaatattt tgcaaaactc      1980
   gaagatgatg gtgaaactac tggatttttc tttaaagagc cgagtgatcc tgattataac      2040
   acaaccaaag atcctctgag ataccctaat ttgtccgacc ttttcttaca aattacgaaa      2100
   ctggactatg actgcctaag ggtgatgggc gcaagaaacc agcagtattc attgtataaa      2160
   ttctctgatt tgaagtttga taaagattgg gaaaactttg tactgaatcc tatttgtaaa      2220
   ttattagaag aggaggaaag gggctga                                          2247
<210> 35
   <211> 6
   <212> PRT
   <213> Artificial Sequence
<220>
   <223> Description of Artificial Sequence: Synthetic 6xHis tag
<400> 35
   His His His His His His
   1               5
<210> 36
   <211> 5
   <212> PRT
   <213> Artificial Sequence
<220>
   <223> Description of Artificial Sequence: Synthetic peptide
<400> 36
   Asp Gln Asn Ala Thr
   1               5